(19) Europäisches Patentamt
European Patent Office
Office européen des brevets

(11) EP 1 227 083 A1

(12) **EUROPEAN PATENT APPLICATION**
published in accordance with Art. 158(3) EPC

(43) Date of publication:
**31.07.2002 Bulletin 2002/31**

(21) Application number: **00964760.3**

(22) Date of filing: **10.10.2000**

(51) Int Cl.[7]: **C07D 211/40**

(86) International application number:
**PCT/JP00/07033**

(87) International publication number:
**WO 01/27082 (19.04.2001 Gazette 2001/16)**

(84) Designated Contracting States:
**AT BE CH CY DE DK ES FI FR GB GR IE IT LI LU MC NL PT SE**
Designated Extension States:
**AL LT LV MK RO SI**

(30) Priority: **08.10.1999 JP 28890499**

(71) Applicant: **Meiji Seika Kaisha, Ltd.**
**Tokyo 104-8002 (JP)**

(72) Inventors:
- **ISHIKAWA,Minoru Meiji Seika Kaisha,Ltd. Phar.Res.C Kanagawa 222-8567 (JP)**
- **KUBOTA,Dai Meiji Seika Kaisha,Ltd. Pharma.Res.Cen. Kanagawa 222-8567 (JP)**
- **HIRAIWA,Yukiko Meiji Seika Kaisha,Ltd. Phar.Res.Ce Kanagaw a 222-8567 (JP)**
- **TSUSHIMA,Masaki Meiji Seika Kaisha,Ltd. Phar.Res.C Kanagawa 222-8567 (JP)**
- **YAMAMOTO,Mikio Meiji Seika Kaisha,Ltd. Pharm.Res.C Kanagaw a 222-8567 (JP)**
- **YAHATA,Naokazu Meiji Seika Kaisha,Ltd. Pharm.Res.C Kanagaw a 222-8567 (JP)**
- **KURODA,Chizuko Meiji Seika Kaisha,Ltd. Pharm.Res.C Kanagaw a 222-8567 (JP)**
- **ABE,Mitsuhiro Meiji Seika Kaisha,Ltd. Pharm.Res.Ce Kanagawa 222-8567 (JP)**
- **FUJISHIMA,Kazuyuki Meiji Seika Kaisha,Ltd. Phar.Re Kanagawa 222-8567 (JP)**
- **MURAKAMI,Shoichi Meiji Seika Kaisha,Ltd. Phar.Res. Kanagawa 222-8567 (JP)**
- **AJITO,Keiichi Meiji Seika Kaisha,Ltd. Phar.Res.Cen Kanagawa 222-8567 (JP)**

(74) Representative: **Hall, Marina**
**Elkington and Fife**
**Prospect House**
**8 Pembroke Road**
**Sevenoaks Kent TN13 1XR (GB)**

(54) **3-AMINOPIPERIDINE DERIVATIVES AS INTEGRIN $g(a)v$g(b)3 ANTAGONISTS**

(57) An object of the present invention is to provide novel derivatives having integrin $\alpha_v\beta_3$ antagonistic activity wherein a basic atomic group has been attached to the 3-position of a piperidine ring either directly or through various atomic groups. The derivatives according to the present invention are compounds represented by formula (I) or pharmaceutically acceptable salts or solvates thereof, which are useful for the treatment or prevention of cardiovascular diseases, angiogenesis-related diseases, cerebrovascular diseases, cancers and metastasis thereof, immunological diseases, osteopathy and other diseases:

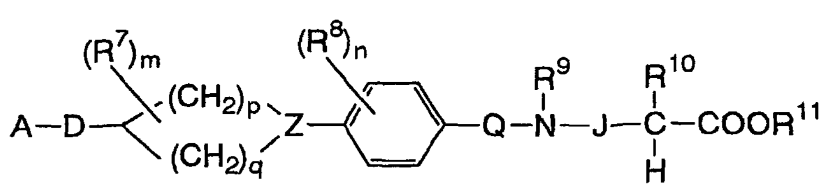

(I)

EP 1 227 083 A1

wherein A represents an optionally substituted heterocyclic group containing at least one nitrogen atom, a bicylic group or the like; D represents a bond, >$NR^4$, >$CR^5R^6$, O, S, or -$NR^4$-$CR^5R^6$-; Z represents CH or N; $R^7$ and $R^8$ represent hydroxyl, alkyl or the like; Q represents >C=O or the like; $R^9$ represents hydrogen, alkyl or the like; J represents a bond or alkylene; $R^{10}$ represents optionally substituted hydroxyl, amino or the like; $R^{11}$ represents hydrogen, alkyl or the like; m is 0 to 5; n is 0 to 4; p is 3 or 4; and q is 0 to 3.

## Description

BACKGROUND OF THE INVENTION

Field of the Invention

The present invention relates to 3-aminopiperidine derivatives having integrin $\alpha_v\beta_3$ antagonistic activity and pharmaceuticals comprising the same.

Description of Related Art

A signal transmission system is very important to organisms from the viewpoints of physiological significance, the regulation of gene expression and the like. It has been clarified that integrins, i.e., glycoprotein receptors which are involved in cell adhesion and penetrate cell membranes, are related, for example, to wound healing and hemostasis, phagocytosis, biophylaxis, and the construction of cytoskeletons and, in addition, as such are signal transfer molecules (Cell, 69, 11, (1992)). For this reason, in recent years, organic chemistry associated with integrins has suddenly become drawn attention from the viewpoint of pharmacology, as well as from the viewpoints of molecular biology and cell biology.

It is being elucidated that, while the conformation of integrins undergoes a dynamic and complicate change, integrins bind to various ligands to transmit signal in both intracellular and extracellular directions (Junichi Takagi et al., The 50th Annual Meeting of the Japan Society for Cell Biology, S5-1, 1997). T. A. Springer of Harvard Medical School has recently predicted that a certain activated integrin has a β-propeller structure and binds to a ligand on the upper face of the β-propeller (Proc. Natl. Acad. Sci. USA, 94, 65, 1997). This hypothesis was also supported by researchers in Japan (Atsushi Irie et al., The 50th Annual Meeting of the Japan Society for Cell Biology, S5-2, 1997), and three-dimensional analysis on a molecular level associated with the activation of integrins as well as binding between integrins and ligands and the like has been initiated in real earnest. T. A. Springer et al. have recently substantiated a hypothesis regarding the β-propeller domain by experimentation, and have suggested that the β-propeller domain in integrin α-subunit has important interaction with integrin β-subunit (Proc. Natl. Acad. Sci. USA, 95, 4870, 1998).

Among others, integrin $\alpha_v\beta_3$ binds to various extracellular matrixes, that is, ligands deeply involved, for example, in biodynamics or the crisis of diseases, such as vitronectin, fibrinogen, fibronectin, osteopontin, thrombospondin, von Willebrand factors, and collagen, to form complexes. Accordingly, integrin $\alpha_v\beta_3$ is of special interest as a potential drug target (DN & P, 10, 456, 1997). In fact, $\alpha_v\beta_3$ is expressed in a large amount in B cells, macrophages, monocytes, smooth muscle, activated endothelial cells and the like. Among others, biological functions of integrin $\alpha_v\beta_3$ around blood vessels are important, and it has become clear that $\alpha_v\beta_3$ is deeply involved in the growth, adhesion, and migration of vascular endothelial cells and vascular smooth muscle cells (T. V. Byzova et al., Thromb Haemost, 80, 726 (1998)). Further, $\alpha_v\beta_3$ is known not to be strongly expressed in endothelial cells in a resting stage, but to be highly activated in the course of growth and infiltration, that is, in vascularization, wound healing, and inflamed sites. Further, the correlation between the frequency of expression of $\alpha_v\beta_3$ and the increase in infiltration of cancer has been observed in various cancer cells. On the other hand, a group of researchers at Scripps Research Institute in the United State have clarified by advanced computer-assisted video imaging microscopy that microvascular expression of $\alpha_v\beta_3$ is observed during experimental middle cerebral artery occlusion and reperfusion in a baboon as a model (Y. Okada et al., Am. J. Pathol., 149, 37, 1996).

As described above, relationship of cell species, which express integrin $\alpha_v\beta_3$ in vivo, with $\alpha_v\beta_3$ activation stage, biophylaxis mechanism and the like has led to an expectation of clinical application of molecules having integrin $\alpha_v\beta_3$ antagonistic activity in various fields. In fact, compounds having integrin $\alpha_v\beta_3$ antagonistic activity are intended to be used clinically, and the results of animal tests on compounds having $\alpha_v\beta_3$ antagonistic activity in a wide range of diseases have been reported (S. S. Srivatsa et al., The 69th Annual Meeting of American Heart Association, 0231, 1996 (DuPont-Merc); J. F. Gourvest et al., The 18th Annual Meeting of The American Society for Bone and Mineral Research, p228, 1996 (Roussel-Hoechst); S. B. Rodan et al., The 18th Annual Meeting of The American Society for Bone and Mineral Research, M430, 1996 (Merck); T. L. Yue et al., The 70th Annual Meeting of American Heart Association, 3733, 1997 (SmithKline Beecham); A. L. Racanelli et al., The 70th Annual Meeting of American Heart Association, 3734, 1997 (DuPont-Merc); M. Friedlander et al., Conference of American IBC, September 11, 1997 (The Scripps Research Institute); W. S. Westlin, Conference of American IBC, February 23, 1998 (Searle); M. W. Lark et al., The 2nd Joint Conference of The American Society for Bone and Mineral Research and International Bone and Mineral Society, T064, 1998 (SmithKline Beecham); R. K. Keenan et al., Bioorg. Med. Chem. Lett., 8, 3171, 1998 (SmithKline Beecham); C. P. Carron et al., Cancer Res., 58, 1930, 1998 (Searle); W. H. Miller et al., Bioorg. Med. Chem. Lett., 9, 1807, 1999 (SmithKline Beecham); and S. A. Mousa et al., The 17th International Congress on Thrombosis and Hemostasis, 228, 1999 (DuPont Pharmaceuticals)).

From the viewpoint of chemical structure, compounds having integrin $\alpha_v\beta_3$ antagonistic activity can be classified into antibodies, low-molecular peptide and compounds analogous thereto, and low-molecular organic compounds. All the antagonists are structurally related to the sequence of tripeptide RGD (arginine-glycine-aspartic acid) that is considered indispensable for recognition in the attachment of a ligand. Low-molecular peptides having antagonistic activity include disintegrins derived from venom of snakes and, in addition, cyclic peptides. One of them, Gpen-GRGDSPCA, has been reported to inhibit migration of smooth muscle and to block integrin $\alpha_v\beta_3$, thereby to actually inhibit neointima formation in rabbits (E.T. Choi et al., J. Vasc. Surg., 19, 125, 1994). Further, RGD-containing cyclic peptide G4120 inhibited neointima formation in hamsters (Circulation, 90, 2203 (1994)). Further, Scripps Research Institute has recently reported that cyclic peptides having $\alpha_v\beta_3$ antagonistic activity are promising novel therapeutic agents for rheumatic arthritis (C. M. Storgard et al., J. Clin. Invest., 103, 47 (1999)). On the other hand, cyclic peptides containing BTD designed by a (β-turn mimic have been proved to strongly bind to $\alpha_v\beta_3$ receptors (M. Goodman et al., Bioorg. Med. Chem. Lett., 7, 997, 1997).

Several methods are known for designing small molecules through the utilization of the amino acid sequence of interest (RGD being used here) as a clue (Gen Ojima et al., Journal of The Society of Synthetic Organic Chemistry, 52, 413 (1994); Toshio Furuya, Shin-Tanpakushitu Oyo Kogaku, Fujitec Corporation). A peptide mimesis for constructing a new molecule based on the backbone of a peptide chain is generally known in the art. The concept of a new de novo design focused on the chemical structure and spatial configuration of amino acid side chains has been introduced for the first time early in the 1990s (R. Hirschman et al., J. Am. Chem. Soc., 115, 12550 (1993)). An attempt to apply this approach to the design and synthesis of $\alpha_v\beta_3$ antagonists has already been initiated (K.C. Nicolaou et al., Tetrahedron, 53, 8751, 1997).

Up to now, small molecules having $\alpha_v\beta_3$ antagonistic activity are disclosed in WO 95/32710 (Merck); WO 96/37492 (Dupont-Merc); WO 97/01540 (SmithKline Beecham); WO 97/08145 (Searle); WO 97/23451 (Merck); WO 97/23480 (Dupont-Merc); WO 97/24119 (SKB); WO 97/26250 (Merck); WO 97/33887 (Dupont-Merc); WO 97/36858 (Searle); WO 97/36859 (Searle); WO 97/36860 (Searle); WO 97/36861 (Searle); WO 97/36862 (Searle); WO 97/24336 (SmithKline Beecham); WO 97/37655 (Merck); WO 98/08840 (Merck); WO 98/18460 (Merck); WO 98/25892 (Lilly); WO 98/30542 (SmithKline Beecham); WO 98/31359 (Merck); WO 98/35949 (Merck); WO 98/43962 (Dupont-Merc); WO 98/46220 (Merck); WO 99/05107 (SmithKline Beecham); WO 99/06049 (SmithKline Beecham); WO 99/11626 (SmithKline Beecham); WO 99/15170 (SmithKline Beecham); WO 99/15178 (SmithKline Beecham); WO 99/15506 (Hoechst Marion Roussel); WO 99/15507 (Hoechst Marion Roussel); WO 99/15508 (SmithKline Beecham); WO 99/30709 (Merck); WO 99/30713 (Merck); WO 99/31061 (Merck); WO 99/31099 (Merck); WO 99/32457 (Hoechst Marion Roussel); WO 99/33798 (Yamanouchi Pharmaceutical Co., Ltd.); WO 99/37621 (Hoechst Marion Roussel); US 5843906 (Searle); US 5852210 (Searle); EP 796855 (Hoechst); EP 820988 (Hoechst); EP 820991 (Hoechst); EP 853084 (Hoechst); EP 928790 (Hoffmann-La Roche Ltd.); EP 928793 (Hoffmann-La Roche Ltd.); GB 2326609 (Merck); GB 2327672 (Merck); R. M. Keenan et al., J. Med. Chem., 40, 2289 (1997); J. W. Corbett et al., Bioorg. Med. Chem. Lett., 7, 1371 (1997); K. C. Nicolaou et al., Bioorg. Med. Chem., 6, 1185 (1998); R. M. Keenan, et al., Bioorg. Med. Chem. Lett., 8, 3165 (1998); A. R. Rockwell et al. Bioorg. Med. Chem. Lett., 9, 937 (1999); R. M. Keenan et al., Bioorg. Med. Chem. Lett., 9, 1801 (1999); and S. A. Mousa et al., J. Cardiovasc. Pharmacol., 33, 641 (1999).

However, small molecules having potent $\alpha_v\beta_3$ antagonistic activity, which have been known up to now, are not always usable in a wide amount range due to their limited solubility. When the administration of these small molecules by oral administration or by application of a liniment to the skin is contemplated, the antagonists are not particularly required to be soluble in water. When the administration of these small molecules, for example, through intraveneous injection, intraveneous drop infusion, or instillation is contemplated, however, the antagonists are preferably soluble in water. When the use of antagonists, which are expected to be used as therapeutic agents for diseases in acute phase, for example, acute myocardial infarction, restenosis after PTCA, unstable angina, cerebral infarction, peripheral infarction diseases, and diabetic retinopathy, is contemplated, highly water-soluble antagonists are desired.

Further, for example, chimeric antibody 7E3 is known to have $\alpha_v\beta_3$ antagonistic activity and $\alpha_{IIb}\beta_3$ antagonistic activity which are substantially identical to each other in activity level (S. H. Tam et al., Circulation, 98, 1085 (1998)). However, it is not easy to chemically modify or chemically convert this chimeric antibody to control the selectivity as desired. When small molecules having $\alpha_v\beta_3$ antagonistic activity and $\alpha_{IIb}\beta_3$ antagonistic activity are used as drugs, the optimal balance of the antagonistic activity against integrins varies depending upon diseases to which the drugs are to be applied. Specifically, in some cases, the optimal ratio of $\alpha_v\beta_3$ antagonistic activity to $\alpha_{IIb}\beta_3$ antagonistic activity is substantially 1 : 1, and, in other cases, the $\alpha_v\beta_3$ antagonistic activity is preferably higher than the $\alpha_{IIb}\beta_3$ antagonistic activity or vice versa. Therefore, when the selectivity to various integrins can be controlled as desired by chemically modifying or chemically converting an identical pharmacophore, this is very beneficial to the creation of drugs. Up to now, the balance between integrin $\alpha_v\beta_3$ antagonistic activity and integrin $\alpha_{IIb}\beta_3$ antagonistic activity has been discussed from the viewpoint of three-dimensional structure of molecules only in G. D. Hartman et al., Bioorg. Med. Chem. Lett., 9, 863 (1999), and M. A. Dechantsreiter et al., J. Med. Chem., 42, 3033 (1999). However, systematic methodology on the balance between integrin $\alpha_v\beta_3$ antagonistic activity and integrin $\alpha_{IIb}\beta_3$ antagonistic activity are not known in the art.

SUMMARY OF THE INVENTION

The present inventors have found that novel compounds have significantly high integrin $\alpha_v\beta_3$ antagonistic activity, have improved $\alpha_v\beta_3$ antagonistic activity in relationship to $\alpha_{IIb}\beta_3$ antagonistic activity, and have improved solubility in water.

An object of the present invention is to provide novel compounds which have integrin $\alpha_v\beta_3$ antagonistic activity, have improved $\alpha_v\beta_3$ antagonistic activity in relationship to $\alpha_{IIb}\beta_3$ antagonistic activity, and, at the same time, have high solubility in water.

According to the present invention, there is provided a compound represented by formula (I) or a pharmaceutically acceptable salt or solvate thereof:

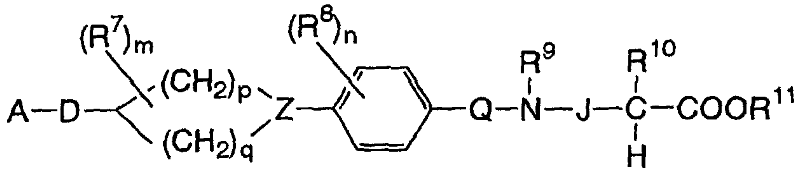

(I)

wherein

A represents a saturated or unsaturated five- to seven-membered heterocyclic group containing at least one nitrogen atom, which is optionally condensed with another saturated or unsaturated five- to seven-membered carbocyclic ring or heterocyclic ring to form a bicyclic group, wherein the heterocyclic group and the bicyclic group are optionally substituted by $C_{1-6}$ alkyl, amino, $C_{2-6}$ alkenyl, hydroxyl, a halogen atom, $C_{1-6}$ alkoxy, $C_{1-6}$ alkoxycarbonyl, or aralkyl and the $C_{1-6}$ alkyl, amino, $C_{2-6}$ alkenyl, hydroxyl, $C_{1-6}$ alkoxy, $C_{1-6}$ alkoxycarbonyl, and aralkyl groups are optionally substituted by $C_{1-6}$ alkyl, $C_{1-6}$ alkoxy, a halogen atom, amino, or hydroxyl, or a group represented by formula

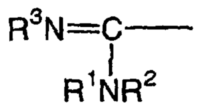

or

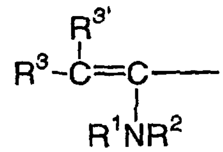

wherein $R^1$, $R^2$, $R^3$, and $R^{3'}$, which may be the same or different, represent a hydrogen atom, $C_{1-6}$ alkyl, $C_{2-6}$ alkenyl, $C_{2-6}$ alkynyl, aralkyl, or nitrile, or $R^1$ and $R^2$ may together form group $-(CH_2)i-$, wherein i represents 4 or 5, or group $-(CH_2)_2-O-(CH_2)_2-$, wherein the $C_{1-6}$ alkyl, $C_{2-6}$ alkenyl, $C_{2-6}$ alkynyl, and aralkyl groups are optionally substituted by a halogen atom, $C_{1-6}$ alkoxy, amino, or hydroxyl;

D represents $>NR^4$ wherein $R^4$ represents a hydrogen atom or $C_{1-6}$ alkyl and this alkyl group is optionally substituted by phenyl optionally substituted by $C_{1-6}$ alkoxy; $>CR^5R^6$ wherein $R^5$ and $R^6$ each independently represent a hydrogen atom or $C_{1-6}$ alkyl and this alkyl group is optionally substituted by phenyl optionally substituted by $C_{1-6}$ alkoxy; -O-; -S-; or $-NR^4-CR^5R^6-$ wherein $R^4$, $R^5$, and $R^6$ are as defined above;

Z represents CH or N;

$R^7$ represents $C_{1-6}$ alkyl, $C_{1-6}$ alkoxy, a halogen atom, amino, nitro, hydroxyl, or an oxygen atom, wherein the $C_{1-6}$ alkyl and $C_{1-6}$ alkoxy groups are optionally substituted by a halogen atom, $C_{1-6}$ alkoxy, amino, or hydroxyl;

$R^8$ represents $C_{1-6}$ alkyl, $C_{1-6}$ alkoxy, a halogen atom, amino, nitro, or hydroxyl, wherein the $C_{1-6}$ alkyl and $C_{1-6}$ alkoxy groups are optionally substituted by a halogen atom, $C_{1-6}$ alkoxy, amino, or hydroxyl;

Q represents $>C=O$, $>CHR^{13}$, or $>CHOR^{13}$ wherein $R^{13}$ represents a hydrogen atom or $C_{1-6}$ alkyl;

$R^9$ represents a hydrogen atom, $C_{1-6}$ alkyl, $C_{2-6}$ alkenyl, $C_{2-6}$ alkynyl, or aralkyl wherein the $C_{1-6}$ alkyl, $C_{2-6}$ alkenyl, $C_{2-6}$ alkynyl, and aralkyl groups are optionally substituted by a halogen atom, $C_{1-6}$ alkoxy, amino, or hydroxyl;

J represents a bond or an alkylene chain having 1 to 3 carbon atoms, wherein one or more hydrogen atoms on the alkylene chain are optionally substituted by the same or different substituent selected from $C_{1-6}$ alkyl, $C_{2-6}$ alkenyl, $C_{2-6}$ alkynyl, aralkyl, hydroxyl, or amino, the $C_{1-6}$ alkyl, $C_{2-6}$ alkenyl, $C_{2-6}$ alkynyl, and aralkyl groups are further optionally substituted by a halogen atom, $C_{1-6}$ alkoxy, amino, or hydroxyl, and the amino group is optionally substituted by $C_{1-6}$ alkyl; $C_{1-6}$ alkoxycarbonyl; benzenesulfonyl of which the phenyl portion is optionally substituted by $C_{1-6}$ alkyl; or benzyloxycarbonyl of which the phenyl portion is optionally substituted by $C_{1-6}$ alkyl;

$R^{10}$ represents a hydrogen atom, hydroxyl, $C_{1-6}$ alkyl, $C_{2-6}$ alkenyl, $C_{2-6}$ alkynyl, aralkyl, or amino, wherein the $C_{1-6}$ alkyl, $C_{2-6}$ alkenyl, $C_{2-6}$ alkynyl, and aralkyl groups are optionally substituted by a halogen atom, $C_{1-6}$ alkoxy, amino, or hydroxyl, the hydroxyl group is optionally substituted by $C_{1-6}$ alkyl, and the amino group is optionally substituted by carboxyl; sulfonyl; $C_{1-6}$ alkyl; $C_{1-6}$ alkylcarbonyl; $C_{1-6}$ alkoxycarbonyl; $C_{1-6}$ alkylsulfonyl; -C(=O)-O-$(CH_2)$u-$R^{14}$ wherein u is an integer of 0 to 4 and $R^{14}$ represents -$OR^{15}$ (wherein $R^{15}$ represents a hydrogen atom or $C_{1-4}$ alkylcarbonyl) or a saturated or unsaturated five- to seven-membered carbocyclic or heterocyclic group and the carbocyclic group and the heterocyclic group are optionally substituted by $C_{1-6}$ alkyl, $C_{1-6}$ alkoxy, phenyl optionally condensed with the carbocyclic group or the heterocyclic group, carboxyl, hydroxyl, nitro, amino, $C_{1-6}$ alkylamino, or a halogen atom; -C(=O)-$R^{14}$ wherein $R^{14}$ is as defined above; or -S(=O)$_2$-$(CH_2)$v-$R^{14}$ wherein v is an integer of 0 to 4 and $R^{14}$ is as defined above;

$R^{11}$ represents a hydrogen atom or $C_{1-6}$ alkyl wherein the $C_{1-6}$ alkyl group is optionally substituted by one or two phenyl groups;

m is an integer of 0 to 5;

n is an integer of 0 to 4;

p is 3 or 4; and

q is an integer of 0 to 3.

The compounds according to the present invention are useful for the treatment or prevention of integrin $\alpha_v\beta_3$-mediated diseases, for example, cardiovascular diseases, angiogenesis-related diseases, cerebrovascular diseases, cancers and metastasis thereof, immunological diseases, and osteopathy.

Another object of the present invention is to provide a process for producing an intermediate useful for the production of the compounds represented by formula (I).

According to the present invention, there is provided a process for producing a compound as an intermediate represented by formula (IX)

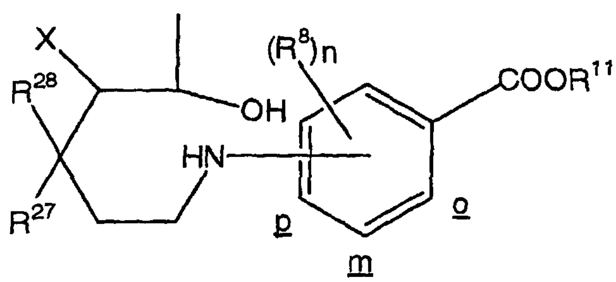

(IX)

wherein $R^{27}$ and $R^{28}$, which may be the same or different, represent a hydrogen atom, hydroxyl, $C_{1-4}$ alkoxy, or $C_{1-4}$ alkyl; X represents hydroxyl, azide, or optionally protected amino; $R^8$, $R^{11}$, and n are as defined in formula (I); and the nitrogen atom is attached to the ortho-, meta-, or para-position of the phenyl group, said process comprising the step of:

reacting a compound represented by formula (X)

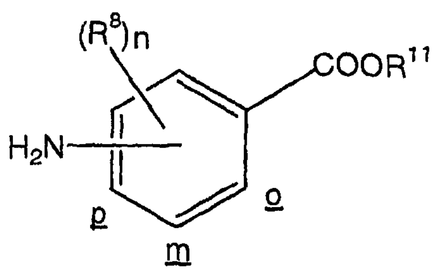

(X)

wherein $R^8$, $R^{11}$, and n are as defined in formula (I); and the nitrogen atom is attached to the ortho-, meta-, or para-position of the phenyl group,
with a compound represented by formula (XI)

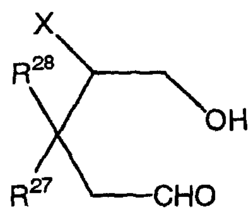

(XI)

wherein X, $R^{27}$, and $R^{28}$ are as defined above.

According to the present invention, there is also provided a process for producing a compound as an intermediate represented by formula (III')

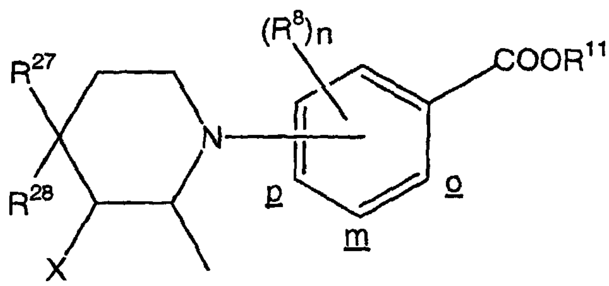

(III')

wherein $R^8$, $R^{11}$, and n are as defined in formula (I); $R^{27}$, $R^{28}$, and X are as defined in formula (IX); and the nitrogen atom is attached to the ortho-, meta-, or para-position of the phenyl group, said process comprising the step of:

cyclizing a compound represented by formula (XII)

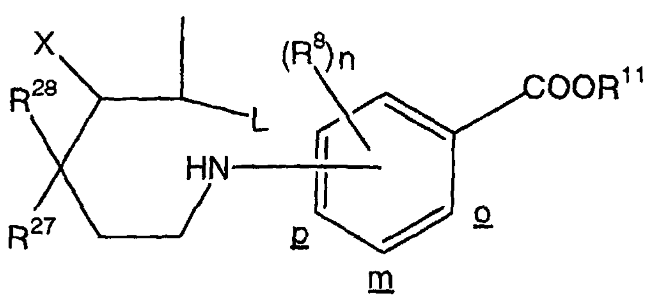

(XII)

wherein $R^8$, $R^{11}$, and n are as defined in formula (I); $R^{27}$, $R^{28}$, and X are as defined in formula (IX); L represents a leaving group; and the nitrogen atom is attached to the ortho-, meta-, or para-position of the phenyl group, by an intramolecular ring-closing reaction.

A further object of the present invention is to provide an intermediate useful for the production of compounds represented by formula (I).

According to the present invention, there is provided an intermediate represented by formula (XIII)

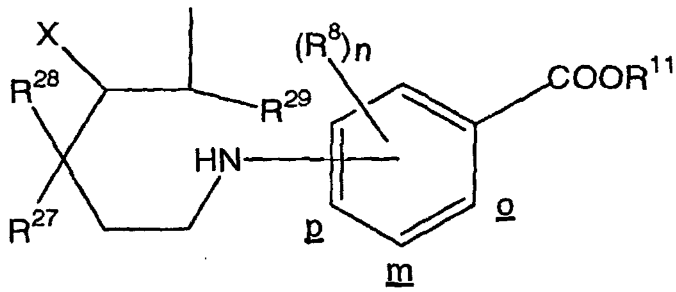

(XIII)

wherein $R^8$, $R^{11}$, and n are as defined in formula (I); $R^{27}$, $R^{28}$, and X are as defined in formula (IX); $R^{29}$ represents hydroxyl or a leaving group; and the nitrogen atom is attached to the ortho-, meta-, or para-position, preferably the para-position, of the phenyl group.

According to the present invention, there is also provided an intermediate represented by formula (III')

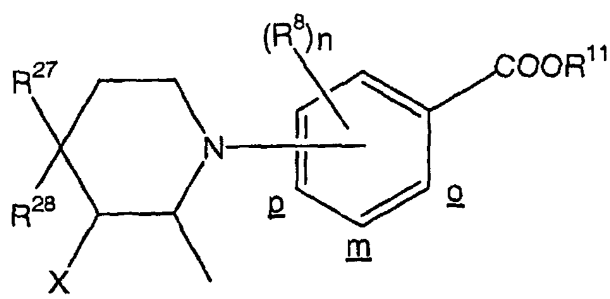

(III')

wherein $R^8$, $R^{11}$, and n are as defined in formula (I); $R^{27}$, $R^{28}$, and X are as defined in formula (IX); and the nitrogen atom is attached to the ortho-, meta-, or para-position, preferably the para-position, of the phenyl group.

## DETAILED DESCRIPTION OF THE INVENTION

### Compound

The term "$C_{1-6}$ alkyl" and "$C_{1-6}$ alkoxy" as used herein as a group or a part of a group respectively mean straight chain, branched chain, or cyclic alkyl and alkoxy having 1 to 6, preferably 1 to 4 carbon atoms.

The term "$C_{2-6}$ alkenyl" and "$C_{2-6}$ alkynyl" as used herein as a group or a part of a group respectively mean straight chain, branched chain, or cyclic alkenyl and alkynyl having 2 to 6, preferably 2 to 4 carbon atoms.

Examples of $C_{1-6}$ alkyl include methyl, ethyl, n-propyl, isopropyl, cyclopropyl, cyclopropylmethyl, n-butyl, i-butyl, s-butyl, t-butyl, n-pentyl, cyclopentyl, n-hexyl, and cyclohexyl.

Examples of $C_{1-6}$ alkoxy include methoxy, ethoxy, n-propoxy, i-propoxy, n-butoxy, i-butoxy, s-butoxy, and t-butoxy.

Examples of $C_{2-6}$ alkenyl include allyl.

Examples of $C_{2-6}$ alkynyl include 2-propynyl and ethynyl.

Examples of "saturated or unsaturated five- to seven-membered carbocyclic groups" include phenyl.

The term "saturated or unsaturated five- to seven-membered heterocyclic ring" as used herein means a five- to seven-membered heterocyclic ring containing at least one hetero-atom selected from oxygen, nitrogen, and sulfur atoms, preferably a five- to seven-membered heterocyclic ring containing one or two nitrogen atoms, more preferably a five- or six-membered heterocyclic ring containing one or two nitrogen atoms. The term "hetero-atom" used herein means an oxygen, nitrogen, or sulfur atom. Examples of saturated or unsaturated five-to seven-membered heterocyclic groups include pyridyl, pyrimidyl, 1,4,5,6-tetrahydropyrimidyl, imidazolyl, tetrahydro-[1,3]diazepinyl, imidazolidinyl, thiophenyl, and morpholyl.

The saturated or unsaturated heterocyclic group may be condensed with other saturated or unsaturated heterocyclic ring to form a bicyclic ring. Such condensed cyclic groups include benzimidazolyl, naphthyl, and azabenzimidazolyl, for example, imidazo[4,5-b]pyridyl.

The term "aralkyl" as used herein as a group or a part of a group means $C_{1-6}$ alkyl, preferably $C_{1-4}$ alkyl, substituted by a saturated or unsaturated five- to seven-membered carbocyclic group or heterocyclic group. Examples of aralkyl include benzyl and phenethyl.
The term "halogen atom" means a fluorine, chlorine, bromine, or iodine atom.
D preferably represents >NH, >$CH_2$, or -NH-$CH_2$-.
Z preferably represents N.
When D represents >$NR^4$, Z preferably represents N.
When D represents -$NR^4$-$CR^5R^6$-, Z preferably represents N.
The "saturated or unsaturated five- to seven-membered heterocyclic group containing at least one nitrogen atom" represented by A is preferably a saturated or unsaturated five- to seven-membered heterocyclic group containing one or two nitrogen atoms, more preferably a saturated or unsaturated five- or six-membered heterocyclic group containing one or two nitrogen atoms.
The "bicyclic group" represented by A is preferably a nine- or ten-membered heterocyclic group, more preferably a nine- or ten-membered heterocyclic group containing two or three nitrogen atoms.
In A, $R^2$ and $R^{3'}$ preferably represent a hydrogen atom.
A preferably represents a group of formula

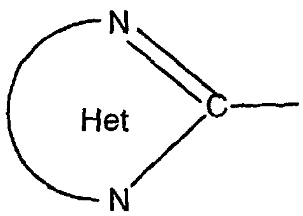

or

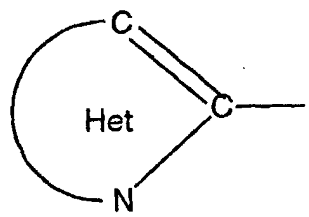

wherein

Het represents a saturated or unsaturated five- to seven-membered heterocyclic group containing at least one nitrogen atom, which is optionally condensed with another saturated or unsaturated five- to seven-membered carbocyclic ring or heterocyclic ring to form a bicyclic group, wherein the heterocyclic group and the bicyclic group are optionally substituted by $C_{1-6}$ alkyl, amino, $C_{2-6}$ alkenyl, hydroxyl, a halogen atom, $C_{1-6}$ alkoxy, $C_{1-6}$ alkoxycarbonyl, or aralkyl and the $C_{1-6}$ alkyl, amino, $C_{2-6}$ alkenyl, hydroxyl, $C_{1-6}$ alkoxy, $C_{1-6}$ alkoxycarbonyl, and aralkyl groups are optionally substituted by $C_{1-6}$ alkyl, $C_{1-6}$ alkoxy, a halogen atom, amino, or hydroxyl.

More preferably, A represents a group of formula

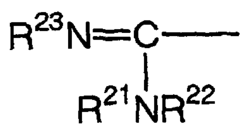

or

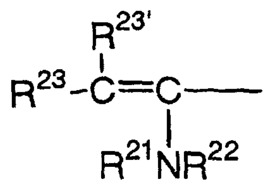

wherein

$R^{21}$, $R^{22}$, $R^{23}$, and $R^{23'}$, which may be the same or different, represent a hydrogen atom, $C_{1-6}$ alkyl, $C_{1-6}$ alkoxy, hydroxyl, $C_{1-6}$ alkoxycarbonyl, $C_{2-6}$ alkenyl, or aralkyl and the $C_{1-6}$ alkyl, $C_{1-6}$ alkoxy, hydroxyl, $C_{1-6}$ alkoxycarbonyl, $C_{2-6}$ alkenyl, and aralkyl groups are optionally substituted by a halogen atom, $C_{1-6}$ alkoxy, amino, $C_{1-6}$ alkyl, or hydroxyl, or
$R^{21}$ and $R^{23}$ may together form

group -$(CH_2)_4$-,
group -$(CH_2)_3$-,
group -$CHR^{24}CH_2CH_2$- wherein $R^{24}$ represents $C_{1-6}$ alkyl, amino, or hydroxyl and the amino and hydroxyl groups are optionally substituted by $C_{1-6}$ alkyl, $C_{1-6}$ alkoxycarbonyl, aralkyl, or aralkyloxycarbonyl,
group -$CH_2CHR^{24}CH_2$- wherein $R^{24}$ is as defined above,
group -$CH_2CH_2$-,

group $-CHR^{24}CH_2-$ wherein $R^{24}$ is as defined above,
group $-CR^{25}=CR^{26}-$ wherein $R^{25}$ and $R^{26}$, which may be the same or different, represent a hydrogen atom or $C_{1-6}$ alkyl, or $R^{25}$ and $R^{26}$ may together form -CH=CH-CH=CH-, $-CR^{24}=CH-CH=CH-$ wherein $R^{24}$ is as defined above, $-CH=CR^{24}-CH=CH-$ wherein $R^{24}$ is as defined above, -N=CH-CH=CH-, or -CH=N-CH=CH-, or

$R^{21}$ and $R^{23}$ may together form

=CH-CH=CH-,
=CH-CH=N-, or
=CH-N=CH-, and

$R^{22}$ may represent a single bond between $R^{21}$ and the nitrogen atom attached to $R^{21}$.
$R^{22}$ and $R^{23'}$ preferably represent a hydrogen atom.

In the compound represented by formula (I), one or more hydrogen atoms in the following portion may be substituted by $R^7$.

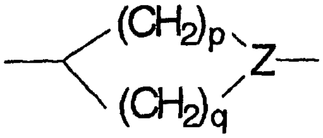

When m is zero (0), $R^7$ is absent. When m is 1, one hydrogen atom in the above portion is substituted by $R^7$. When m is 2 or more, two or more hydrogen atoms in the above portion are substituted by $R^7$. In this case, the substituents may be the same or different. When $R^7$ represents an oxygen atom, the bond between the $R^7$ and the above portion is a double bond. m is preferably an integer of 0 to 2.

In the compound represented by formula (I), one or more hydrogen atoms in the phenylene portion may be substituted by $R^8$.

When n is zero (0), $R^8$ is absent. When n is 1, one hydrogen atom in the phenylene portion is substituted by $R^8$. When n is 2 or more, two or more hydrogen atoms in the phenylene portion are substituted by $R^8$. In this case, the substituents may be the same or different. n is preferably an integer of 0 to 2.

Q preferably represents >C=O or $>CH_2$.

$R^9$ preferably represents a hydrogen atom, $C_{1-6}$ alkyl (preferably, methyl, propyl, cyclopropylmethyl), or aralkyl (preferably benzyl or phenethyl).

J preferably represents a methylene chain or an ethylene chain, more preferably a methylene chain.

One or more hydrogen atoms on the alkylene chain represented by J may be substituted. The substituent is preferably $C_{2-6}$ alkynyl or optionally substituted amino, more preferably $C_{2-6}$ alkynyl.

$R^{10}$ preferably represents a hydrogen atom, $C_{2-6}$ alkynyl, hydroxyl, optionally substituted hydroxyl, or optionally substituted amino, more preferably a hydrogen atom, hydroxyl, or optionally substituted amino.

Hydrogen atoms in the amino group represented by $R^{10}$ may be substituted by two substituents which may be the same or different.

Preferred examples of $-C(=O)-O-(CH_2)u-R^{14}$, which is a substituent for the amino group represented by $R^{10}$, include groups wherein u is an integer of 0 to 3 (more preferably 0 or 1) and $R^{14}$ represents a five- to seven-membered carbocyclic group (more preferably phenyl).

Preferred examples of $-S(=O)_2-(CH_2)v-R^{14}$, which is a substituent for the amino group represented by $R^{10}$, include groups wherein v is an integer of 0 to 3 (more preferably 0 or 1) and $R^{14}$ represents a five- to seven-membered carbocyclic group (more preferably phenyl) or a five- to seven-membered heterocyclic group (more preferably pyridyl, thiophenyl, or morpholyl).

Preferred additional examples of $-S(=O)_2-(CH_2)v-R^{14}$, which is a substituent for the amino group represented by $R^{10}$, include groups wherein v is an integer of 1 to 4 and $R^{14}$ represents $-OR^{15}$ (more preferably hydroxyl or acetyloxy).

One or more hydrogen atoms in the carbocyclic group and the heterocyclic group represented by $R^{14}$ are preferably optionally substituted by $C_{1-6}$ alkyl (more preferably methyl), $C_{1-6}$ alkoxy (more preferably methoxy), carboxyl, hydroxyl, nitro, amino, or a halogen atom.

The substituent of the amino group represented by $R^{10}$ is preferably $C_{1-6}$ alkyl; acetyl; $C_{1-6}$ alkylcarbonyl; $C_{1-6}$ alkylsulfonyl; benzoyl or benzyloxycarbonyl of which the phenyl portion is optionally substituted by $C_{1-6}$ alkyl, $C_{1-6}$

alkoxy, carboxyl, hydroxyl, nitro, amino, or a halogen atom; or $-S(=O)_2-(CH_2)v-R^{14}$ wherein v is an integer of 0 to 4 and $R^{14}$ represents -OH, -OAc (wherein Ac represents acetyl), phenyl optionally substituted by $C_{1-6}$ alkyl, $C_{1-6}$ alkoxy, carboxyl, hydroxyl, nitro, amino, or a halogen atom, pyridyl, thiophenyl, or morpholyl.

The $C_{1-6}$ alkyl group represented by $R^{11}$ is preferably methyl, ethyl, t-butyl, or diphenylmethyl.

p is preferably 3 or 4.
q is preferably 0 or 1.

When p is 3, q is preferably 0 or 1.

When p is 4, q is preferably 1.

Preferred compounds represented by formula (I) are those wherein

A represents a group of formula

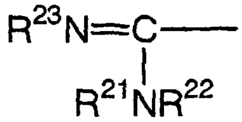

or

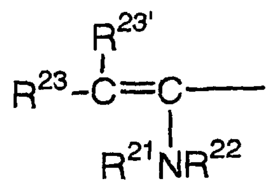

wherein

$R^{21}$, $R^{22}$, $R^{23}$, and $R^{23'}$ are as defined above;
D represents >NH, or $-NH-CH_2-$;
Z represents N;
Q represents >C=O or $>CH_2$;
$R^9$ represents a hydrogen atom, $C_{1-6}$ alkyl or aralkyl wherein the $C_{1-6}$ alkyl and aralkyl groups are optionally substituted by a halogen atom, $C_{1-6}$ alkoxy, amino, or hydroxyl;
J represents a methylene chain optionally substituted by $C_{2-6}$ alkynyl;
$R^{10}$ represents a hydrogen atom, hydroxyl, or amino, wherein the hydroxyl group is optionally substituted by $C_{1-6}$ alkyl and the amino group is optionally substituted by $C_{1-6}$ alkyl; acetyl; $C_{1-6}$ alkylcarbonyl; $C_{1-6}$ alkylsulfonyl; benzoyl or benzyloxycarbonyl of which the phenyl portion is optionally substituted by $C_{1-6}$ alkyl, $C_{1-6}$ alkoxy, carboxyl, hydroxyl, nitro, amino, or a halogen atom; or $-S(=O)_2-(CH_2)v-R^{14}$ wherein v is an integer of 0 to 4 and $R^{14}$ represents -OH, -OAc (wherein Ac represents acetyl), phenyl optionally substituted by $C_{1-6}$ alkyl, $C_{1-6}$ alkoxy, carboxyl, hydroxyl, nitro, amino, or a halogen atom, pyridyl, thiophenyl, or morpholyl;
m and n are each an integer of 0 to 2;
p is 3 or 4; and
q is 0 or 1.

Preferred compounds represented by formula (I) are the following compounds:

1. t-butyl (2S)-benzenesulfonylamino-3-[4-{3-(pyrimidin-2-ylamino)piperidin-1-yl}benzoylamino]-propionate;
2. (2S)-benzenesulfonylamino-3-[4-{3-(pyrimidin-2-ylamino)piperidin-1-yl}benzoylamino]propionic acid;
3. (2S)-benzenesulfonylamino-3-[4-{3-(1,4,5,6-tetrahydropyrimidin-2-ylamino)piperidin-1-yl}benzoyl-amino]propionic acid;
4. t-butyl (2S)-benzenesulfonylamino-3-[3-fluoro-4 -{(3S)-(pyrimidin-2-ylamino)piperidin-1-yl}benzoylamino]propionate;
5. (2S)-benzenesulfonylamino-3-[3-fluoro-4-{(3S)-(pyrimidin-2-ylamino)piperidin-1-yl}benzoylamino]-propionic acid;
6. (2S)-benzenesulfonylamino-3-[3-fluoro-4-{(3S)-(1,4,5,6-tetrahydropyrimidin-2-ylamino)piperidin-1-yl}-benzoylamino]propionic acid;
7. t-butyl (2S)-benzenesulfonylamino-3-[3-fluoro-4 -{(3R)-(pyrimidin-2-ylamino)piperidin-1-yl}benzoylamino]propionate;
8. (2S)-benzenesulfonylamino-3-[3-fluoro-4-{(3R)-(pyrimidin-2-ylamino)piperidin-1-yl}benzoylamino]-propionic acid;
9. (2S)-benzenesulfonylamino-3-[3-fluoro-4-{(3R)-(1,4,5,6-tetrahydropyrimidin-2-ylamino)piperidin-1-yl}-benzoylamino]propionic acid;

10. t-butyl (2S)-(benzyloxycarbonyl)amino-3-[3-fluoro-4-{(3S)-(pyrimidin-2-ylamino)piperidin-1-yl}-benzoylamino] propionate;
11. t-butyl (2S)-amino-3-[3-fluoro-4-{(3S)-(pyrimidin-2-ylamino)piperidin-1-yl}benzoylamino]-propionate;
12. t-butyl 3-[3-fluoro-4-{(3S)-(pyrimidin-2-ylamino)piperidin-1-yl}benzoylamino]-(2S)-{(4-methoxy-benzenesulfonyl)amino}propionate;
13. 3-[3-fluoro-4-{(3S)-(pyrimidin-2-ylamino)-piperidin-1-yl}benzoylamino]-(2S)-{(4-methoxybenzene-sulfonyl) amino}propionic acid;
14. 3-[3-fluoro-4-{(3S)-(1,4,5,6-tetrahydropyrimidin-2-ylamino)piperidin-1-yl}benzoylamino]-(2S)-{(4-methoxybenzenesulfonyl)amino}propionic acid;
15. 3-[3-fluoro-4-{(3S)-(pyrimidin-2-ylamino)-piperidin-1-yl}benzoylamino]-(2S)-{(4-hydroxybenzene-sulfonyl) amino}propionic acid;
16. 3-[3-fluoro-4-{(3S)-(1,4,5,6-tetrahydropyrimidin-2-ylamino)piperidin-1-yl}benzoylamino]-(2S)-{(4-hydroxybenzenesulfonyl)amino}propionic acid;
17. t-butyl (2S)-benzenesulfonylamino-3-[2,3-difluoro-4-{(3S)-(pyrimidin-2-ylamino)piperidin-1-yl}-benzoylamino] propionate;
18. (2S)-benzenesulfonylamino-3-[2,3-difluoro-4-{(3S)-(pyrimidin-2-ylamino)piperidin-1-yl}benzoylamino]-propionic acid;
19. (2S)-benzenesulfonylamino-3-[2,3-difluoro-4-{(3S)-(1,4,5,6-tetrahydropyrimidin-2-ylamino)piperidin-1 -yl}benzoylamino]propionic acid;
20. t-butyl (2S)-benzenesulfonylamino-3-[3-chloro-4-{(3S)-(pyrimidin-2-ylamino)piperidin-1-yl}benzoyl-amino]propionate;
21. (2S)-benzenesulfonylamino-3-[3-chloro-4-{(3S)-(pyrimidin-2-ylamino)piperidin-1-yl}benzoylamino]-propionic acid;
22. (2S)-benzenesulfonylamino-3-[3-chloro-4-{(3S)-(1,4,5,6-tetrahydropyrimidin-2-ylamino)piperidin-1-yl}-benzoylamino]propionic acid;
23. t-butyl (2S)-benzenesulfonylamino-3-[4-{(3S)-(pyrimidin-2-ylamino)piperidin-1-yl}benzoylamino]-propionate;
24. (2S)-benzenesulfonylamino-3-[4-{(3S)-(pyrimidin-2-ylamino)piperidin-1-yl}benzoylamino]-propionic acid;
25. (2S)-benzenesulfonylamino-3-[4-{(3S)-(1,4,5,6-tetrahydropyrimidin-2-ylamino)piperidin-1-yl}benzoyl-amino] propionic acid;
26. t-butyl (2S)-benzenesulfonylamino-3-[4-{(3S)-(pyridin-2-ylamino)piperidin-1-yl}benzoylamino]-propionate;
27. (2S)-benzenesulfonylamino-3-[4-{(3S)-(pyridin-2-ylamino)piperidin-1-yl}benzoylamino]-propionic acid;
28. t-butyl (2S)-benzenesulfonylamino-3-[2-fluoro-4-{(3S)-(pyrimidin-2-ylamino)piperidin-1-yl}benzoyl-amino]propionate;
29. (2S)-benzenesulfonylamino-3-[2-fluoro-4-{(3S)-(pyrimidin-2-ylamino)piperidin-1-yl}benzoylamino]-propionic acid;
30. (2S)-benzenesulfonylamino-3-[2-fluoro-4-{(3S)-(1,4,5,6-tetrahydropyrimidin-2-ylamino)piperidin-1-yl}-benzoylamino]propionic acid;
31. t-butyl (2S)-benzenesulfonylamino-3-[2-chloro-4-{(3S)-(pyrimidin-2-ylamino)piperidin-1-yl}benzoyl-amino]propionate;
32. (2S)-benzenesulfonylamino-3-[2-chloro-4-{(3S)-(pyrimidin-2-ylamino)piperidin-1-yl}benzoylamino]-propionic acid;
33. (2S)-benzenesulfonylamino-3-[2-chloro-4-{(3S)-(1,4,5,6-tetrahydropyrimidin-2-ylamino)piperidin-1-yl}-benzoylamino]propionic acid;
34. t-butyl (2S)-benzenesulfonylamino-3-[4-{(4S)-methoxy-(2R)-methyl-(3R)-(pyrimidin-2-ylamino)piperidin-1-yl} benzoylamino]propionate;
35. (2S)-benzenesulfonylamino-3-[4-{(4S)-methoxy-(2R)-methyl-(3R)-(pyrimidin-2-ylamino)piperidin-1-yl}-benzoylamino]propionic acid;
36. (2S)-benzenesulfonylamino-3-[4-{(4S)-methoxy-(2R)-methyl-(3R)-(1,4,5,6-tetrahydropyrimidin-2-yl-amino) piperidin-1-yl}benzoylamino]propionic acid;
37. t-butyl (2S)-benzenesulfonylamino-3-[4-{(3S)-(pyrimidin-2-ylamino)piperidin-1-yl}-2-trifluoromethyl-benzoylamino]propionate;
38. (2S)-benzenesulfonylamino-3-[4-{(3S)-(pyrimidin-2-ylamino)piperidin-1-yl}-2-trifluoro-methylbenzoylamino] propionic acid;
39. (2S)-benzenesulfonylamino-3-[4-{(3S)-(1,4,5,6-tetrahydropyrimidin-2-ylamino)piperidin-1-yl}-2-tri-fluoromethylbenzoylamino]propionic acid;
40. t-butyl (2S)-(benzyloxycarbonyl)amino-3-[4-{(3S)-(pyrimidin-2-ylamino)piperidin-1-yl}benzoylamino]-propionate;
41. t-butyl (2S)-amino-3-[4-{(3S)-(pyrimidin-2-ylamino)piperidin-1-yl}benzoylamino]propionate;

42. t-butyl 3-[4-{(3S)-(pyrimidin-2-ylamino)-piperidin-1-yl}benzoylamino]-(2S)-{(thiophene-2-sulfonyl)amino}propionate;
43. 3-[4-{(3S)-(pyrimidin-2-ylamino)piperidin-1-yl}benzoylamino]-(2S)-{(thiophene-2-sulfonyl)amino}-propionic acid;
44. 3-[4-{(3S)-(1,4,5,6-tetrahydropyrimidin-2-ylamino)piperidin-1-yl}benzoylamino]-(2S)-{(thiophene-2-sulfonyl) amino}propionic acid;
51. t-butyl (2S)-(pyridine-3-sulfonyl)amino-3-[4-{(3S)-(pyrimidin-2-ylamino)piperidin-1-yl}benzoylamino]-propionate;
52. (2S)-(pyridine-3-sulfonyl)amino-3-[4-{(3S)-(pyrimidin-2-ylamino)piperidin-1-yl}benzoylamino]-propionic acid;
53. (2S)-(pyridine-3-sulfonyl)amino-3-[4-{(3S)-(1,4,5,6-tetrahydropyrimidin-2-ylamino)piperidin-1-yl}-benzoylamino]propionic acid;
54. t-butyl (2S)-(butane-1-sulfonyl)amino-3-[4-{(3S)-(pyrimidin-2-ylamino)piperidin-1-yl}benzoylamino]-propionate;
55. (2S)-(butane-1-sulfonyl)amino-3-[4-{(3S)-(pyrimidin-2-ylamino)piperidin-1-yl}benzoylamino]-propionic acid;
56. (2S)-(butane-1-sulfonyl)amino-3-[4-{(3S)-(1,4, 5,6-tetrahydropyrimidin-2-ylamino)piperidin-1-yl}-benzoylamino]propionic acid;
57. t-butyl (2S)-benzoylamino-3-[4-{(3S)-(pyrimidin-2-ylamino)piperidin-1-yl}benzoylamino]-propionate;
58. (2S)-benzoylamino-3-[4-{(3S)-(pyrimidin-2-ylamino)piperidin-1-yl}benzoylamino]propionic acid;
59. (2S)-benzoylamino-3-[4-{(3S)-(1,4,5,6-tetra-hydropyrimidin-2-ylamino)piperidin-1-yl}benzoylamino]-propionic acid;
60. t-butyl (2S)-(3-acetoxypropane-1-sulfonyl)-amino-3-[4-{(3S)-(pyrimidin-2-ylamino)piperidin-1-yl}-benzoylamino]propionate;
61. (2S)-(3-acetoxypropane-1-sulfonyl)amino-3-[4-{(3S)-(pyrimidin-2-ylamino)piperidin-1-yl}benzoylamino]-propionic acid;
62. (2S)-(3-acetoxypropane-1-sulfonyl)amino-3-[4-{(3S)-(1,4,5,6-tetrahydropyrimidin-2-ylamino)piperidin-1 -yl} benzoylamino]propionic acid;
63. t-butyl (2S)-(3-hydroxypropane-1-sulfonyl)-amino-3-[4-{(3S)-(pyrimidin-2-ylamino)piperidin-1-yl}-benzoylamino]propionate;
64. (2S)-(3-hydroxypropane-1-sulfonyl)amino-3-[4-{(3S)-(pyrimidin-2-ylamino)piperidin-1-yl}benzoylamino]-propionic acid;
65. (2S)-(3-hydroxypropane-1-sulfonyl)amino-3-[4-{(3S)-(1,4,5,6-tetrahydropyrimidin-2-ylamino)piperidin-1 -yl} benzoylamino]propionic acid;
66. t-butyl (2S)-(morpholine-4-sulfonyl)amino-3-[4 -{(3S)-(pyrimidin-2-ylamino)piperidin-1-yl}benzoylamino]propionate;
69. benzhydryl (2S)-hydroxy-3-[4-{(3S)-(pyrimidin-2-ylamino)piperidin-1-yl}benzoylamino]propionate;
70. (2S)-hydroxy-3-[4-{(3S)-(pyrimidin-2-ylamino)-piperidin-1-yl}benzoylamino]propionic acid;
71. (2S)-hydroxy-3-[4-{(3S)-(1,4,5,6-tetrahydropyrimidin-2-ylamino)piperidin-1-yl}benzoylamino]-propionic acid;
72. t-butyl (2S)-benzenesulfonylamino-3-[4-{(3S)-(pyrimidin-2-ylamino)azepan-1-yl}benzoylamino]-propionate;
73. (2S)-benzenesulfonylamino-3-[4-{(3S)-(pyrimidin-2-ylamino)azepan-1-yl}benzoylamino]-propionic acid;
74. (2S)-benzenesulfonylamino-3-(4-{(3S)-(1,4,5,6-tetrahydropyrimidin-2-ylamino)azepan-1-yl}benzoylamino]-propionic acid;
75. t-butyl (2S)-benzenesulfonylamino-3-[4-{(3S)-guanidinopiperidin-1-yl}benzoylamino]propionate;
76. (2S)-benzenesulfonylamino-3-[4-{(3S)-guanidinopiperidin-1-yl}benzoylamino]propionic acid;
77. t-butyl (2S)-benzenesulfonylamino-3-[4-{(3S)-(6-hydroxypyridin-2-ylamino)piperidin-1-yl}benzoylamino]propionate;
78. (2S)-benzenesulfonylamino-3-[4-{(3S)-(6-hydroxypyridin-2-ylamino)piperidin-1-yl}benzoylamino]-propionic acid;
89. t-butyl (2S)-benzenesulfonylamino-3-[4-[2-{(pyrimidin-2-ylamino)methyl}pyrrolidin-1-yl]benzoylamino]propionate;
90. (2S)-benzenesulfonylamino-3-[4-[2-{(pyrimidin-2-ylamino)methyl}pyrrolidin-1-yl]benzoylamino]propionic acid; and
91. (2S)-benzenesulfonylamino-3-[4-[2-{(1,4,5,6-tetrahydropyrimidin-2-ylamino)methyl}pyrrolidin-1-yl]-benzoylamino]propionic acid.

The compounds according to the present invention may form pharmacologically acceptable salts thereof. Such salts include non-toxic salts. Preferred salts include: hydrohalogenic acid salts such as hydrochloride salts, hydrobromide salts, or hydroiodide salts; inorganic acid salts such as nitric acid salts, perchloric acid salts, sulfuric acid salts, or phosphoric acid salts; lower alkylsulfonic acid salts such as methanesulfonic acid salts, trifluoromethanesulfonic acid salts, or ethanesulfonic acid salts; arylsulfonic acid salts such as benzenesulfonic acid salts or p-toluenesulfonic acid salts; organic acid salts such as fumaric acid salts, succinic acid salts, citric acid salts, tartaric acid salts, oxalic acid salts, or maleic acid salts; amino acid salts such as glutamic acid salts or aspartic acid salts; alkali metal or alkaline earth metal salts such as sodium salts, potassium salts, or calcium salts; and organic alkali salts such as pyridine salts or triethylamine salts.

The compounds according to the present invention may form solvates (for example, hydrates; alcoholates, such as methanolates and ethanolates; and etherates).

Production of compounds

Compounds represented by formula (I), wherein Z represents N, according to the present invention may be produced according to the following scheme.

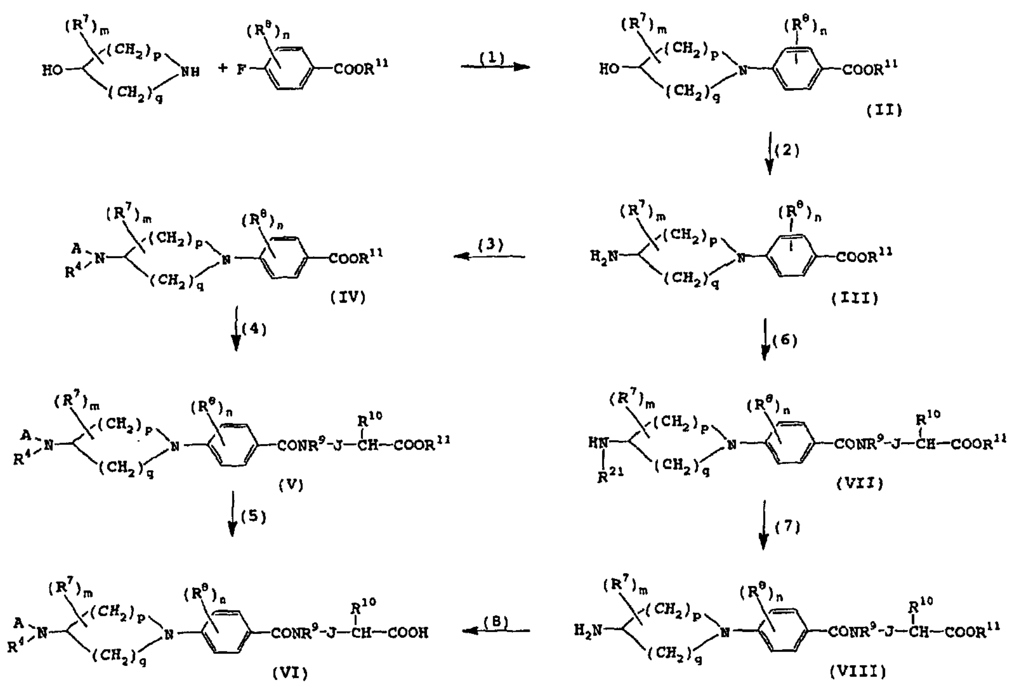

wherein $R^{21}$ represents a protective group for amino; and A, J, $R^4$, $R^7$, $R^8$, $R^9$, $R^{10}$, $R^{11}$, m, n, p, and q are as defined in formula (I).

Step (1)

A compound represented by formula (II) can be produced by reacting optionally substituted ethyl 4-fluorobenzoate with 3-hydroxypiperidine optionally having a substituent on its carbon atom in the presence or absence of a reaction solvent such as dimethyl sulfoxide, sulfolane, butanol, or N-methylpyrrolidone, preferably dimethyl sulfoxide, at 50 to 180°C, preferably 80 to 140°C. In this reaction, an organic base, such as diisopropylethylamine, or an inorganic salt, such as ammonium chloride, may be added.

Ester compounds other than ethyl 4-fluorobenzoate, for example, methyl esters, propyl esters, butyl esters, or benzyl esters, may be used as the starting compound.

Further, instead of ethyl 4-fluorobenzoate, other 4-halogen-subsituted compounds, for example, ethyl 4-iodobenzoate and ethyl 4-bromobenzoate, may be used. The use of ethyl 4-fluorobenzoate is preferred from the viewpoint of yield.

Furthermore, instead of ethyl 4-fluorobenzoate, optionally substituted 4-fluorobenzonitrile may be used as the starting compound. In this case, a compound represented by formula (VI) can be produced by converting, in a proper later step, the nitrile group to a free carboxyl group, for example, by acid hydrolysis.

The compound represented by formula (II) may also be produced by reacting ethyl 4-bromobenzoate with a piperidine derivative in the presence of palladium, a phosphine ligand, and a base. Phosphine ligands usable herein include 2,2'-bis(diphenylphosphino)-1,1'-binaphthyl, bis(diphenylphosphino)propane, bis(diphenylphosphino)ferrocene, tri-t-butylphosphine, tri-o-tolylphosphine, and triphenylphosphine. Among them, 2,2'-bis(diphenylphosphino)-1,1'-binaphthyl is preferred. Bases include sodium t-butoxide, cesium carbonate, potassium carbonate, and triethylamine, and sodium t-butoxide is preferred. Piperidine compounds include 3-hydroxypiperidine, azetidinol, azepanol, and azocanol.

Step (2)

A compound represented by formula (III) can be produced by reacting the compound represented by formula (II) with phthalimide and an azo compound in a reaction solvent such as tetrahydrofuran, benzene, toluene, dioxane, or dimethylformamide, preferably tetrahydrofuran, in the presence of triphenylphosphine or a trialkylphosphine, preferably tributylphosphine, at -40 to 100°C, preferably -10 to 40°C and then removing the phthaloyl group. Azo compounds include 1,1'-(azodicarbonyl)dipiperidine, diethyl azodicarboxylate, diisopropyl azodicarboxylate, and 1,1'-azobis(N,N-dimethylformamide). Among them, 1,1'-(azodicarbonyl)dipiperidine is preferred.

Alternatively, the compound represented by formula (III) may be produced by converting the hydroxyl group in the compound represented by formula (II) to a leaving group, for example, a sulfonyloxy group such as a methanesulfonyloxy group, or a halogen atom such as a bromine atom, allowing sodium azide or a combination of hydrazoic acid with an azo compound to act on the leaving group to convert the leaving group to an azide group, and then reducing the azide group.

The intermediate represented by formula (III) may also be produced directly from 3-aminopiperidine without use of 3-hydroxypiperidine as the starting compound. The use of the aminopiperidine derivative is advantageous in that the step of introducing amino (step 2) can be omitted.

As described above, the introduction of 3-aminopiperidine into the 4-position of benzoic acid can be carried out in the same manner as used in the case where 3-hydroxybenzoic acid is used as the starting compound, that is, by applying a method wherein coupling is carried out using a 4-fluorobenzoic ester with heating, or a method wherein coupling is carried out using a 3-bromo- or 3-chlorobenzoic ester with heating in the presence of palladium, a phosphine ligand, and a base. In this case, amino at the 3-position of 3-aminopiperidine may be in a free form, or alternatively may be protected, for example, by triphenylmethyl. When coupling has been carried out using 3-aminopiperidine in which amino at the 3-position is protected, the protective group is removed immediately before the step (3).

When the method wherein coupling is carried out using a 4-fluorobenzoic ester and 3-aminopiperidine with heating, the contents of the step (1) described above can be applied to this coupling reaction. Also in the case of the method using palladium and the like, the contents of the step (1) described above can be applied. In this case, palladium is preferably palladium acetate, dibenzylidene acetone palladium ($Pd_2(dba)_3$) or the like, the phosphine ligand is preferably 2,2'-bis(diphenylphosphino)-1,1'-binaphthyl, tri-t-butylphosphine or the like, the base is preferably cesium carbonate, tripotassium phosphate or the like, the reaction solvent is preferably toluene or the like, and the reaction temperature is preferably 50°C to 120°C, preferably 90°C to 110°C. In the reaction using palladium, amino at the 3-position is preferably in a protected state.

Optically active 3-aminopiperidine or racemic modification thereof can be produced by applying a conventional method described in a literature using ornithine or its ester as a starting compound (Hiroyuki Nagano et al., Japanese Patent Laid-Open No. 112554/1993, R. Pellegata et al., Synthesis, 614, 1978, C. E. Masse et al., Org. Lett., 2, 2571, 2000, Peter A. Stark et al., J. Med. Chem., 35, 4264, 1992), either as such or after modification. For example, in the production of (3S)-aminopiperidine described in Reference Example 8, L-ornithine, which is a naturally occurring material, can be used as the starting compound. In fact, for example, in the production of the compound of Example 25 and the compound of Example 30, a production route was used wherein L-ornithine is used as the starting compound and the contemplated compound is produced through (3S)-aminopiperidine.

In producing the compound represented by formula (III), the use of 3-aminopiperidine as the starting compound is advantageous over the use of 3-hydroxypiperidine as the starting compound, for example, in that a side reaction is less likely to occur in the construction of the piperidine ring portion.

The use of a corresponding benzonitrile compound instead of the benzoic ester as the starting compound, for example, the use of 4-fluorobenzonitrile instead of ethyl 4-fluorobenzoate, can also produce a bicyclic key intermediate, that is, 4-(3-aminopiperidin-1-yl)-benzonitrile (see Reference Example 20). When the benzonitrile derivative has been used instead of the benzoic ester derivative, the nitrile group is converted to a corresponding free carboxyl group after coupling of 3-aminopiperidine or after the introduction of an atomic group represented by formula A into amino at the 3-position, preferably after the introduction of an atomic group represented by formula A into amino at the 3-position, by a conventional method or a newly developed method in one step or two or three steps.

Step (3)

A compound represented by formula (IV) can be produced by introducing group A into the free primary amine

in the compound represented by formula (III). The N-C bond between the compound represented by formula (III) and group A can be formed by reacting the compound represented by formula (III) with a reagent, such as optionally modified or substituted 2-bromopyrimidine, modified or substituted 2-chlorobenzimidazole, or 2-methylthio-2-imidazoline, in the presence of a reaction solvent, such as dimethylformamide, dimethyl sulfoxide, sulfolane, pyridine, N-methylpyrrolidone, or methanol, preferably dimethylformamide, in the temperature range of 50 to 170°C, preferably in the temperature range of 60 to 140°C.

Reagents usable in this step are not limited to those recited herein, and any reagent may be used so far as a carbon atom attached to two nitrogen atoms finally combines with the nitrogen atom in the primary amine attached to a carbon atom in the piperidine derivative to form a single bond. Further, optimization of the kind of substrates used and reaction conditions permits the N-C bond to be formed by reacting palladium having a valency of 0 (zero), a phosphine ligand, and a base. See Reference Examples 24 and 54.

Furthermore, the N-C bond may be formed in accordance with the method of Tetrahedron, 51 (2), 353, 1995.

An organic base, such as diisopropylethylamine, N-methylmorpholine, dimethylaminopyridine, or triethylamine, is preferably added as an acid scavenger from the viewpoint of improving the yield. The addition of 2 to 10 equivalents of diisopropylethylamine is preferred.

The compound represented by formula (IV) wherein $R^4$ has been substituted may be prepared by conventional or reductive N-alkylation followed by the introduction of group A into the primary amino group in the compound represented by formula (III) or by the introduction of group A into the primary amino group in the compound represented by formula (IIa) followed by N-alkylation of the secondary amino group in the compound represented by formula (III), if necessary.

The compound represented by formula (IV) can also be produced by converting a hydroxyl group in the piperidine derivative portion of the compound represented by formula (II) to a ketone by a suitable oxidation reaction and then conducting a reductive amination reaction with an amino-containing compound (a compound corresponding to group A), for example, 2-aminopyrimidine.

Alternatively, the compound represented by formula (IV) may be produced by reacting a compound, wherein a basic atomic group corresponding to group A, for example, pyrimidine or benzimidazole, has been previously attached to the primary amino group in the aminopiperidine compound, with ethyl 4-fluorobenzoate.

Step (4)

A compound represented by formula (V) can be produced by hydrolyzing the carboxylic ester represented by formula (IV) and then forming an amide bond. Specifically, the amide compound represented by formula (V) can be produced by reacting an amine represented by formula

$$R^9HNJCHR^{10}COOR^{11}$$

wherein $R^9$, $R^{10}$, $R^{11}$, and J are as defined in formula (I), provided that $R^{11}$ does not represent a hydrogen atom, with a free carboxyl group, produced by hydrolysis with an alkali according to a conventional method, to cause condensation.

The compounds represented by formula (V) having an optionally substituted pyrimidine ring can, if necessary, be reduced to corresponding tetrahydropyrimidine.

In the condensation reaction, a condensing agent, such as dicyclohexylcarbodiimide, diisopropylcarbodiimide, 1-ethyl-3-(3-dimethylaminopropyl)carbodiimide hydrochloride, or benzotriazol-1-yloxytri(dimethylamino)phosphonium hexafluorophosphate, may be used either solely or in combination with a peptide synthesizing reagent, such as N-hydroxysuccinimide or 1-hydroxybenzotriazole. The combination of these reagents permits the desired condensation reaction to proceed with high efficiency. Preferably, from the viewpoint of optimizing the yield, 1 to 3 equivalents of 1-ethyl-3-(3-dimethylaminopropyl)carbodiimide hydrochloride is used in combination with 1 to 2 equivalents of 1-hydroxybenzotriazole.

Reaction solvents usable in the condensation reaction include dimethylformamide, dioxane, and tetrahydrofuran. Among them, dimethylformamide is preferred. The reaction may be carried out in a range of 0 to 80°C, preferably in a range of 20 to 60°C.

In the condensation reaction, a tertiary amine, such as diisopropylethylamine, N-methylmorpholine, dimethylaminopyridine, or triethylamine, may be added as an organic base from the viewpoint of improving the yield. Preferably, 2 to 10 equivalents of N-methylmorpholine is added.

A compound represented by formula (V), wherein >C=O bonded to the phenylene portion is $>CH_2$, that is, a compound analogous to the compound represented by formula (V), may be produced by reductively converting the carboxylic ester of the compound represented by formula (IV) to an aldehyde group and then reductively reacting the aldehyde compound with an amine represented by formula

$$R^9HNJCHR^{10}COOR^{11}$$

wherein $R^9$, $R^{10}$, $R^{11}$, and J are as defined in formula (I).

Analogous compounds represented by formula (V), wherein >C=O is $CH_2$ and $R^9$ represents a group other than a hydrogen atom, which is produced by the reductive amination can be produced by a reaction process other than the method described herein. Specifically, analogous compounds represented by formula (V), wherein >C=O is $CH_2$ and $R^9$ represents a hydrogen atom, can be produced by reductively reacting the free aldehyde group with an amine of formula

$$H_2NJCHR^{10}COOR^{11}$$

wherein $R^{10}$, $R^{11}$, and J are as defined in formula (I). The compounds thus obtained can be further subjected to reductive amination to introduce alkyl, alkenyl, or aralkyl into $R^9$.

The introduction of alkyl, alkenyl, or aralkyl into $R^9$ is not always carried out only for the compound represented by formula (V) in the scheme. That is, the introduction of alkyl, alkenyl, or aralkyl into $R^9$ may be carried out for the compound represented by formula (VI) in the scheme.

Step (5)

The compound represented by formula (VI) may be prepared by converting the carboxylic ester portion in the compound represented by formula (V) to a free carboxyl group, if necessary.

The carboxylic ester portion in the compound represented by formula (V) may be converted to the contemplated free carboxyl group by a conventional method, for example, by hydrolysis with an alkali, hydrolysis with an acid, or reaction with an acid. The deesterification reaction may be achieved by a novel method without any restriction or limitation.

The compound represented by formula (V) is orally administrable integrin $\alpha_v\beta_3$ antagonist and/or GP IIb/IIIa antagonist. Therefore, the step of converting the carboxylic ester to the free carboxyl group is not always necessary.

Compounds represented by formula (VI) containing an optionally substituted pyrimidine ring may be, if necessary, reduced to the corresponding tetrahydropyrimidine. The reduction may be carried out by a conventional method. Examples of reduction methods usable herein include catalytic reduction in the presence of a catalyst, such as palladium-carbon, ruthenium-carbon, rhodium-carbon, palladium oxide, platinum oxide, ruthenium oxide, rhodium platinum oxide complex, rhodium aluminum oxide complex, Raney nickel, or palladium black, and a reaction, for example, with metallic sodium or metallic lithium in liquid ammonia. Preferably, the reduction is carried out in an acidic solvent, for example, in acetic acid acidified with hydrochloric acid, in the presence of palladium-carbon with hydrogen under normal or applied pressure.

Steps (6), (7), and (8)

A compound represented by formula (VII) can be produced by previously protecting the primary amine of the piperidine derivative portion in the compound represented by formula (III), converting the benzoic ester to a free carboxyl group, and then reacting the carboxyl group with an amine of the following formula to form an amide bond:

$$R^9HNJCHR^{10}COOR^{11}$$

wherein $R^9$, $R^{10}$, $R^{11}$, and J are as defined in formula (I), provided that $R^{11}$ does not represent a hydrogen atom. In the compound represented by formula (VII), $R^{21}$ represents a protective group for amino. Protective groups for amino include t-butyloxycarbonyl, benzyloxycarbonyl, and p-methoxybenzyloxycarbonyl. Preferred is t-butyloxycarbonyl.

Next, a compound represented by formula (VIII) can be produced by removing the protective group in the piperidine derivative portion and then optionally converting the carboxylic ester portion to a free carboxyl group.

A compound represented by formula (VI) can be produced by introducing a basic atomic group corresponding to group A, for example, pyrimidine, benzimidazole, or amidino, into the deprotected primary amine according to the step (3) and then, if necessary, converting the carboxylic ester portion to a free carboxyl group. For more details, see Reference Examples 46 and 47 and Examples 75 and 76.

In the above scheme, for example, the formation of an amide bond to convert the compound represented by formula (IV) to the compound represented by formula (V) is first carried out, and, if necessary, in the compounds

represented by formula (VI), an optionally substituted pyrimidine ring is then reduced. However, for example, in the compounds represented by formula (IV), a basic atomic group attached to the primary amino group of the piperidine derivative, for example, an optionally substituted pyrimidine ring may be first reduced followed by amide bond formation.

In the compounds represented by formula (V) and the compounds represented by formula (VI) in the scheme, atomic groups, which have already been constructed in the molecule, for example, $R^7$, $R^8$, $R^9$, and $R^{10}$, may be, if necessary, further converted. For more details, see, for example, Examples 11, 12, 13, 14, 15, and 16.

Alternative production process of compound (III)

The production of low-molecular organic compounds having a substituted piperidine skeleton in a wide range of structures is important for the search and development of drugs useful for humans in an early stage in an efficient manner. For example, the production of a 4-aminopiperidine ring substituted at its 3-position by a fluorine atom has already been reported (M. B. Niel et al., J. Med. Chem., 42, 2087, 1999). However, the construction of a 3-aminopiperidine skeleton, in which the 6-position is not substituted and the 2-position is substituted by methyl, has hardly been reported. On the other hand, there is a report on the synthesis of a 3-aminopiperidine skeleton substituted at its 4-position by methyl (for example, Z. Ma et al., J. Med. Chem., 42, 4202, 1999).

In the above scheme, a bicyclic key intermediate, that is, a 4-(3-aminopiperidin-1-yl)benzoic ester or a corresponding benzonitrile equivalent, is produced by using mainly a hetero ring, in which a cyclic structure has already been constructed, specifically a piperidine ring. The present inventors have succeeded in the production of the bicyclic key intermediate by previously attaching an element for constituting the hetero ring to the 4-position of the benzoic ester and then cyclizing the compound by an intramolecular ring-closing reaction to construct a hetero ring.

Thus, according to the present invention, there is provided a process for producing an intermediate (III') represented by the following scheme.

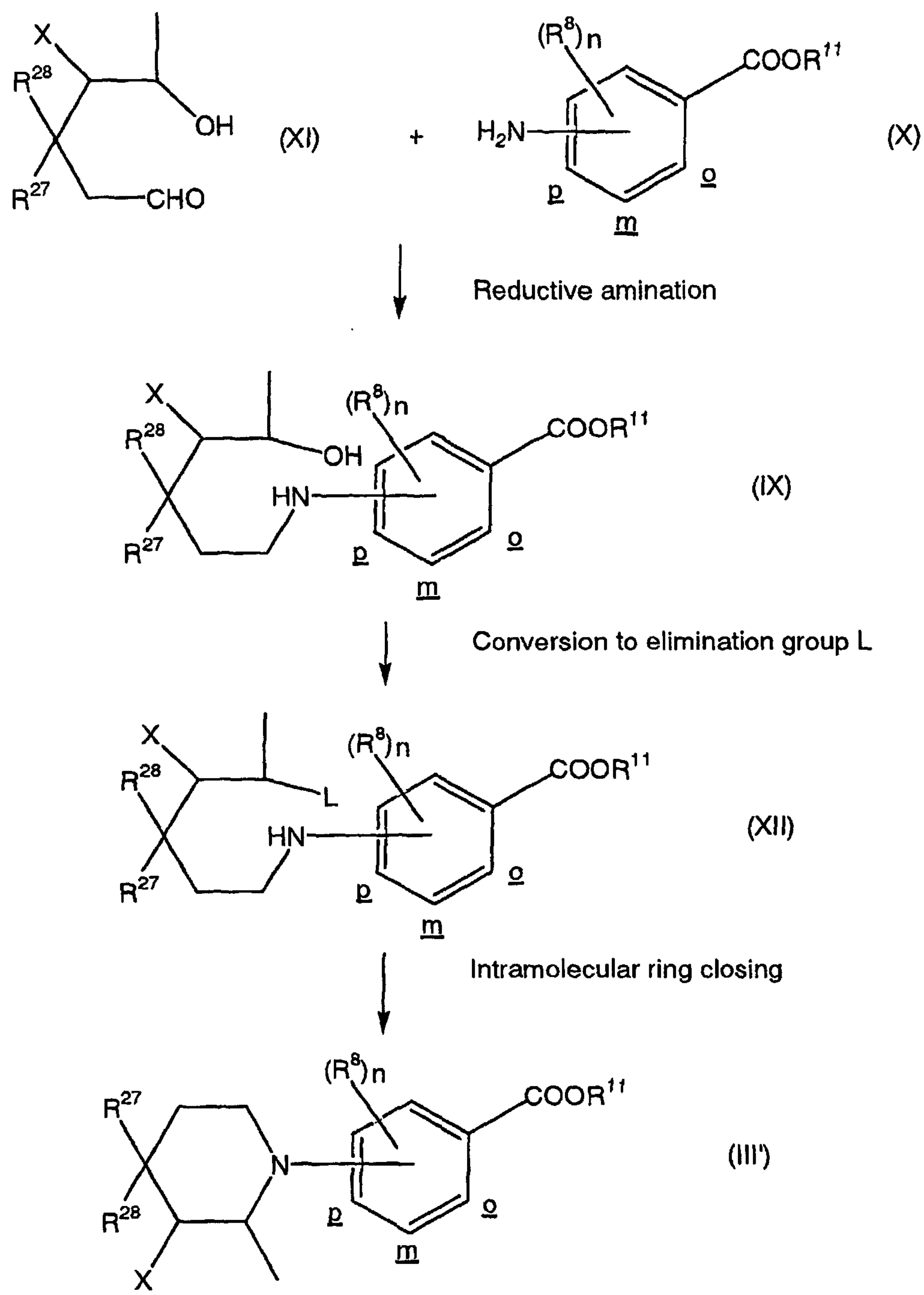


(XI) + (X)

(IX)

(XII)

(III')

wherein $R^{27}$ and $R^{28}$, which may be the same or different, represent a hydrogen atom, hydroxyl, $C_{1-4}$ alkoxy, or $C_{1-4}$ alkyl; X represents hydroxyl, azide, or optionally protected amino; L represents a leaving group; $R^8$, $R^{11}$, and n are as defined in formula (I); and the nitrogen atom is attached to the ortho-position (o-position), meta-position (m-position), or para-position (p-position), preferably para-position, of the phenyl group.

A compound represented by formula (X) is first reacted with a compound represented by formula (XI) to give a compound represented by formula (IX).

The compound represented by formula (XI) (reducing sugar) can be obtained by treating a naturally occurring organic compound, for example, midecamycin, erythromycin, or oleandomycin, with an acid in the presence of a lower alcohol to isolate the compound as a neutral sugar present in the structure, specifically a lower alkylglycoside of mycarose, cladinose, or oleandorose, and then, if necessary, converting a free hydroxyl group present at the 4-position to an azide group while performing steric inversion and, further, converting the compound to a reducing sugar by acid

hydrolysis. The conversion to the azide group can be carried out according to a conventional method.

The compound represented by formula (X) may be reacted with the compound represented by formula (XI) by reductive amination. The reductive amination can be carried out in the presence of a hydride reagent, for example, a reagent such as sodium boron cyanohydride, sodium borohydride, sodium triacetoxy borohydride, or lithium borohydride, in a reaction solvent such as methanol, methylene chloride, dimethylformamide, or dichloroethane, or a mixed solvent thereof, at 0°C to 50°C (provided that the temperature should not exceed the boiling point of the reaction solvent), preferably at room temperature.

For some structures of naturally occurring saccharides used in the reaction, a certain reaction time is required for the formation of an imine which is one of intermediates. The timing of addition of the hydride reagent can be determined according to the substrate used. More specifically, the hydride reagent may be added immediately after the initiation of the reaction, or alternatively a method may be adopted wherein two substrates are dissolved in the reaction solvent and, after the elapse of a certain period of time after the dissolution of the substrates in the reaction solvent, for example, 24 hr after the dissolution of the substrates in the reaction solvent, the hydride reagent is added. Further, in this case, certain organic acids, such as, acetic acid, citric acid, formic acid, methanesulfonic acid, monochloroacetic acid, and monofluoroacetic acid, or inorganic acids, such as, hydrochloric acid or sulfuric acid, may be added, for example, from the viewpoint of improving the selectivity of the hydride reagent in the reaction.

Next, the free hydroxyl group (secondary hydroxyl group) in the compound represented by formula (IX) is converted to a leaving group L to give the compound represented by formula (XII).

The conversion to the leaving group L can be carried out by a conventional method, for example, by reaction with methanesulfonyl chloride in methylene chloride in a range of 0°C to room temperature.

Leaving groups L include, for example, methanesulfonyloxy, p-toluenesulfonyloxy, benzenesulfonyloxy, trifluoromethanesulfonyloxy, and halogen atoms, for example, chlorine, bromine, and iodine atoms.

A compound represented by formula (III') can be produced by cyclizing the compound represented by formula (XII), in which leaving group L has been constructed, by an intramolecular ring-closing reaction.

Bases usable in the intramolecular ring-closing reaction include tertiary organic bases, such as diisopropylethylamine, triethylamine, tribenzylamine, and inorganic bases, such as sodium carbonate and potassium carbonate. The use of organic bases is preferred from the viewpoint of the selectivity in reaction.

After the intramolecular ring-closing reaction, if necessary, the azide group may be reduced to an amino group. The reduction reaction of the azide group may be carried out according to a conventional method.

The reaction solvent used in the step of conversion to a leaving group and the step of an intramolecular ring-closing reaction is not particularly limited.

The compound represented by formula (I) can be produced by subjecting the compound represented by formula (III') thus obtained to steps (3) to (8).

In the formulae (IX), (XI), (XII), and (XIII), $R^{27}$ and $R^{28}$ may represent respective groups selected from the group consisting of the following:

- $R^{27}$ represents a hydrogen atom and $R^{28}$ represents methoxy;
- $R^{27}$ represents methyl and $R^{28}$ represents hydroxyl; and
- $R^{27}$ represents methyl and $R^{28}$ represents methoxy.

In the formulae (IX), (X), (XII), (XIII), and (III'), the nitrogen atom is preferably attached to phenyl at its p-position.

The compound represented by formula (I), wherein Z represents CH, can be produced from 4-bromobenzyl alcohol of which hydroxyl is protected according to the method described in WO 94/12181. Specifically, a 3-(hydroxycyclohexyl)benzoic ester corresponding to formula (II) can be produced by reacting lithiumized 4-bromobenzyl alcohol of which hydroxyl is protected with 3-hydroxycyclohexanone of which hydroxyl is protected to prepare a benzylcyclohexane derivative, reductively removing the resultant hydroxyl group, and conducting proper chemical conversion. Thus, the compound represented by formula (I), wherein Z represents CH, can be produced according to steps (2) to (5).

The compound represented by formula (I), wherein D represents $>CR^5R^6$, can be produced from a compound corresponding to formula (IV), wherein D represents $>CR^5R^6$, according to steps (4) and (5). The compound corresponding to formula (IV), wherein D represents $>CR^5R^6$, can be produced, for example, by reducing 3-pyridyl acetate with platinum oxide in the presence of hydrogen to prepare 3-piperidyl acetate, coupling 3-piperidyl acetate to ethyl 4-fluorobenzoate, and allowing phenylenediamine to act on the carboxyl group in the resultant compound under acidic conditions with heating to cause cyclization.

The compound represented by formula (I), wherein D represents -O-, can be produced from a compound corresponding to formula (IV), wherein D represents -O-, according to steps (4) and (5). The compound corresponding to formula (IV), wherein D represents -O-, can be produced by reacting the hydroxyl group in the compound represented by formula (II) with an alkylsulfonyl-containing basic atomic group, that is, a compound corresponding to group A. This

reaction can be carried out under reaction conditions described, for example, in Japanese Patent Laid-Open No. 97818/1993 or EP 468766 A1.

The compund represented by formula (I), wherein D represents -S-, can be produced from a compound corresponding to formula (IV), wherein D represents -S-, according to steps (4) and (5). The compound corresponding to formula (IV), wherein D represents -S-, can be produced by halogenating the hydroxyl group in the compound represented by formula (II) and reacting this halogen atom with a basic atomic group containing a group -SH, that is, a compound corresponding to group A. The reaction of the halogen atom with the group -SH can be carried out under conditions described, for example, in Res. Lab., Kohjin Co., Ltd., Japan Chem. Pharm. Bull. (1977), 25 (10), 2624-37.

The compound represented by formula (I), wherein D represents -$NHCH_2$-, p is 3, and q is 0 (zero), can be synthesized by reducing ethyl {2-(azidomethyl) pyrrolidin-1-yl}benzoate (Reference Example 3) to give ethyl {2-(aminomethyl)pyrrolidin-1-yl}benzoate and subjecting the resultant compound to steps (3) to (5). This compound may also be produced by a method wherein, in step (1), 2-(hydroxymethyl)pyrrolidine is used instead of 3-hydroxypiperidine, or a method wherein, in step (2), 2-(aminomethyl)pyrrolidine is used instead of 3-aminopiperidine.

Use of compounds/pharmaceutical composition

The compounds according to the present invention have potent integrin $\alpha_v\beta_3$ antagonistic activity, as demonstrated in Pharmacological Test Example 1. Accordingly, the compounds according to the present invention may be used in the treatment of integrin $\alpha_v\beta_3$-mediated diseases. The integrin $\alpha_v\beta_3$ mediates cardiovascular diseases such as acute myocardial infarction, neointima formation hypertrophy, restenosis after PTCA/stent operation, unstable angina, acute coronary syndrome, angina pectoris after PTCA/stent operation, arterial sclerosis, particularly atherosclerosis; angiogenesis-related diseases such as diabetic retinopathy, diabetic vascular complication, or vascular grafting; cerebrovascular diseases such as cerebral infarction; cancers such as solid tumors or metastasis thereof; immunological diseases such as arthritis, particularly rheumatic arthritis; and osteopathy such as osteoporosis, hypercalcemia, periodontitis, hyperparathyroidism, periarticular sore, or Paget's diseases (DN & P, 10 (8), 456 (1997)). The term "therapy" or "treatment" as used herein includes "prevention" or "prophylaxis."

The compounds according to the present invention have cell adhesion inhibitory activity, as demonstrated in Pharmacological Test Example 3. Accordingly, the compounds according to the present invention may be used in the treatment of diseases where the inhibition of cell adhesion is therapeutically effective. More specifically, diseases such as cardiovascular diseases, angiogenesis-related diseases, cerebrovascular diseases, cancers, and immunological diseases can be treated by inhibiting the adhesion between smooth muscle cells and cell adherent proteins, particularly vitronectin (DN & P, 10 (8), 456 (1997)). Further, cancers or metastasis thereof can be treated by inhibiting the adhesion between vascular endothelial cells and cell adherent proteins, particularly vitronectin. Furthermore, osteopathy can be treated by inhibiting the adhesion between osteoclasts and cell adherent proteins, particularly osteopontin.

The term "cell adhesion" as used herein means adhesion between vascular cells, specifically smooth muscle cells and endothelial cells, and cell adherent proteins, specifically vitronectin, osteopontin, and von Willebrand factors; adhesion between vascular cells and hemocyte cells, specifically leukocyte; and adhesion between hemocyte cells themselves, particularly adhesion between human vascular smooth muscle cells and human vitronectin.

As described in Pharmacological Test Example 2, the compounds according to the present invention have GP IIb/IIIa antagonistic activity and human platelet aggregation inhibitory activity. Therefore, the compounds according to the present invention can be used in the treatment of diseases where GP IIb/IIIa antagonism and the inhibition of human platelet aggregation are therapeutically effective. More specifically, the compounds according to the present invention can be used in the treatment of platelet thrombosis and thromboembolism during and after the treatment of thrombolysis and after angioplasty of coronary artery and other arteries and after bypassing of coronary artery, the improvement of peripheral circulating blood stream, and the inhibition of blood clotting during extracorporeal circulation. Furthermore, the compounds according to the present invention can be used in the treatment of thrombotic thrombocytopenic purpura and hemolytic uremic syndrome (Gendai Iryo, 29, (11), 2753 (1997)).

Not only compounds represented by formula (I) wherein $R^{11}$ represents an alkyl group, but also compounds represented by formula (I) wherein $R^{11}$ represents a hydrogen atom (for example, the compound of Example 5), had oral absorption in rats (data not shown). Therefore, any of the compounds, wherein $R^{11}$ represents alkyl or a hydrogen atom, can be used in the treatment of the above diseases.

The compounds according to the present invention and pharmacologically acceptable salts and solvates thereof can be administered orally or parenterally by administration routes, for example, inhalation administration, rhinenchysis, instillation, subcutaneous administration, intravenous injection, intravenous drip infusion, intramuscular injection, rectal administration, or percutaneous administration, and thus may be formed into appropriate various dosage forms depending on oral or parenteral administration routes and administered to human and non-human animals.

The compounds according to the present invention may be formulated into, for example, oral preparation, such as tablets, capsules, granules, powders, pills, particulates, troches, syrups, or emulsions; liquids for external use

such as inhalants, nasal drops, or eye drops; injections such as intravenous injections or intramuscular injections; intravenous drop infusions; preparations for rectal administrations; oleaginous suppositories; water-soluble suppositories; and liniments such as ointments depending upon applications thereof.

These various preparations may be prepared by conventional methods with commonly used components, for example, excipients, extenders, binders, humidifiers, disintegrants, surface active agents, lubricants, dispersants, buffers, preservatives, dissolution aids, antiseptics, flavoring agents, analgesic agents, stabilizers and the like. Non-toxic additives usable herein include, for example, lactose, fructose, glucose, starch, gelatin, magnesium carbonate, synthetic magnesium silicate, talc, magnesium stearate, methylcellulose, carboxymethylcellulose or a salt thereof, gum arabic, olive oil, propylene glycol, polyethylene glycol, syrup, petrolatum, glycerin, ethanol, citric acid, sodium chloride, sodium sulfite, sodium phosphate, ascorbic acid, and cyclodextrins.

The dose of the compound according to the present invention in the medicament may vary depending on the dosage form. The dose is, however, generally 1 to 70% by weight, preferably 5 to 50% by weight, based on the whole composition. The dose for the treatment and prevention of heart diseases and the like may be appropriately determined depending on, for example, the dosage route and the age, sex and severity of condition of patients, and the active ingredient may be administered usually in an amount of about 0.1 to 2,000 mg, preferably about 5 to 400 mg per day per adult. This dose may be administered at a time daily, divided doses of several times daily, or at a time every several days.

## EXAMPLES

The present invention will be described in more detail with reference to the following examples, though it is not limited to these examples only.

### Intermediate 1: Ethyl 4-(3-hydroxypiperidin-1-yl)benzoate

Dimethyl sulfoxide (100 ml) was added to 3-hydroxypiperidine (10.0 g, 99.0 mmol) to prepare a solution. Ethyl 4-fluorobenzoate (14.5 ml, 99.0 mmol) was added to the solution, and the mixture was stirred at 100°C for 16 hr. The temperature of the reaction solution was returned to room temperature, and the reaction solution was then poured into 1000 ml of water with stirring. The mixture was adjusted to pH 11 by the addition of concentrated aqueous ammonia and was then extracted three times with ethyl acetate. The organic layers were combined, and the combined organic layers were washed twice with 500 ml of saturated brine, were then dried over anhydrous sodium sulfate, and were concentrated under the reduced pressure. The resultant solid was broken under cooling to 0°C, and 100 ml of hexane was added thereto. The solid was then collected by filtration through a glass filter and was washed three times with 100 ml of hexane and twice with 100 ml of water. The solid thus obtained was dried to give the title compound (17.2 g, 70%).

Physicochemical properties of intermediate 1

(1) Color and form: Light brown solid
(2) Molecular formula: : $C_{14}H_{19}NO_3$
(3) Mass spectrum (EIMS): m/z 249 $(M)^+$
(4) $^1H$ NMR spectrum (400 MHz, $CDCl_3$) $\delta$ (ppm): 1.37 (3H, t, $CH_2CH_3$), 1.65 (1H, m, piperidine), 1.92 (3H, m, piperidine), 3.15 (2H, m, piperidine), 3.37 (1H, m, piperidine), 3.57 (1H, dd, piperidine), 3.91 (1H, m, piperidine), 4.33 (2H, q, $CH_2CH_3$), 6.89 (2H, d, $C_6H_4$), 7.92 (2H, d, $C_6H_4$)

### Intermediate 2: Ethyl 4-{3-(methanesulfonyloxy)-piperidin-1-yl}benzoate

Methylene chloride (480 ml) was added to intermediate 1 (12.0 g, 48.1 mmol) to prepare a solution, and triethylamine (19 ml, 135 mmol) was added to the solution. Methanesulfonyl chloride (5.2 ml, 67.3 mmol) was gradually added dropwise thereto at room temperature, and the mixture was stirred at that temperature for 30 min. A saturated aqueous sodium hydrogencarbonate solution (1000 ml) was added to stop the reaction, and the mixture was extracted three times with 500 ml of ethyl acetate. The organic layers were combined, and the combined organic layers were dried over anhydrous sodium sulfate and were concentrated under the reduced pressure to give the title compound (15.1 g, 96%).

Physicochemical properties of intermediate 2

(1) Color and form: Brown solid
(2) Molecular formula: $C_{15}H_{21}NO_5S$
(3) $^1$H NMR spectrum (400 MHz, $CDCl_3$) δ (ppm): 1.37 (3H, t, $CH_2CH_3$), 1.69 (1H, m, piperidine), 1.93 (2H, m, piperidine), 2.07 (1H, m, piperidine), 3.03 (3H, s, Ms), 3.21 (1H, ddd, piperidine), 3.40 (1H, dd, piperidine), 3.46 (1H, m, piperidine), 3.77 (1H, dd, piperidine), 4.33 (2H, q, $CH_2CH_3$), 4.85 (1H, dddd, piperidine), 6.89 (2H, d, $C_6H_4$), 7.93 (2H, d, $C_6H_4$)

Intermediate 3: Ethyl 4-(3-azidopiperidin-1-yl)benzoate

Dimethylformamide (96 ml) was added to intermediate 2 (15.1 g, 48.1 mmol) to prepare a solution. Sodium azide (3.75 g, 57.7 mmol) was added to the solution, and the mixture was stirred at 80°C for 5 hr. The temperature of the reaction solution was returned to room temperature, and the reaction solution was then poured into 1500 ml of water. The mixture was extracted three times with 500 ml of ethyl acetate. The organic layers were combined, and the combined organic layers were washed twice with 500 ml of water, were then dried over anhydrous sodium sulfate, and were concentrated under the reduced pressure to give an about 1 : 1 mixture (12.0 g, 91%) of the title compound with ethyl 4-{2-(azidomethyl)pyrrolidin-1-yl)benzoate.

Physicochemical properties of mixture of intermediate 3 with ethyl 4-{2-(azidomethyl)pyrrolidin-1-yl)benzoate

(1) Color and form: Brown oil
(2) Molecular formula: $C_{14}H_{18}N_4O_2$
(3) Mass spectrum (EIMS): m/z 274 $(M)^+$

Intermediate 4: Ethyl 4-(3-aminopiperidin-1-yl)benzoate

Dioxane (220 ml) and 10 ml of acetic acid were added to the about 1 : 1 mixture (6.0 g, 21.9 mmol) of intermediate 3 with ethyl 4-{2-(azidomethyl)pyrrolidin-1-yl)benzoate to prepare a solution. To the solution was added 600 mg of 10% palladium-carbon. The mixture was vigorously stirred under a hydrogen pressure of 1 atm at room temperature for 23 hr. The insolubles were collected by filtration and were then washed twice with dioxane and twice with methanol. The filtrate and the washings were combined, followed by concentration under the reduced pressure. The residue was purified by column chromatography on silica gel (development system: methylene chloride : methanol = 7 : 1) to give the title compound (1.45 g, 27%).

Physicochemical properties of intermediate 4

(1) Color and form: Brown oil
(2) Molecular formula: $C_{14}H_{20}N_2O_2$
(3) Mass spectrum (TSPMS): m/z 249 $(M+H)^+$
(4) $^1$H NMR spectrum (400 MHz, $CDCl_3$) δ (ppm): 1.31 (1H, m, piperidine), 1.36 (3H, t, $CH_2CH_3$), 1.66 (1H, m, piperidine), 1.82 (1H, m, piperidine), 1.98 (1H, dddd, piperidine), 2.70 (1H, dd, piperidine), 2.94 (2H, m, piperidine), 3.63 (1H, ddd, piperidine), 3.74 (1H, dddd, piperidine), 4.33 (2H, q, $CH_2CH_3$), 6.87 (2H, d, $C_6H_4$), 7.91 (2H, d, $C_6H_4$)

Intermediate 5: Ethyl 4-{3-(pyrimidin-2-ylamino)piperidin-1-yl}benzoate

Dimethyl sulfoxide (20 ml) was added to intermediate 4 (500 mg, 2.01 mmol) to prepare a solution. 2-Bromopyrimidine (352 mg, 2.21 mmol) was added to the solution. Further, diisopropylethylamine (2.0 ml, 11.3 mmol) was added thereto, and the mixture was heated to 120°C and was stirred for 9 hr. The temperature of the reaction solution was returned to room temperature, 200 ml of saturated brine was then added thereto, and the mixture was extracted three times with 150 ml of ethyl acetate. The organic layers were combined, and the combined organic layers were washed with saturated brine and were then dried over anhydrous sodium sulfate, followed by concentration under the reduced

pressure. The residue was purified by column chromatography on silica gel (development system: hexane : ethyl acetate = 1 : 1) to give the title compound (554 mg, 84%).

Physicochemical properties of intermediate 5

(1) Color and form: Light yellow solid
(2) Molecular formula: $C_{18}H_{22}N_4O_2$
(3) Mass spectrum (TSPMS): m/z 327 $(M+H)^+$
(4) $^1H$ NMR spectrum (400 MHz, $CDCl_3$) $\delta$ (ppm): 1.36 (3H, t, $CH_2CH_3$), 1.70 (2H, m, piperidine), 1.87 (1H, m, piperidine), 2.01 (1H, m, piperidine), 3.04 (1H, dd, piperidine), 3.17 (1H, ddd, piperidine), 3.52 (1H, ddd, piperidine), 3.87 (1H, dd, piperidine), 4.14 (1H, m, piperidine), 4.33 (2H, q, $CH_2CH_3$), 6.56 (1H, t, pyrimidine), 6.92 (2H, d, $C_6H_4$), 7.91 (2H, d, $C_6H_4$), 8.30 (2H, d, pyrimidine)

Intermediate 6: 4-{3-(Pyrimidin-2-ylamino)piperidin-1-yl}benzoic acid

Tetrahydrofuran (14 ml) and 4.6 ml of methanol were added to intermediate 5 (300 mg, 0.920 mmol) to prepare a solution, and 4.6 ml of a 1.0 mol/liter aqueous sodium hydroxide solution was added to the solution. The mixture was stirred at 40°C for 4 hr and was then concentrated under the reduced pressure. Water (100 ml) was added to the residue, and the mixture was washed with ethyl acetate. The aqueous layer was adjusted to pH 6 by the addition of 5.0 mol/liter hydrochloric acid. The resultant precipitate was collected by filtration through a glass filter, was washed with water, and was then dried to give the title compound (249 mg, 91%).

Physicochemical properties of intermediate 6

(1) Color and form: Colorless solid
(2) Molecular formula: $C_{16}H_{18}N_4O_2$
(3) Mass spectrum (EIMS): m/z 298 $(M)^+$
(4) $^1H$ NMR spectrum (400 MHz, DMSO-$d_6$) $\delta$ (ppm): 1.56 (2H, m, piperidine), 1.77 (1H, m, piperidine), 1.96 (1H, m, piperidine), 2.74 (1H, dd, piperidine), 2.86 (1H, br dd, piperidine), 3.84 (2H, m, piperidine), 3.96 (1H, br d, piperidine), 6.59 (1H, t, pyrimidine), 6.96 (2H, d, $C_6H_4$), 7.74 (2H, d, $C_6H_4$), 8.31 (2H, d, pyrimidine)

Example 1: t-Butyl (2S)-benzenesulfonylamino-3-[4-{3-(pyrimidin-2-ylamino)piperidin-1-yl}benzoylamino]-propionate

Dimethylformamide (3.5 ml) was added to intermediate 6 (100 mg, 0.335 mmol) to prepare a solution, and t-butyl (2S)-N-benzenesulfonyl-2,3-diaminopropionate (111 mg, 0.369 mmol) was added to the solution. Further, 1-hydroxybenzotriazole (59 mg, 0.436 mmol), N-methylmorpholine (0.11 ml, 1.01 mmol), and 1-ethyl-3-(3-dimethylaminopropyl)carbodiimide hydrochloride (84 mg, 0.436 mmol) were added thereto, and the mixture was stirred at room temperature for 17 hr. Saturated brine (100 ml) and 50 ml of a saturated aqueous potassium carbonate solution were added to the reaction solution, and the mixture was extracted three times with 100 ml of ethyl acetate. The organic layers were combined, and the combined organic layers were washed twice with a mixed solution composed of 250 ml of saturated brine and 250 ml of water, were dried over anhydrous sodium sulfate, and were then concentrated under the reduced pressure. The residue was purified by column chromatography on silica gel (development system: methylene chloride : methanol = 30 : 1) to give the title compound (190 mg, 100%).

Physicochemical properties of compound prepared in Example 1

(1) Color and form: Colorless oil
(2) Molecular formula: $C_{29}H_{36}N_6O_5S$
(3) Mass spectrum (FABMS): m/z 581 $(M+H)^+$
(4) Specific rotation: $[\alpha]_D^{25}$ +27° (c 1.0, MeOH)
(5) $^1H$ NMR spectrum (400 MHz, $CD_3OD$) $\delta$ (ppm): 1.23 (9H, s, t-Bu), 1.67 (2H, m, piperidine), 1.82 (1H, m, piperidine), 2.13 (1H, m, piperidine), 2.90 (1H, m, piperidine), 3.02 (1H, m, piperidine), 3.51 (1H, dd, $CONHCH_2CH$),

3.64 (1H, dd, $CONHCH_2CH$), 3.68 (1H, m, piperidine), 3.93 (1H, br d, piperidine), 4.03 (1H, m, piperidine), 4.10 (1H, m, $CONHCH_2CH$), 6.62 (1H, t, pyrimidine), 6.98 (2H, d, $C_6H_4$), 7.46 (2H, t, $C_6H_5$), 7.53 (1H, t, $C_6H_5$), 7.64 (2H, d, $C_6H_4$), 7.82 (2H, d, $C_6H_5$), 8.29 (2H, d, pyrimidine)

Example 2: (2S)-Benzenesulfonylamino-3-[4-{3-(pyrimidin-2-ylamino)piperidin-1-yl}benzoylamino]propionic acid

Methylene chloride (3.0 ml) was added to the compound prepared in Example 1 (175.6 mg, 0.302 mmol) to prepare a solution. Trifluoroacetic acid (3.0 ml) was added to the solution at room temperature, and the mixture was stirred for 4 hr. The reaction solution was concentrated under the reduced pressure, and the residue was subjected to azeotropic distillation twice with toluene to give a trifluoroacetate of the title compound.

Physicochemical properties of compound prepared in Example 2

(1) Color and form: Colorless amorphous form
(2) Molecular formula: $C_{25}H_{28}N_6O_5S$
(3) Mass spectrum (FABMS): m/z 525 $(M+H)^+$
(4) Specific rotation: $[\alpha]_D^{25}$ +24° (c 1.2, MeOH) (as trifluoroacetate)
(5) $^1H$ NMR spectrum (400 MHz, $CD_3OD$) (as trifluoroacetate) δ (ppm): 1.79 (2H, m, piperidine), 1.94 (1H, m, piperidine), 2.10 (1H, m, piperidine), 3.16 (2H, m, piperidine), 3.49 (1H, dd, $CONHCH_2CH$), 3.63 (1H, br d, piperidine), 3.72 (1H, dd, $CONHCH_2CH$), 3.86 (1H, dd, piperidine), 4.18 (1H, dd, $CONHCH_2CH$), 4.27 (1H, dddd, piperidine), 6.95 (1H, t, pyrimidine), 7.05 (2H, d, $C_6H_4$), 7.42 (2H, t, $C_6H_5$), 7.49 (1H, t, $C_6H_5$), 7.68 (2H, d, $C_6H_4$), 7.82 (2H, d, $C_6H_5$), 8.60 (2H, m, pyrimidine)

Example 3: (2S)-Benzenesulfonylamino-3-[4-{3-(1,4,5,6-tetrahydropyrimidin-2-ylamino)piperidin-1-yl}benzoylamino] propionic acid

Dioxane (3.0 ml) and 0.3 ml of water were added to the trifluoroacetate (0.302 mmol) of the crude compound prepared in Example 2 to prepare a solution. To the solution was added 40 mg of 10% palladium-carbon. The mixture was vigorously stirred under a hydrogen pressure of 1 atm at room temperature for 3 hr. The insolubles were collected by filtration and were then washed twice with a mixed solution composed of 10 ml of dioxane and 1 ml of water and twice with a mixed solution composed of 10 ml of methanol and 1 ml of concentrated aqueous ammonia. The filtrate and the washings were combined followed by concentration under the reduced pressure. The residue was purified by column chromatography on silica gel (development system: methylene chloride : ethanol : water : concentrated aqueous ammonia = 15 : 10 : 1 : 1) and was then purified by Sephadex LH-20 (development system: methanol) to give the title compound (93.7 mg, 80%).

Physicochemical properties of compound prepared in Example 3

(1) Color and form: Colorless solid
(2) Molecular formula: $C_{25}H_{32}N_6O_5S$
(3) Mass spectrum (TSPMS): m/z 529 $(M+H)^+$
(4) Specific rotation: $[\alpha]_D^{26}$ +80° (c 0.28, MeOH-conc. $NH_4OH$ (10 : 1))
(5) $^1H$ NMR spectrum (400 MHz, $CD_3OD$) δ (ppm): 1.59 (1H, m, piperidine), 1.71 (1H, m, piperidine), 1.85 (1H, m, piperidine), 1.93 (1H, m, piperidine), 1.95 (2H, quintet, tetrahydropyrimidine), 3.03 (1H, dd, piperidine), 3.10 (1H, br dd, piperidine), 3.34 (1H, m, piperidine), 3.35 (4H, t, tetrahydropyrimidine), 3.58 (4H, m, piperidine and $CONHCH_2CH$), 3.71 (1H, dd, $CONHCH_2CH$), 6.94 (2H, d, $C_6H_4$), 7.48 (2H, t, $C_6H_5$), 7.55 (1H, t, $C_6H_5$), 7.69 (2H, d, $C_6H_4$), 7.85 (2H, d, $C_6H_5$)

Intermediate 7: (3S)-Aminopiperidin-2-one

Methanol (120 ml) was added to L-ornithine hydrochloride (20.0 g, 119 mmol) to prepare a suspension, and the suspension was cooled to -78°C. Thionyl chloride (25.7 ml, 297 mmol) was added dropwise to the cooled suspension in the internal temperature range of -78°C to -45°C over a period of 20 min. Fifteen min after the completion of the dropwise addition, the temperature of the mixture was raised to room temperature, and the mixture was further

vigorously stirred for 13 hr. The reaction solution was concentrated under the reduced pressure, and the residue was further dried by means of a vacuum pump for 3 hr. The amorphous material thus obtained was subjected to column chromatography using a column packed with an Amberlite IRA-400 ($OH^-$) anion exchange resin (130 g) to give the title compound as a crude product. A mixed solution composed of 800 ml of chloroform and 80 ml of methanol was added to the crude compound. The insolubles were collected by filtration through Celite. A mixed solvent composed of 400 ml of chloroform and 40 ml of methanol was added again to 9.6 g of the insolubles, and the insolubles were then removed by filtration. The filtrates were combined followed by concentration to give the title compound (13.7 g, 77%).

Physicochemical properties of intermediate 7

(1) Color and form: Colorless solid
(2) Molecular formula: $C_5H_{10}N_2O$
(3) Mass spectrum (EIMS): m/z 114 $(M)^+$
(4) Specific rotation: $[\alpha]_D^{24}$ -13° (c 1.5, $CHCl_3$)
(5) $^1H$ NMR spectrum (400 MHz, $D_2O$) δ (ppm): 1.48 (1H, m, piperidine), 1.72 (2H, m, piperidine), 1.99 (1H, dddd, piperidine), 3.16 (2H, dd, piperidine), 3.24 (1H, dd, piperidine)

Intermediate 8: (3S)-Aminopiperidine

Tetrahydrofuran (270 ml) was added to aluminum lithium hydride (2.52 g, 66.4 mmol) to prepare a suspension which was then ice cooled. Intermediate 7 (4.0 g, 26.6 mmol) was gradually added to the cooled suspension in the internal temperature range of 5°C to 16°C. Ten min after the completion of the addition, the temperature of the mixture was raised to room temperature, and the mixture was further vigorously stirred for three hr. Aluminum lithium hydride (202 mg, 5.32 mmol) was added thereto, and the mixture was stirred for 50 min. The mixture was ice cooled, and 2.7 ml of water, 2.7 ml of a 5.0 mol/liter aqueous sodium hydroxide solution, and 9.1 ml of water were added in that order to the cooled mixture. The temperature of the mixture was raised to room temperature, and the mixture was vigorously stirred for 1.5 hr. The precipitated inorganic material was collected by filtration through Celite and was then washed with tetrahydrofuran. The filtrate and the washings were combined followed by drying over anhydrous sodium sulfate. Filtration was then carried out, a 4.0 mol/liter solution of hydrochloric acid in ethyl acetate (13.3 ml, 53.2 mmol) was added to the filtrate, and the solvent was removed by distillation under the reduced pressure. The residue was subjected to azeotropic distillation with methanol to give a dihydrochloride of the title compound (3.57 g, 78%).

Physicochemical properties of intermediate 8

(1) Color and form: Brown solid
(2) Molecular formula: $C_5H_{12}N_2$
(3) Mass spectrum (EIMS): m/z 100 $(M)^+$
(4) $^1H$ NMR spectrum (400 MHz, $CD_3OD$) (as dihydrochloride) δ (ppm): 1.75 (1H, dddd, piperidine), 1.90 (1H, m, piperidine), 2.09 (1H, ddddd, piperidine), 2.23 (1H, br d, piperidine), 3.02 (1H, ddd, piperidine), 3.09 (1H, dd, piperidine), 3.41 (1H, br d, piperidine), 3.62 (2H, m, piperidine)

Intermediate 9: Methyl 4-{(3S)-aminopiperidin-1-yl}-3-fluorobenzoate

N-Methylpyrrolidone (40 ml) was added to methyl 3,4-difluorobenzoate (1.29 g, 7.5 mmol), and the mixture was stirred at room temperature. Intermediate 8 (2.6 g, 15.0 mmol) and sodium hydrogencarbonate (4.03 g, 48 mmol) were added thereto, and the mixture was stirred at 110°C overnight. Water (70 ml) was added to the reaction solution, and the mixture was extracted three times with ethyl acetate. The organic layer was dried over anhydrous magnesium sulfate and was then concentrated under the reduced pressure. The residue was purified by column chromatography on silica gel (development system: chloroform : methanol = 8 : 2) to give the title compound (0.55 g, 29%).

Physicochemical properties of intermediate 9

(1) Color and form: Brown liquid
(2) Molecular formula: $C_{13}H_{17}N_2O_2F$
(3) Mass spectrum (FABMS) : m/z 253 $(M+H)^+$
(4) $^1H$ NMR spectrum (400 MHz, $CDCl_3$) δ (ppm): 1.30 (1H, m, piperidine), 1.73 (1H, m, piperidine), 1.86 (1H, m, piperidine), 1.96 (1H, m, piperidine), 2.66 (1H, dd, piperidine), 2.85 (1H, m, piperidine), 3.04 (1H, m, piperidine), 3.35 (1H, m, piperidine), 3.46 (1H, m, piperidine), 3.87 (3H, s, $OCH_3$), 6.90 (1H, dd, $C_6H_3$), 7.64 (1H, dd, $C_6H_3$), 7.72 (1H, dd, $C_6H_3$)

Intermediate 10: Methyl 3-fluoro-4-{(3S)-(pyrimidin-2-ylamino)piperidin-1-yl}benzoate

N-Methylpyrrolidone (20 ml) was added to intermediate 9 (550 mg, 2.1 mmol) to prepare a solution. N,N-Diisopropylethylamine (0.63 ml, 3.6 mmol) and 2-bromopyrimidine (400 mg, 2.5 mmol) were added to the solution, and the mixture was stirred at 100°C for 20 hr. Water (50 ml) was then added to the reaction solution, and the mixture was extracted three times with ethyl acetate. The organic layers were combined, and the combined organic layers were dried over anhydrous magnesium sulfate and were then concentrated under the reduced pressure. The residue was purified by column chromatography on silica gel (development system: hexane : ethyl acetate = 7 : 3) to give the title compound (0.15 g, 21%).

Physicochemical properties of intermediate 10

(1) Color and form: Colorless syrup
(2) Molecular formula: $C_{17}H_{19}N_4O_2F$
(3) Mass spectrum (FABMS): m/z 331 $(M+H)^+$
(4) Specific rotation: $[\alpha]_D^{26}$ +69° (c 0.27, $CHCl_3$)
(5) $^1H$ NMR spectrum (400 MHz, $CDCl_3$) δ (ppm): 1.76 (2H, m, piperidine), 1.92 (2H, m, piperidine), 3.15 (3H, m, piperidine), 3.47 (1H, dd, piperidine), 3.87 (3H, s, $OCH_3$), 4.25 (1H, m, piperidine), 5.52 (1H, d, NH), 6.52 (1H, d, pyrimidine), 6.97 (1H, dd, $C_6H_3$), 7.64 (1H, dd, $C_6H_3$), 7.72 (1H, dd, $C_6H_3$), 8.27 (2H, d, pyrimidine)

Intermediate 11: 3-Fluoro-4-{(3S)-(pyrimidin-2-ylamino)-piperidin-1-yl}benzoic acid

Tetrahydrofuran (3.6 ml) and 1.2 ml of methanol were added to intermediate 10 (81.1 mg, 0.245 mmol) to prepare a solution, and 1.2 ml of a 1.0 mol/liter aqueous sodium hydroxide solution was added to the solution. The mixture was stirred at 40°C for 8 hr and was then concentrated under the reduced pressure. Water (5 ml) was added to the residue to prepare a solution which was then washed twice with 5 ml of ethyl acetate. The aqueous layer was adjusted to pH 4 by the addition of 1.0 mol/liter hydrochloric acid. The precipitated solid was collected by filtration through a glass filter, was washed twice with water, and was then dried to give the title compound (54.6 mg, 70%).

Physicochemical properties of intermediate 11

(1) Color and form: Colorless solid
(2) Molecular formula: $C_{16}H_{17}N_4O_2F$
(3) Mass spectrum (TSPMS): m/z 317 $(M+H)^+$
(4) $^1H$ NMR spectrum (400 MHz, DMSO-$d_6$) δ (ppm): 1.50 (1H, m, piperidine), 1.66 (1H, m, piperidine), 1.82 (1H, m, piperidine), 1.94 (1H, m, piperidine), 2.63 (1H, m, piperidine), 2.73 (1H, br t, piperidine), 3.30 (1H, m, piperidine), 3.56 (1H, br d, piperidine), 3.99 (1H, m, piperidine), 6.57 (1H, t, pyrimidine), 7.05 (1H, t, $C_6H_3$), 7.49 (1H, dd, $C_6H_3$), 7.61 (1H, dd, $C_6H_3$), 8.28 (2H, d, pyrimidine)

Example 4: t-Butyl (2S)-benzenesulfonylamino-3-[3-fluoro-4-{(3S)-(pyrimidin-2-ylamino)piperidin-1 yl}benzoylamino] propionate

Dimethylformamide (2.0 ml) was added to intermediate 11 (54.6 mg, 0.173 mmol) to prepare a solution, and t-butyl (2S)-N-benzenesulfonyl-2,3-diaminopropionate (57 mg, 0.190 mmol) was added to the solution. Further, 1-hydroxybenzotriazole (25.7 mg, 0.190 mmol), N-methylmorpholine (29 µl, 0.260 mmol), and 1-ethyl-3-(3-dimethylaminopropyl)carbodiimide hydrochloride (43.1 mg, 0.225 mmol) were added thereto, and the mixture was stirred at room temperature for 20 hr. Water (30 ml) and 20 ml of a saturated aqueous potassium carbonate solution were added to the reaction solution, and the mixture was extracted three times with ethyl acetate. The organic layers were combined, and the combined organic layers were washed with a mixed solution composed of 50 ml of saturated brine and 50 ml of water, were dried over anhydrous sodium sulfate, and were then concentrated under the reduced pressure. The residue was purified by preparative thin-layer chromatography on silica gel (development system: hexane : ethyl acetate = 1 : 2) to give the title compound (90.0 mg, 87%).

Physicochemical properties of compound prepared in Example 4

(1) Color and form: Colorless solid
(2) Molecular formula: $C_{29}H_{35}N_6O_5FS$
(3) Mass spectrum (TSPMS): m/z 599 $(M+H)^+$
(4) Specific rotation: $[\alpha]_D^{24}$ +28° (c 1.4, MeOH)
(5) $^1$H NMR spectrum (400 MHz, $CD_3OD$) δ (ppm): 1.24 (9H, s, t-Bu), 1.66 (1H, ddt, piperidine), 1.80 (1H, m, piperidine), 1.96 (2H, m, piperidine), 2.92 (1H, dd, piperidine), 2.99 (1H, ddd, piperidine), 3.36 (1H, m, piperidine), 3.48 (1H, dd, $CONHC\underline{H}_2CH$), 3.56 (1H, dd, piperidine), 3.65 (1H, dd, $CONHC\underline{H}_2CH$), 4.11 (1H, dd, $CONHCH_2C\underline{H}$), 4.15 (1H, m, piperidine), 6.61 (1H, t, pyrimidine), 7.11 (1H, t, $C_6H_3$), 7.49 (5H, m, $C_6H_5$ and $C_6H_3$), 7.83 (2H, m, $C_6H_5$), 8.28 (2H, d, pyrimidine)

Example 5: (2S)-Benzenesulfonylamino-3-[3-fluoro-4-{(3S)-(pyrimidin-2-ylamino)piperidin-1-yl}benzoylamino]-propionic acid

Methylene chloride (1.5 ml) was added to the compound prepared in Example 4 (90.0 mg, 0.150 mmol) to prepare a solution. Trifluoroacetic acid (1.5 ml) was added to the solution, and the mixture was stirred at room temperature for 20 hr. The reaction solution was concentrated under the reduced pressure to give a trifluoroacetate of the title compound.

Physicochemical properties of compound prepared in Example 5

(1) Color and form: Colorless solid
(2) Molecular formula: $C_{25}H_{27}N_6O_5FS$
(3) Mass spectrum (TSPMS): m/z 543 $(M+H)^+$
(4) Specific rotation: $[\alpha]_D^{24}$ +42° (c 0.5, MeOH) (as trifluoroacetate)
(5) $^1$H NMR spectrum (400 MHz, $CD_3OD$) (as trifluoroacetate) δ (ppm): 1.82 (2H, m, piperidine), 2.01 (2H, m, piperidine), 3.11 (2H, m, piperidine), 3.26 (1H, m, piperidine), 3.46 (1H, dd, $CONHC\underline{H}_2CH$), 3.50 (1H, dd, piperidine), 3.72 (1H, dd, $CONHC\underline{H}_2CH$), 4.19 (1H, dd, $CONHCH_2C\underline{H}$), 4.34 (1H, ddt, piperidine), 6.96 (1H, t, pyrimidine), 7.11 (1H, m, $C_6H_3$), 7.48 (5H, m, $C_6H_5$ and $C_6H_3$), 7.82 (2H, m, $C_6H_5$), 8.60 (2H, m, pyrimidine)

Example 6: (2S)-Benzenesulfonylamino-3-[3-fluoro-4-{(3S)-(1,4,5,6-tetrahydropyrimidin-2-ylamino)piperidin-1-yl} benzoylamino]propionic acid

Dioxane (1.5 ml) and 0.15 ml of water were added to the trifluoroacetate of the crude compound (0.150 mmol) prepared in Example 5 to prepare a solution. To the solution was added 9.0 mg of 10% palladium-carbon. The mixture was vigorously stirred under a hydrogen pressure of 1 atm at room temperature for 4 hr. The insolubles were collected by filtration and were then washed with a mixed solvent composed of 10 ml of dioxane and 1 ml of water. The filtrate and the washings were combined followed by concentration under the reduced pressure. The residue was purified by preparative thin-layer chromatography on silica gel (development system: methylene chloride : ethanol : water : concentrated aqueous ammonia = 8 : 8 : 1 : 1) and was then purified by Sephadex LH-20 (development system: methanol) to give the title compound (80.0 mg, 98%).

Physicochemical properties of compound prepared in Example 6

(1) Color and form: Colorless solid
(2) Molecular formula: $C_{25}H_{31}N_6O_5FS$
(3) Mass spectrum (TSPMS): m/z 547 $(M+H)^+$
(4) Specific rotation: $[\alpha]_D^{26}$ +87° (c 1.0, MeOH)
(5) $^1H$ NMR spectrum (400 MHz, $CD_3OD$) δ (ppm): 1.66 (1H, m, piperidine), 1.78 (1H, m, piperidine), 1.90 (2H, m, piperidine), 1.96 (2H, quintet, tetrahydropyrimidine), 3.12 (3H, m, piperidine), 3.25 (1H, dd, piperidine), 3.37 (4H, t, tetrahydropyrimidine), 3.53 (1H, dd, CONHC$\underline{H_2}$CH), 3.66 (1H, dd, CONHC$\underline{H_2}$CH), 3.72 (1H, m, piperidine), 3.75 (1H, dd, $CONHCH_2C\underline{H}$), 7.06 (1H, t, $C_6H_3$), 7.51 (4H, m, $C_6H_5$ and $C_6H_3$), 7.58 (1H, dd, $C_6H_3$), 7.86 (2H, m, $C_6H_5$)

<u>Intermediate 12: (3R)-Aminopiperidin-2-one</u>

Methanol (25 ml) was added to D-ornithinehydrochloride (4.0 g, 24 mmol) to prepare a suspension, and the suspension was cooled to -78°C. Thionyl chloride (5.1 ml, 59 mmol) was added dropwise thereto in the internal temperature range of -78°C to -45°C over a period of 20 min. Fifteen min after the completion of the dropwise addition, the temperature of the mixture was raised to room temperature, and the mixture was further vigorously stirred for 13 hr. The reaction solution was concentrated under the reduced pressure, and the residue was then dried by means of a vacuum pump for 3 hr. The amorphous material was subjected to column chromatography using a column packed with an Amberlite IRA-400 ($OH^-$) anion exchange resin (25 g) to give the title compound as a crude product. The crude product was purified by column chromatography on silica gel (development system: dichloromethane : ethanol : water : aqueous ammonia = 8 : 8 : 1 : 1) to give the title compound (1.7 g, 63%).

Physicochemical properties of intermediate 12

(1) Color and form: Colorless solid
(2) Molecular formula: $C_5H_{10}N_2O$
(3) Mass spectrum (EIMS): m/z 114 $(M^+)$
(4) $^1H$ NMR spectrum (400 MHz, $CD_3OD$) δ (ppm): 1.48 (1H, m, piperidine), 1.72 (2H, m, piperidine), 1.99 (1H, dddd, piperidine), 3.16 (2H, dd, piperidine), 3.24 (1H, dd, piperidine)

<u>Intermediate 13: (3R)-Aminopiperidine</u>

Tetrahydrofuran (100 ml) was added to aluminum lithium hydride (0.92 g, 24.8 mmol) to prepare a suspension, and the suspension was ice cooled. Intermediate 12 (1.1 g, 9.9 mmol) was gradually added to the suspension in the internal temperature range of 5°C to 16°C. Ten min after the completion of the addition, the temperature of the mixture was raised to room temperature, and the mixture was further vigorously stirred for 3 hr. The mixture was ice cooled, and 5.6 ml of a 5 mol/liter aqueous sodium hydroxide solution was added to the cooled mixture. The temperature of the mixture was raised to room temperature, and the mixture was vigorously stirred for 2.0 hr. Anhydrous sodium sulfate was then added thereto, and the mixture was further stirred for 10 min and was filtered through Celite, followed by washing with tetrahydrofuran. A 4 mol/liter solution of hydrochloric acid in ethyl acetate (5.0 ml, 20.0 mmol) was added to the filtrate, and the solvent was removed by distillation under the reduced pressure. The residue was subjected to azeotropic distillation with methanol to give a dihydrochloride of the title compound (1.03 g, 60%).

Physicochemical properties of intermediate 13

(1) Color and form: Brown solid
(2) Molecular formula: $C_5H_{12}N_2$
(3) Mass spectrum (EIMS): m/z 100 $(M^+)$

(4) $^1H$ NMR spectrum (400 MHz, $CD_3OD$) (as dihydrochloride) δ (ppm): 1.75 (1H, dddd, piperidine), 1.90 (1H, m, piperidine), 2.09 (1H, ddddd, piperidine), 2.23 (1H, br d, piperidine), 3.02 (1H, ddd, piperidine), 3.09 (1H, dd, piperidine), 3.41 (1H, br d, piperidine), 3.62 (2H, m, piperidine)

Intermediate 14: Methyl 3-fluoro-4-{(3R)-(pyrimidin-2-ylamino)piperidin-1-yl}benzoate

N-Methylpyrrolidone (25 ml) was added to intermediate 13 (852 mg, 4.9 mmol) to prepare a solution, and sodium hydrogencarbonate (1.3 g, 15 mmol) and methyl 3,4-difluorobenzoate (0.33 g, 2.5 mmol) were added to the solution. The mixture was stirred at 110°C for 15 hr, and N,N-diisopropylethylamine (3.4 ml, 20 mmol) and 2-bromopyrimidine (1.37 g, 8.7 mmol) were then added to the reaction solution. The mixture was stirred at 100°C for 20 hr, 50 ml of water was then added thereto, and the mixture was extracted three times with ethyl acetate. The organic layers were combined, and the combined organic layers were dried over anhydrous magnesium sulfate and were then concentrated under the reduced pressure. The residue was purified by column chromatography on silica gel (development system: hexane : ethyl acetate = 7 : 3) to give the title compound (0.38 g, 48%).

Physicochemical properties of intermediate 14

(1) Color and form: Colorless syrup
(2) Molecular formula: $C_{17}H_{19}N_4O_2F$
(3) Mass spectrum (EIMS): m/z 330 ($M^+$)
(4) $^1H$ NMR spectrum (400 MHz, $CDCl_3$) δ (ppm): 1.76 (2H, m, piperidine), 1.92 (2H, m, piperidine), 3.15 (3H, m, piperidine), 3.47 (1H, dd, piperidine), 3.87 (3H, s, $OCH_3$), 4.25 (1H, m, piperidine), 5.52 (1H, d, NH), 6.52 (1H, t, pyrimidine), 6.97 (1H, dd, $C_6H_3$), 7.64 (1H, dd, $C_6H_3$), 7.72 (1H, dd, $C_6H_3$), 8.27 (2H, d, pyrimidine)

Intermediate 15: 3-Fluoro-4-{(3R)-(pyrimidin-2-ylamino)piperidin-1-yl}benzoic acid

Tetrahydrofuran (15 ml) and 4.8 ml of methanol were added to intermediate 14 (320 mg, 0.96 mmol) to prepare a solution, and 4.8 ml of a 1 mol/liter aqueous sodium hydroxide solution was added to the solution. The mixture was stirred at 40°C for 16 hr and was then concentrated under the reduced pressure. Water (8 ml) was added to the residue to prepare a solution. The solution was washed twice with ethyl acetate. The aqueous layer was adjusted to pH 4 by the addition of 1 mol/liter hydrochloric acid. The precipitated solid was separated by centrifugation and was then dried to give the title compound (280 mg, 92%).

Physicochemical properties of intermediate 15

(1) Color and form: Colorless solid
(2) Molecular formula: $C_{16}H_{17}N_4O_2F$
(3) Mass spectrum (EIMS): m/z 316 ($M^+$)
(4) $^1H$ NMR spectrum (400 MHz, DMSO-$d_6$) δ (ppm): 1.50 (1H, m, piperidine), 1.66 (1H, m, piperidine), 1.82 (1H, m, piperidine), 1.94 (1H, m, piperidine), 2.63 (1H, m, piperidine), 2.73 (1H, br t, piperidine), 3.30 (1H, m, piperidine), 3.56 (1H, br d, piperidine), 3.99 (1H, m, piperidine), 6.57 (1H, t, pyrimidine), 7.05 (1H, t, $C_6H_3$), 7.49 (1H, dd, $C_6H_3$), 7.61 (1H, dd, $C_6H_3$), 8.28 (2H, d, pyrimidine)

Example 7: t-Butyl (2S)-benzenesulfonylamino-3-[3-fluoro-4-{(3R)-(pyrimidin-2-ylamino)piperidin-1-yl}-benzoylamino] propionate

Dimethylformamide (4.3 ml) was added to intermediate 15 (140 mg, 0.44 mmol) to prepare a solution, and t-butyl (2S)-N-benzenesulfonyl-2,3-diaminopropionate (160 mg, 0.53 mmol) was added to the solution. Further, 1-hydroxybenzotriazole (77 mg, 0.58 mmol), N-methylmorpholine (0.14 ml, 1.3 mmol), and 1-ethyl-3-(3-dimethylaminopropyl)carbodiimide hydrochloride (110 mg, 0.58 mmol) were added thereto, and the mixture was stirred at room temperature for 20 hr. Water and a saturated aqueous potassium carbonate solution were added to the reaction solution, and the mixture was extracted three times with ethyl acetate. The organic layers were combined, and the combined organic layers were dried over anhydrous magnesium sulfate and were then concentrated under the reduced pressure. The residue was purified by column chromatography on silica gel (development system: hexane : ethyl acetate = 1 : 1) to

give the title compound (210 mg, 80%).

Physicochemical properties of compound prepared in Example 7

(1) Color and form: Colorless solid
(2) Molecular formula: $C_{29}H_{35}N_6O_5FS$
(3) Mass spectrum (FABMS): m/z 599 $(M+H)^+$
(4) Specific rotation: $[\alpha]_D^{25}+11°$ (c 1.0, MeOH)
(5) $^1H$ NMR spectrum (400 MHz, $CDCl_3$) δ (ppm): 1.29 (9H, S , t-Bu), 1.76 (2H, m, piperidine), 1.91 (2H, m, piperidine), 3.08 (3H, m, piperidine), 3.44 (1H, dd, piperidine), 3.56 (1H, m, CONH$\underline{CH_2}$CH), 3.89 (2H, m, CONH$\underline{CH_2CH}$), 4.24 (1H, m, piperidine), 5.58 (1H, d, NH), 5.78 (1H, brs, NH), 6.52 (1H, t, pyrimidine), 6.60 (1H, m, NH), 6.97 (1H, dd, $C_6H_3$), 7.50 (5H, m, $C_6H_3$ and $C_6H_5$), 7.84 (2H, m, $C_6H_5$), 8.28 (2H, d, pyrimidine)

<u>Example 8: (2S)-Benzenesulfonylamino-3-[3-fluoro-4-{(3R)-(pyrimidin-2-ylamino)piperidin-1-yl}benzoylamino]-propionic acid</u>

Methylene chloride (3.0 ml) was added to the compound prepared in Example 7 (176 mg, 0.29 mmol) to prepare a solution. Trifluoroacetic acid (3.0 ml) was added to the solution, and the mixture was stirred at room temperature for 6 hr. The reaction solution was concentrated under the reduced pressure to give a trifluoroacetate of the title compound.

Physicochemical properties of compound prepared in Example 8

(1) Color and form: Colorless solid
(2) Molecular formula: $C_{25}H_{27}N_6O_5FS$
(3) Mass spectrum (TSPMS): m/z 543 $(M+H)^+$
(4) $^1H$ NMR spectrum (400 MHz, $CD_3OD$) δ (ppm): 1.79 (2H, m, piperidine), 2.00 (2H, m, piperidine), 3.11 (2H, m, piperidine), 3.26 (1H, m, piperidine), 3.47 (1H, dd, CONH$\underline{CH_2}$CH), 3.49 (1H, m, piperidine), 3.71 (1H, dd, CONH$\underline{CH_2}$CH), 4.17 (1H, dd, CONH$\underline{CH_2}$CH), 4.30 (1H, m, piperidine), 6.90 (1H, t, pyrimidine), 7.09 (1H, dd, $C_6H_3$), 7.47 (5H, m, $C_6H_3$ and $C_6H_5$), 7.80 (2H, m, $C_6H_5$), 8.53 (2H, brs, pyrimidine)

<u>Example 9: (2S)-Benzenesulfonylamino-3-[3-fluoro-4-{(3R)-(1,4,5,6-tetrahydropyrimidin-2-ylamino)piperidin1-yl} benzoylamino]propionic acid</u>

Dioxane (2.0 ml) and 0.2 ml of water were added to the trifluoroacetate of the crude compound (0.11 mmol) prepared in Example 8 to prepare a solution. To the solution was added 20 mg of 10% palladium-carbon. The mixture was vigorously stirred under a hydrogen pressure of 1 atm at room temperature for 16 hr. The reaction solution was filtered through Celite, followed by washing with dioxane and water. The filtrate and the washings were combined, and the combined solution was concentrated under the reduced pressure. The residue was purified by preparative thin-layer chromatography on silica gel (development system: methylene chloride : ethanol : water : concentrated aqueous ammonia = 8 : 8 : 1 : 1) and was then purified by Sephadex LH-20 (development system: methanol) to give the title compound (46 mg, 77%).

Physicochemical properties of compound prepared in Example 9

(1) Color and form: Colorless solid
(2) Molecular formula: $C_{25}H_{31}N_6O_5FS$
(3) Mass spectrum (TSPMS): m/z 547 $(M+H)^+$
(4) $^1H$ NMR spectrum (400 MHz, $CD_3OD$) δ (ppm): 1.67 (1H, m, piperidine), 1.77 (1H, m, piperidine), 1.90 (2H, m, piperidine), 1.96 (2H, quintet, tetrahydropyrimidine), 3.11 (3H, m, piperidine), 3.28 (1H, m, piperidine), 3.37 (4H, t, tetrahydropyrimidine), 3.52 (1H, dd, CONH$\underline{CH_2}$CH), 3.68 (1H, dd, CONH$\underline{CH_2}$CH), 3.72 (1H, m, piperidine), 3.89 (1H, dd, CONHCH$_2\underline{CH}$), 7.04 (1H, dd, $C_6H_3$), 7.50 (5H, m, $C_6H_3$ and $C_6H_5$), 7.85 (2H, m, $C_6H_5$)

Example 10: t-Butyl (2S)-(benzyloxycarbonyl)amino-3-[3-fluoro-4-{(3S)-(pyrimidin-2-ylamino)piperidin-1-yl}-benzoylamino]propionate

Dimethylformamide (4.8 ml) was added to intermediate 11 (75 mg, 0.24 mmol) to prepare a solution. N,N-Diisopropylethylamine (0.083 ml, 0.48 mmol) and BOP reagent (128 mg, 0.29 mmol) were added to the solution, and the mixture was stirred at room temperature for 30 min. t-Butyl (2S)-N-benzyloxycarbonyl-2,3-diaminopropionate hydrochloride (85 mg, 0.29 mmol) was then added thereto, and the mixture was stirred at room temperature for 4 hr. Water was added to the reaction solution, and the mixture was extracted three times with ethyl acetate. The organic layers were combined, and the combined organic layers were dried over anhydrous magnesium sulfate and were then concentrated under the reduced pressure. The residue was purified by column chromatography on silica gel (development system: hexane : ethyl acetate = 1 : 3) to give the title compound (140 mg, 100%).

Physicochemical properties of compound prepared in Example 10

(1) Color and form: Colorless solid
(2) Molecular formula: $C_{31}H_{37}N_6O_5F$
(3) Mass spectrum (FABMS): m/z 593 $(M+H)^+$
(4) $^1H$ NMR spectrum (400 MHz, $CDCl_3$) $\delta$ (ppm): 1.45 (9H, s, t-Bu), 1.75 (2H, m, piperidine), 1.92 (2H, m, piperidine), 3.10 (1H, m, piperidine), 3.14 (1H, m, piperidine), 3.42 (1H, m, piperidine), 3.79 (3H, m, CONH$\underline{CH_2}$CH and piperidine), 4.25 (1H, m, piperidine), 4.44 (1H, m, CONHCH$_2$$\underline{CH}$), 5.10 (2H, s, $CH_2C_6H_5$), 5.58 (1H, d, NH), 5.83 (1H, d, NH), 6.51 (1H, t, pyrimidine), 6.84 (1H, brs, NH), 6.97 (1H, dd, $C_6H_3$), 7.32 (7H, m, $C_6H_3$ and $C_6H_5$), 8.27 (2H, d, pyrimidine)

Example 11: t-Butyl (2S)-amino-3-[3-fluoro-4-{(3S)-(pyrimidin-2-ylamino}piperidin-1-yl}benzoylamino]-propionate

Tetrahydrofuran was added to the compound (128 mg, 0.21 mmol) prepared in Example 10 to prepare a solution. To the solution was added 100 mg of 5% palladium-carbon. The mixture was vigorously stirred under a hydrogen pressure of 1 atm at room temperature for 12 hr. The reaction solution was filtered through Celite, followed by washing with tetrahydrofuran and ethanol. The filtrate and the washings were combined, and the combined solution was concentrated under the reduced pressure. The residue was purified by column chromatography on silica gel (development system: chloroform : methanol = 8 : 2) to give the title compound (80 mg, 83%).

Physicochemical properties of compound prepared in Example 11

(1) Color and form: Colorless solid
(2) Molecular formula: $C_{23}H_{31}N_6O_3F$
(3) Mass spectrum (EIMS): m/z 458 $(M^+)$
(4) Specific rotation: $[\alpha]_D^{25}$ +57° (c 0.23, $CHCl_3$)
(5) $^1H$ NMR spectrum (400 MHz, $CDCl_3$) $\delta$ (ppm): 1.47 (9H, s, t-Bu), 1.76 (2H, m, piperidine), 1.89 (2H, m, piperidine), 3.14 (3H, m, piperidine), 3.43 (2H, m, piperidine and CONH$\underline{CH_2}$CH) ,3.57 (1H, m, CONH$\underline{CH_2}$CH), 3.79 (1H, m, CONHCH$_2$$\underline{CH}$), 4.24 (1H, m, piperidine), 5.55 (1H, d, NH), 6.52 (1H, t, pyrimidine), 6.68 (1H, m, NH), 6.97 (1H, dd, $C_6H_3$), 7.45 (2H, m, $C_6H_3$), 8.27 (2H, d, pyrimidine)

Example 12: t-Butyl 3-[3-fluoro-4-{(3S)-(pyrimidin-2-ylamino)piperidin-1-yl}benzoylamino]-(2S)-{(4-methoxybenzenesulfonyl)amino}propionate

Dimethylformamide (3.0 ml) was added to the compound (70 mg, 0.15 mmol) prepared in Example 11 to prepare a solution. N,N-Diisopropylethylamine (0.055 ml, 0.30 mmol) and 4-methoxybenzenesulfonyl chloride (31 mg, 0.15 mmol) were adeed to the solution, and the mixture was stirred at room temperature for 3 hr. Piperidine was added to the reaction solution, water and a saturated aqueous sodium hydrogencarbonate solution were then added thereto, and the mixture was extracted three times with ethyl acetate. The organic layers were combined, and the combined organic layers were dried over anhydrous magnesium sulfate and were then concentrated under the reduced pressure. The residue was purified by column chromatography on silica gel (development system: chloroform : methanol = 8 : 2) to give the title compound (80 mg, 83%).

Physicochemical properties of compound prepared in Example 12

(1) Color and form: Colorless solid
(2) Molecular formula: $C_{30}H_{37}N_6O_6FS$
(3) Mass spectrum (TSPMS): m/z 629 $(M+H)^+$
(4) $^1$H NMR spectrum (400 MHz, $CDCl_3$) δ (ppm): 1.31 (9H, s, t-Bu), 1.77 (2H, m, piperidine), 1.92 (2H, m, piperidine), 3.09 (1H, dd, piperidine), 3.15 (2H, m, piperidine), 3.43 (1H, dd, piperidine), 3.52 (1H, ddd, CONH$\underline{CH_2}$CH), 3.84 (3H, s, OMe), 3.90 (2H, m, CONH$\underline{CH_2CH}$), 4.25 (1H, m, piperidine), 5.56 (1H, d, NH), 6.52 (1H, t, pyrimidine), 6.61 (1H, brs, NH), 6.94 (2H, d, $C_6H_4$), 6.98 (1H, dd, $C_6H_3$), 7.48 (2H, m, $C_6H_3$), 7.76 (2H, d, $C_6H_4$), 8.28 (2H, d, pyrimidine)

Example 13: 3-[3-Fluoro-4-{(3S)-(pyrimidin-2-ylamino)piperidin-1-yl}benzoylamino]-(2S)-{(4-methoxybenzenesulfonyl)amino}propionic acid

Methylene chloride (2.0 ml) was added to the compound (30 mg, 0.048 mmol) prepared in Example 12 to prepare a solution. Trifluoroacetic acid (0.5 ml) was added to the solution, and the mixture was stirred at room temperature for 4 hr. The reaction solution was concentrated under the reduced pressure to give the title compound.

Physicochemical properties of compound prepared in Example 13

(1) Color and form: Colorless solid
(2) Molecular formula: $C_{25}H_{29}N_6O_6FS$

Example 14: 3-[3-Fluoro-4-{(3S)-(1,4,5,6-tetrahydropyrimidin-2-ylamino)piperidin-1-yl}benzoylamino]-(2S)-{(4-methoxybenzenesulfonyl)amino}propionic acid

Tetrahydrofuran (2.0 ml) and 0.2 ml of water were added to the compound (0.048 mmol) prepared in Example 13 to prepare a solution. To the solution was added 50 mg of 5% palladium-carbon. The mixture was vigorously stirred under a hydrogen pressure of 1 atm at room temperature for 15 hr. The reaction solution was filtered through Celite, followed by washing with tetrahydrofuran and ethanol. The filtrate and the washings were combined, and the combined solution was concentrated under the reduced pressure. The residue was purified by preparative thin-layer chromatography on silica gel (development system: chloroform : methanol : concentrated aqueous ammonia = 8 : 2 : 0.2) and was then purified by Sephadex LH-20 (development system: methanol) to give the title compound (20 mg, 72%).

Physicochemical properties of compound prepared in Example 14

(1) Color and form: Colorless solid
(2) Molecular formula: $C_{26}H_{33}N_6O_6FS$
(3) Mass spectrum (TSPMS): m/z 577 $(M+H)^+$
(4) $^1$H NMR spectrum (400 MHz, $CDCl_3$) δ (ppm): 1.67 (1H, m, piperidine), 1.78 (1H, m, piperidine), 1.91 (2H, m, piperidine), 1.96 (2H, quintet, tetrahydropyrimidine), 3.12 (3H, m, piperidine), 3.27 (1H, m, piperidine), 3.36 (4H, t, tetrahydropyrimidine), 3.48 (1H, dd, CONH$\underline{CH_2}$CH), 3.68 (1H, dd, CONH$\underline{CH_2}$CH), 3.72 (1H, m, piperidine), 3.80 (3H, s, OMe), 3.85 (1H, dd, $CONHCH_2\underline{CH}$), 6.94 (2H, d, $C_6H_4$), 7.05 (1H, dd, $C_6H_3$), 7.48 (1H, dd, $C_6H_3$), 7.53 (1H, dd, $C_6H_3$), 7.74 (2H, d, $C_6H_4$)

Example 15: 3-[3-Fluoro-4-{(3S)-(pyrimidin-2-ylamino)piperidin-1-yl}benzoylamino]-(2S)-{(4-hydroxybenzenesulfonyl)amino}propionic acid

1,2-Dichloroethane (7.0 ml) was added to the compound (45 mg, 0.07mmol) prepared in Example 13 to prepare a solution. A 1.0 mol/l solution (0.7 ml) of boron tribromide in dichloromethane was then added to the solution, and the mixture was stirred at 40°C for 4 hr. The reaction solution was concentrated under the reduced pressure, and the residue was then purified by preparative column chromatography on silica gel (development system: chloroform :

methanol : concentrated aqueous ammonia = 8 : 2 : 0.2) to give the title compound (39 mg, 100%).

Physicochemical properties of compound prepared in Example 15

(1) Color and form: Colorless solid
(2) Molecular formula: $C_{25}H_{27}N_6O_6FS$
(3) Mass spectrum (FABMS): m/z 559 $(M+H)^+$

Example 16: 3-[3-Fluoro-4-{(3S)-(1,4,5,6-tetrahydropyrimidin-2-ylamino)piperidin-1-yl}benzoylamino]-(2S)-{(4-hydroxybenzenesulfonyl)amino}propionic acid

Tetrahydrofuran (3.5 ml), 1.0 ml of water, and 0.5 ml of acetic acid were added to the compound (45 mg, 0.08 mmol) prepared in Example 15 to prepare a solution. To the solution was added 50 mg of 5% palladium-carbon. The mixture was vigorously stirred under a hydrogen pressure of 1 atm at room temperature for 15 hr. The reaction solution was filtered through Celite, followed by washing with tetrahydrofuran and ethanol. The filtrate and the washings were combined, and the combined solution was concentrated under the reduced pressure. The residue was purified by preparative thin-layer chromatography on silica gel (development system: chloroform : methanol : concentrated aqueous ammonia = 8 : 2 : 0.2) and was then purified by Sephadex LH-20 (development system: methanol) to give the title compound.

Physicochemical properties of compound prepared in Example 16

(1) Color and form: Colorless solid
(2) Molecular formula: $C_{25}H_{31}N_6O_6FS$
(3) Mass spectrum (TSPMS): m/z 563 $(M+H)^+$

Intermediate 16: Methyl 2,3-difluoro-4-{(3S)-(pyrimidin-2-ylamino)piperidin-1-yl}benzoate

N-Methylpyrrolidone (40 ml) was added to intermediate 8 (15.0 mmol) to prepare a solution. Sodium hydrogencarbonate (4.0 g, 48.0 mmol) and methyl 2,3,4-trifluorobenzoate (1.43 g, 7.52 mmol) were added to the solution, and the mixture was then stirred at 110°C for 15 hr. N,N-Diisopropylethylamine (10.4 ml, 60.0 mmol) and 2-bromopyrimidine (3.6 g, 23.3 mmol) were then added to the reaction solution, and the mixture was stirred at 100°C for 20 hr. Water (100 ml) was then added thereto, and the mixture was extracted three times with ethyl acetate. The organic layers were combined, and the combined organic layers were dried over anhydrous magnesium sulfate and were then concentrated under the reduced pressure. The residue was purified by column chromatography on silica gel (development system: hexane : ethyl acetate = 7 : 3) to give the title compound (0.17 g, 7.0%).

Physicochemical properties of intermediate 16

(1) Color and form: Colorless syrup
(2) Molecular formula: $C_{17}H_{18}N_4O_2F_2$
(3) Mass spectrum (EIMS): m/z 348 $(M^+)$
(4) Specific rotation: $[\alpha]_D^{24}$ +43° (c 0.56, $CHCl_3$)
(5) $^1H$ NMR spectrum (400 MHz, $CDCl_3$) $\delta$ (ppm): 1.75 (2H, m, piperidine), 1.94 (2H, m, piperidine), 3.07 (1H, dd, piperidine), 3.16 (1H, ddd, piperidine), 3.32 (1H, m piperidine), 3.58 (1H, dd, piperidine), 3.89 (3H, s, $OCH_3$), 4.21 (1H, m, piperidine), 5.38 (1H, d, NH), 6.54 (1H, t, pyrimidine), 6.75 (1H, ddd, $C_6H_2$), 7.60 (1H, ddd, $C_6H_2$), 8.27 (2H, d, pyrimidine)

Intermediate 17: 2,3-Difluoro-4-{(3S)-(pyrimidin-2-ylamino)piperidin-1-yl}benzoic acid

Tetrahydrofuran (6.3 ml) and 2.1 ml of methanol were added to intermediate 16 (150 mg, 0.43 mmol) to prepare a solution. A 1 mol/liter aqueous sodium hydroxide solution (2.1 ml) was added to the solution. The mixture was stirred

at 40°C for 5 hr and was then concentrated under the reduced pressure. Water (8 ml) was added to the residue to prepare a solution which was then washed twice with ethyl acetate. The aqueous layer was adjusted to pH 4 by the addition of 1 mol/liter hydrochloric acid. The precipitated solid was separated by centrifugation and was then dried to give the title compound (104 mg, 72%).

Physicochemical properties of intermediate 17

(1) Color and form: Colorless solid
(2) Molecular formula: $C_{16}H_{16}N_4O_2F_2$
(3) Mass spectrum (EIMS): m/z 334 ($M^+$)

Example 17: t-Butyl (2S)-benzenesulfonylamino-3-[2,3-difluoro-4-{(3S)-(pyrimidin-2-ylamino)piperidin-1-yl}-benzoylamino]propionate

Dimethylformamide (1.5 ml) was added to intermediate 17 (50 mg, 0.15 mmol) to prepare a solution, and t-butyl (2S)-N-benzenesulfonyl-2,3-diaminopropionate (54 mg, 0.18 mmol) was added to the solution. Further, 1-hydroxybenzotriazole (26 mg, 0.20 mmol), N-methylmorpholine (0.050 ml, 0.45 mmol), and 1-ethyl-3-(3-dimethylaminopropyl)carbodiimide hydrochloride (37mg, 0.20 mmol) were added thereto, and the mixture was stirred at room temperature for 20 hr. Water and a saturated aqueous potassium carbonate solution were added to the reaction solution, and the mixture was extracted three times with ethyl acetate. The organic layers were combined, and the combined organic layers were dried over anhydrous magnesium sulfate and were then concentrated under the reduced pressure. The residue was purified by column chromatography on silica gel (development system: hexane : ethyl acetate = 1 : 3) to give the title compound (84 mg, 91%).

Physicochemical properties of compound prepared in Example 17

(1) Color and form: Colorless solid
(2) Molecular formula: $C_{29}H_{34}N_6O_5F_2S$
(3) Mass spectrum (FABMS): m/z 617 $(M+H)^+$
(4) Specific rotation: $[\alpha]_D^{25}$ +66° (c 0.65, $CHCl_3$)
(5) $^1H$ NMR spectrum (400 MHz, $CDCl_3$) δ (ppm): 1.29 (9H, s, t-Bu), 1.74 (2H, m, piperidine), 1.97 (2H, m, piperidine), 3.03 (1H, dd, piperidine), 3.12 (1H, ddd, piperidine), 3.31 (1H, m, piperidine), 3.55 (1H, m, piperidine), 3.71 (1H, m, CONH$\underline{CH_2}$CH), 3.79 (1H, m, CONH$\underline{CH_2}$CH), 4.01 (1H, m, CONH$CH_2$$\underline{CH}$), 4.20 (1H, m, piperidine), 5.47 (1H, d, NH), 5.80 (1H, d, NH), 6.53 (1H, t, pyrimidine), 6.80 (1H, m, $C_6H_2$), 7.45 (2H, m, $C_6H_5$), 7.52 (1H, m, $C_6H_5$), 7.67 (1H, ddd, $C_6H_2$), 7.83 (2H, m, $C_6H_5$), 8.28 (2H, d, pyrimidine)

Example 18: (2S)-Benzenesulfonylamino-3-[2,3-difluoro-4-{(3S)-(pyrimidin-2-ylamino)piperidin-1-yl}benzoylamino]-propionic acid

Methylene chloride (1.1 ml) was added to the compound (70 mg, 0.11 mmol) prepared in Example 17 to prepare a solution. Trifluoroacetic acid (1.1 ml) was added to the solution, and the mixture was stirred at room temperature for 7 hr. The reaction solution was concentrated under the reduced pressure to give a trifluoroacetate of the title compound.

Physicochemical properties of compound prepared in Example 18

(1) Color and form: Colorless solid
(2) Molecular formula: $C_{25}H_{26}N_6O_5F_2S$
(3) Mass spectrum (FABMS): m/z 561 $(M+H)^+$

Example 19: (2S)-Benzenesulfonylamino-3-[2,3-difluoro-4- {(3S)-(1,4,5,6-tetrahydropyrimidin-2-ylamino)piperidin-1-yl}benzoylamino]propionic acid

Dioxane (1.2 ml) and 0.2 ml of water were added to the trifluoroacetate (0.062 mmol) of the crude compound prepared in Example 18 to prepare a solution. To the solution was added 10 mg of 10% palladium-carbon. The mixture was vigorously stirred under a hydrogen pressure of 1 atm at room temperature for 16 hr. The reaction solution was filtered through Celite, followed by washing with dioxane and water. The filtrate and the washings were combined, and the combined solution was concentrated under the reduced pressure. The residue was purified by preparative thin-layer chromatography on silica gel (development system: methylene chloride : ethanol : water : concentrated aqueous ammonia = 8 : 8 : 1 : 1) and was then purified by Sephadex LH-20 (development system: methanol) to give the title compound (35 mg, 100%).

Physicochemical properties of compound prepared in Example 19

(1) Color and form: Colorless solid
(2) Molecular formula: $C_{25}H_{30}N_6O_5F_2S$
(3) Mass spectrum (TSPMS): m/z 565 $(M+H)^+$
(4) Specific rotation: $[\alpha]_D^{28}$+39° (c 0.55, MeOH)
(5) $^1H$ NMR spectrum (400 MHz, $CD_3OD$) δ (ppm): 1.67 (1H, m, piperidine), 1.78 (1H, m, piperidine), 1.91 (2H, m, piperidine), 1.96 (2H, quintet, tetrahydropyrimidine), 3.12 (3H, m, piperidine), 3.19 (1H, m, piperidine), 3.37 (4H, t, tetrahydropyrimidine), 3.63 (3H, m, CONH$\underline{CH_2}$CH and piperidine), 3.76 (1H, dd, CONH$CH_2$$\underline{CH}$), 6.85 (1H, dd, $C_6H_2$), 7.50 (4H, m, $C_6H_2$ and $C_6H_5$), 7.84 (2H, m, $C_6H_5$)

Intermediate 18: Methyl 3-chloro-4-{(3S)-(pyrimidin-2-ylamino)piperidin-1-yl}benzoate

N-Methylpyrrolidone (40 ml) was added to intermediate 8 (2.5 g, 15 mmol) to prepare a solution, and sodium hydrogencarbonate (4.0 g, 48 mmol) and methyl 3-chloro-4-fluorobenzoate (1.4 g, 7.5 mmol) were added to the solution. The mixture was stirred at 110°C for 15 hr. N,N-Diisopropylethylamine (10.4 ml, 60 mmol) and 2-bromopyrimidine (3.6 g, 22 mmol) were then added to the reaction solution, and the mixture was stirred at 100°C for 20 hr. Water (50 ml) was then added thereto, and the mixture was extracted three times with ethyl acetate. The organic layers were combined, and the combined organic layers were dried over anhydrous magnesium sulfate and were then concentrated under the reduced pressure. The residue was purified by column chromatography on silica gel (development system: hexane : ethyl acetate = 1 : 1) to give the title compound (370 mg, 14%).

Physicochemical properties of intermediate 18

(1) Color and form: Colorless solid
(2) Molecular formula: $C_{17}H_{19}N_4O_2Cl$
(3) Mass spectrum (TSPMS): m/z 347 $(M+H)^+$
(4) Specific rotation: $[\alpha]_D^{26}$ +43° (c 0.19, $CHCl_3$)
(5) $^1H$ NMR spectrum (400 MHz, $CDCl_3$) δ (ppm) : 1.80 (2H, m, piperidine), 1.87 (1H, m, piperidine), 1.97 (1H, m, piperidine), 3.11 (3H, m, piperidine), 3.32 (1H, m, piperidine), 3.88 (3H, s, OMe), 4.30 (1H, m, piperidine), 5.69 (1H, brs, NH), 6.51 (1H, t, pyrimidine), 7.04 (1H, d, $C_6H_3$), 7.86 (1H, dd, $C_6H_3$), 8.01 (1H, d, $C_6H_3$), 8.26 (2H, d, pyrimidine)

Intermediate 19: 3-Chloro-4-{(3S)-(pyrimidin-2-ylamino)-piperidin-1-yl}benzoic acid

Tetrahydrofuran (13.5 ml) and 4.5 ml of methanol were added to intermediate 18 (310 mg, 0.90 mmol) to prepaer a solution, and 4.5 ml of a 1 mol/liter aqueous sodium hydroxide solution was added to the solution. The mixture was stirred at 40°C for 4 hr and was then concentrated under the reduced pressure. Water (20 ml) of water was added to the residue to prepare a solution which was then washed twice with ethyl acetate. The aqueous layer was adjusted to pH 4 by the addition of 1 mol/liter hydrochloric acid. The precipitated solid was separated by centrifugation and was then dried to give the title compound (210 mg, 70%).

Physicochemical properties of intermediate 19

(1) Color and form: Colorless solid
(2) Molecular formula: $C_{16}H_{17}N_4O_2Cl$
(3) Mass spectrum (FABMS): m/z 333 $(M+H)^+$

Example 20: t-Butyl (2S)-benzenesulfonylamino-3-[3-chloro-4-{(3S)-(pyrimidin-2-ylamino)piperidin-1-yl}benzoylamino]propionate

Dimethylformamide (5.4 ml) was added to intermediate 19 (90 mg, 0.27 mmol) to prepare a solution, and t-butyl (2S)-N-benzenesulfonyl-2,3-diaminopropionate (98 mg, 0.32 mmol) was added to the solution. Further, 1-hydroxybenzotriazole (47 mg, 0.35 mmol), N-methylmorpholine (0.089 ml, 0.81 mmol), and 1-ethyl-3-(3-dimethylaminopropyl)carbodiimide hydrochloride (67 mg, 0.35 mmol) were added thereto, and the mixture was stirred at room temperature for 20 hr. Water and a saturated aqueous potassium carbonate solution were added to the reaction solution, and the mixture was extracted three times with ethyl acetate. The organic layers were combined, and the combined organic layers were dried over anhydrous magnesium sulfate and were then concentrated under the reduced pressure. The residue was purified by column chromatography on silica gel (development system: hexane : ethyl acetate = 1 : 3) to give the title compound (188 mg, 100%).

Physicochemical properties of compound prepared in Example 20

(1) Color and form: Colorless solid
(2) Molecular formula: $C_{29}H_{36}N_6O_5ClS$
(3) Mass spectrum (TSPMS): m/z 617 $(M+H)^+$
(4) Specific rotation: $[\alpha]_D^{26}$+86° (c 0.65, $CHCl_3$)
(5) $^1H$ NMR spectrum (400 MHz, $CDCl_3$) δ (ppm): 1.29 (9H, s, t-Bu), 1.79 (2H, m, piperidine), 1.86 (1H, m, piperidine), 1.97 (1H, m, piperidine), 3.07 (3H, m, piperidine), 3.27 (1H, m, piperidine), 3.57 (1H, m, CONH$\underline{CH_2}$CH), 3.88 (2H, m, CONH$\underline{CH_2CH}$), 4.29 (1H, m, piperidine), 5.74 (1H, d, NH), 5.77 (1H, m, NH), 6.51 (1H, t, pyrimidine), 6.61 (1H, d, NH), 7.04 (1H, d, $C_6H_3$), 7.55 (4H, m, $C_6H_5$), 7.79 (1H, d, $C_6H_3$), 7.83 (1H, dd, $C_6H_3$), 7.84 (1H, m, $C_6H_3$), 8.27 (2H, d, pyrimidine)

Example 21: (2S)-Benzenesulfonylamino-3-[3-chloro-4-{(3S)-(pyrimidin-2-ylamino)piperidin-1-yl}benzoylamino]-propionic acid

Methylene chloride (2.2 ml) was added to the compound (140 mg, 0.22 mmol) prepared in Example 20 to prepare a solution. Trifluoroacetic acid (2.2 ml) was added to the solution, and the mixture was stirred at room temperature for 3 hr. The reaction solution was concentrated under the reduced pressure to give a trifluoroacetate of the title compound.

Physicochemical properties of compound prepared in Example 21 (as trifluoroacetate)

(1) Color and form: Colorless solid
(2) Molecular formula: $C_{25}H_{27}N_6O_5ClS$
(3) Mass spectrum (TSPMS): m/z 559 $(M+H)^+$
(4) Specific rotation: $[\alpha]_D^{26}$+41° (c 0.38,MeOH)
(5) $^1H$ NMR spectrum (400 MHz, $CD_3OD$) δ (ppm): 1.77 (1H, m, piperidine), 1.85 (1H, m, piperidine), 2.00 (2H, m, piperidine), 3.03 (2H, m, piperidine), 3.26 (1H, m, piperidine), 3.42 (1H, m, piperidine), 3.47 (1H, m, CONH$\underline{CH_2}$CH), 3.71 (1H, dd, CONH$\underline{CH_2}$CH), 4.19 (1H, dd, CONH$CH_2$$\underline{CH}$), 4.31 (1H, m, piperidine), 6.84 (1H, t, pyrimidine), 7.18 (1H, d, $C_6H_3$), 7.41 (2H, m, $C_6H_5$), 7.47 (1H, m, $C_6H_5$), 7.65 (1H, dd, $C_6H_3$), 7.76 (1H, d, $C_6H_3$), 7.80 (2H, m, $C_6H_5$), 8.47 (2H, m, pyrimidine)

Example 22: (2S)-Benzenesulfonylamino-3-[3-chloro-4-{(3S)-(1,4,5,6-tetrahydropyrimidin-2-ylamino)piperidin-1-yl}benzoylamino]propionic acid

Acetic acid (25 ml) and 2.5 ml of concentrated hydrochloric acid were added to the trifluoroacetate of the crude compound (0.050 mmol) prepared in Example 21 to prepare a solution. To the solution was added 23 mg of 10% palladium-carbon, and the mixture was vigorously stirred under a hydrogen pressure of 1 atm at room temperature for 16 hr. The reaction solution was filtered through Celite, followed by washing with water. The filtrate and the washings were combined, and the combined solution was concentrated under the reduced pressure. The residue was purified by preparative thin-layer chromatography on silica gel (development system: methylene chloride : ethanol : water : concentrated aqueous ammonia = 8 : 8 : 1 : 1) and was then purified by Sephadex LH-20 (development system: methanol) to give the title compound (22 mg, 78%).

Physicochemical properties of compound prepared in Example 22

(1) Color and form: Colorless solid
(2) Molecular formula: $C_{25}H_{31}N_6O_5ClS$
(3) Mass spectrum (TSPMS): m/z 565 $(M+H)^+$
(4) Specific rotation: $[\alpha]_D^{28}+51°$ (c 0.17, MeOH)
(5) $^1H$ NMR spectrum (400 MHz, $CD_3OD$) $\delta$ (ppm): 1.64 (1H, m, piperidine), 1.83 (1H, m, piperidine), 1.96 (4H, m, piperidine and tetrahydropyrimidine), 3.12 (1H, m, piperidine), 3.20 (2H, m, piperidine), 3.36 (4H, t, tetrahydropyrimidine), 3.49 (2H, m, CONH$\underline{CH_2}$CH and piperidine), 3.70 (2H, m, CONH$\underline{CH_2}$CH and piperidine), 3.98 (1H, dd, $CONHCH_2\underline{CH}$), 7.16 (1H, d, $C_6H_3$), 7.46 (3H, m, $C_6H_5$), 7.69 (1H, dd, $C_6H_3$), 7.81(3H, m, $C_6H_5$ and $C_6H_3$)

Intermediate 20: 4-{(3S)-Aminopiperidin-1-yl}benzonitrile

Sodium hydrogencarbonate (21.4 g, 255 mmol) was placed in a pressure test tube, and 30 ml of N-methylpyrrolidone was added thereto to prepare a suspension. 4-Fluorobenzonitrile (6.7 g, 55.5 mmol) and intermediate 8 (14.3 g, 82.6 mmol) were added in that order to the pressure test tube. The mixture was stirred at room temperature for 5 min in such a state that the system was opened. Thereafter, the system was hermetically sealed, and, in this state, stirring was carried out at 120°C for 22 hr. The temperature of the reaction solution was returned to room temperature, and the reaction system was then purified by chromatography on silica gel (development system: methylene chloride : methanol : aqueous ammonia = 100 : 10 : 1) to give the title compound (9.89 g, 89%).

Physicochemical properties of intermediate 20

(1) Color and form: Light yellow syrup
(2) Molecular formula: $C_{12}H_{15}N_3$
(3) Mass spectrum (TSPMS): m/z 202 $(M+H)^+$
(4) $^1H$ NMR spectrum (400 MHz, $CDCl_3$) $\delta$ (ppm): 1.33 (1H, dddd, piperidine), 1.63 (1H, ddddd, piperidine), 1.79 - 1.87 (1H, m, piperidine), 1.95 - 2.04 (1H, m, piperidine), 2.74 (1H, dd, piperidine), 2.90 - 2.99 (2H, m, piperidine), 3.61 (1H, ddd, piperidine), 3.72 (1H, dddd, piperidine), 6.86 (2H, d, $C_6H_4$), 7.47 (2H, d, $C_6H_4$)

Intermediate 21: 4-{(3S)-(Pyrimidin-2-ylamino)piperidin-1-yl}benzonitrile

Dimethyl sulfoxide (250 ml) was added to 2-bromopyrimidine (8.72 g, 54.9 mmol) and intermediate 20 (9.89 g, 49.2 mmol) to prepare a solution. Diisopropylethylamine (50 ml, 287 mmol) was added to the solution, and the mixture was vigorously stirred at 120°C for 12 hr. The temperature of the reaction solution was returned to room temperature. Water (500 ml) was then added to the reaction solution, and the mixture was extracted three times with 500 ml of ethyl acetate. The organic layers were combined, and the combined organic layers were washed three times with 500 ml of water and once with 500 ml of saturated brine, were dried over anhydrous magnesium sulfate, and were then concentrated under the reduced pressure. The residue was purified by chromatography on silica gel (development system: hexane : ethyl acetate = 1 : 1 → only ethyl acetate) to give the title compound (10.5 g, 77%).

Physicochemical properties of intermediate 21

(1) Color and form: Light yellow solid
(2) Molecular formula: $C_{16}H_{17}N_5$
(3) Mass spectrum (EIMS): m/z 279 $(M)^+$
(4) Specific rotation: $[\alpha]_D^{26}$ +46° (c 0.50, $CHCl_3$)
(5) $^1$H NMR spectrum (400 MHz, $CDCl_3$) δ (ppm): 1.60 - 1.72 (1H, m, piperidine), 1.75 (1H, dddd, piperidine), 1.83 - 1.92 (1H, m, piperidine), 2.01 - 2.10 (1H, m, piperidine), 3.03 (1H, dd, piperidine), 3.17 (1H, ddd, piperidine), 3.56 (1H, ddd, piperidine), 3.92 (1H, dd, piperidine), 4.09 (1H, ddddd, piperidine), 6.58 (1H, t, pyrimidine), 6.92 (2H, d, $C_6H_4$), 7.47 (2H, d, $C_6H_4$), 8.30 (2H, d, pyrimidine)

Intermediate 22: 4-{(3S)-(Pyrimidin-2-ylamino)piperidin-1-yl}benzoic acid

Intermediate 21 (7.75 g, 27.7 mmol) was dissolved in 28 ml of 50% sulfuric acid to prepare a solution which was then heated under reflux for 2 hr. The temperature of the reaction solution was returned to room temperature, and the reaction solution was then slowly poured into 800 ml of a saturated aqueous sodium hydrogencarbonate solution under ice cooling. The mixture was adjusted to pH 4 by the addition of 1.0 mol/liter hydrochloric acid and a saturated aqueous sodium hydrogencarbonate solution. The precipitate was collected by filtration through a glass filter, was washed with water, and was then dried to give the title compound (8.11 g, 98%).

Physicochemical properties of intermediate 22

(1) Color and form: Colorless solid
(2) Molecular formula: $C_{16}H_{18}N_4O_2$
(3) Mass spectrum (EIMS): m/z 298 $(M)^+$
(4) $^1$H NMR spectrum (400 MHz, DMSO-$\underline{d}_6$) δ (ppm): 1.52 - 1.61 (2H, m, piperidine), 1.73 - 1.81 (1H, m, piperidine), 1.93 - 2.01 (1H, m, piperidine), 2.76 (1H, dd, piperidine), 2.87 (1H, ddd, piperidine), 3.79 - 3.91 (2H, m, piperidine), 3.98 (1H, br d, piperidine), 6.58 (1H, t, pyrimidine), 6.96 (2H, d, $C_6H_4$), 7.74 (2H, d, $C_6H_4$), 8.29 (2H, d, pyrimidine)

Example 23: t-Butyl (2S)-benzenesulfonylamino-3-[4-{(3S)-(pyrimidin-2-ylamino)piperidin-1-yl}benzoylamino]-propionate

Dimethylformamide (3.4 ml) was added to intermediate 22 (50 mg, 0.17 mmol) to prepare a solution. N,N-Diisopropylethylamine (0.087 ml, 0.50 mmol) and BOP reagent (90 mg, 0.20 mmol) were added to the solution, and the mixture was stirred at room temperature for 10 min. t-Butyl (2S)-N-benzenesulfonyl-2,3-diaminopropionate (60 mg, 0.20 mmol) was then added thereto, and the mixture was stirred at room temperature for 14 hr. Water was added to the reaction solution, and the mixture was extracted three times with ethyl acetate. The organic layers were combined, and the combined organic layers were dried over anhydrous magnesium sulfate and were then concentrated under the reduced pressure. The residue was purified by column chromatography on silica gel (development system: chloroform : methanol = 9 : 1) to give the title compound (93 mg, 95%).

Physicochemical properties of compound prepared in Example 23

(1) Color and form: Colorless solid
(2) Molecular formula: $C_{29}H_{36}MeO_5S$
(3) Mass spectrum (TSPMS): m/z 581 $(M+H)^+$
(4) Specific rotation: $[\alpha]_D^{25}$ +75° (c 0.26, $CHCl_3$)
(5) $^1$H NMR spectrum (400 MHz, $CDCl_3$) δ (ppm) : 1.28 (9H, s, t-Bu), 1.66 (1H, m, piperidine), 1.75 (1H, m, piperidine), 1.86 (1H, m, piperidine), 1.99 (1H, m, piperidine), 3.02 (1H, dd, piperidine), 3.16 (1H, ddd, piperidine), 3.48 (1H, m, piperidine), 3.58 (1H, ddd, CONH$\underline{CH_2}$CH), 3.81 (1H, dd, piperidine), 3.89 (2H, m, CONH$\underline{CH_2CH}$), 4.15 (1H, m, piperidine), 5.30 (1H, d, NH), 5.78 (1H, d, NH), 6.52 (1H, m, NH), 6.55 (1H, t, pyrimidine), 6.93 (2H, d, $C_6H_4$), 7.47 (2H, m, $C_6H_5$), 7.55 (1H, m, $C_6H_5$), 7.66 (2H, d, $C_6H_4$), 7.84 (2H, m, $C_6H_5$), 8.29 (2H, d, pyrimidine)

Example 24: (2S)-Benzenesulfonylamino-3-[4-{(3S)-(pyrimidin-2-ylamino)piperidin-1-yl}benzoylamino]-propionic acid

Methylene chloride (1.3 ml) was added to the compound (72 mg, 0.13 mmol) prepared in Example 23 to prepare a solution. Trifluoroacetic acid (1.3 ml) was added to the solution, and the mixture was stirred at room temperature for 5 hr. The reaction solution was concentrated under the reduced pressure to give a trifluoroacetate of the title compound.

Physicochemical properties of compound prepared in Example 24 (as trifluoroacetate)

(1) Color and form: Colorless solid
(2) Molecular formula: $C_{25}H_{28}N_6O_5S$

Example 25: (2S)-Benzenesulfonylamino-3-[4-{(3S)-(1,4,5,6-tetrahydropyrimidin-2-ylamino)piperidin-1-yl}benzoylamino]propionic acid

Dioxane (2.0 ml) and 0.2 ml of water were added to the trifluoroacetate of the crude compound (0.072 mmol) prepared in Example 24 to prepare a solution. To the solution was added 13 mg of 10% palladium-carbon, and the mixture was vigorously stirred under a hydrogen pressure of 1 atm at room temperature for 3 hr. The reaction solution was filtered through Celite, followed by washing with dioxane and water. The filtrate and the washings were combined, and the combined solution was concentrated under the reduced pressure. The residue was purified by preparative thin-layer chromatography on silica gel (development system: methylene chloride : ethanol : water : concentrated aqueous ammonia = 8 : 8 : 1 : 1) and was then purified by Sephadex LH-20 (development system: methanol) to give the title compound (26 mg, 68%).

Physicochemical properties of compound prepared in Example 25

(1) Color and form: Colorless solid
(2) Molecular formula: $C_{25}H_{32}N_6O_5S$
(3) Mass spectrum (TSPMS): m/z 529 $(M+H)^+$
(4) Specific rotation: $[\alpha]_D^{28}+54°$ (c 0.23, MeOH)
(5) $^1H$ NMR spectrum (400 MHz, $CD_3OD$) δ (ppm): 1.62 (1H, m, piperidine), 1.75 (1H, m, piperidine), 1.87 (1H, m, piperidine), 1.98 (3H, m, piperidine and tetrahydropyrimidine), 3.12 (1H, dd, piperidine), 3.18 (1H, m, piperidine), 3.33 (1H, m, piperidine), 3.36 (4H, t, tetrahydropyrimidine), 3.54 (2H, m, CONH$\underline{CH_2}$CH and piperidine), 3.66 (2H, m, CONH$\underline{CH_2}$CH and piperidine), 3.87 (1H, dd, CONHCH$_2\underline{CH}$), 6.97 (2H, d, $C_6H_4$), 7.46 (2H, m, $C_6H_5$), 7.53 (1H, m, $C_6H_5$), 7.69 (2H, d, $C_6H_4$), 7.84 (2H, m, $C_6H_5$)

Intermediate 23: Ethyl 4-{(3S)-aminopiperidin-1-yl}benzoate

N-Methylpyrrolidone (2.0 ml) was added to intermediate 8 (200 mg, 1.16 mmol) to prepare a solution. Sodium hydrogencarbonate (487 mg, 5.80 mmol) and ethyl 4-fluorobenzoate (85 µl, 0.580 mmol) were added to the solution. The mixture was stirred at 120°C for 23 hr. Water (50 ml) and 50 ml of saturated brine were then added to the reaction solution, and the mixture was extracted three times with ethyl acetate. The organic layers were combined, and the combined organic layers were washed with a mixed solution composed of 50 ml of saturated brine and 50 ml of water, were dried over anhydrous sodium sulfate, and were then concentrated under the reduced pressure. The residue was purified by column chromatography on silica gel (development system: methylene chloride : methanol = 7 : 1) to give the title compound (61.2 mg, 42%).

Physicochemical properties of intermediate 23

(1) Color and form: Colorless solid
(2) Molecular formula: $C_{14}H_{20}N_2O_2$
(3) Mass spectrum (TSPMS): m/z 249 $(M+H)^+$

(4) Specific rotation: $[\alpha]_D^{22}$ +49° (c 0.71, MeOH)
(5) $^1$H NMR spectrum (400 MHz, $CDCl_3$) δ (ppm): 1.33 (1H, m, piperidine), 1.36 (3H, t, $CH_2\underline{CH_3}$), 1.63 (1H, m, piperidine), 1.82 (1H, m, piperidine), 1.98 (1H, m, piperidine), 2.74 (1H, dd, piperidine), 2.95 (2H, m, piperidine), 3.58 (1H, dt, piperidine), 3.71 (1H, m, piperidine), 4.32 (2H, q, $\underline{CH_2}CH_3$), 6.86 (2H, d, $C_6H_4$), 7.89 (2H, d, $C_6H_4$)

Intermediate 24: Ethyl 4-{(3S)-(pyridin-2-ylamino)piperidin-1-yl}benzoate

Under an argon atmosphere, 0.62 ml of toluene was added to a mixture of intermediate 23 (20.0 mg, 0.0805 mmmol), 2-chloropyridine (7.0 µl, 0.0725 mmol), palladium acetate (1.8 mg, 0.00805 mmol), (R)-(+)-2,2'-bis(diphenylphosphino)-1,1'-binaphthyl (BINAP) (5.0 mg, 0.00805 mmol), and sodium t-butoxide (9.3 mg, 0.0966 mmol) to prepare a suspension. Argon was blown into the mixture with ultrasonication for 5 min, and the reaction mixture was stirred at 70°C for 5.5 hr. A saturated aqueous ammonium chloride solution (20 ml) was then added to the reaction solution, and the mixture was extracted three times with ethyl acetate. The organic layers were combined, and the combined organic layers were dried over anhydrous sodium sulfate and were then concentrated under the reduced pressure. The residue was purified by preparative thin-layer chromatography on silica gel (development system: hexane : ethyl acetate = 1 : 2) to give the title compound (11.2 mg, 43%).

Physicochemical properties of intermediate 24

(1) Color and form: Colorless syrup
(2) Molecular formula: $C_{19}H_{23}N_3O_2$
(3) Mass spectrum (EIMS): m/z 325 $(M)^+$
(4) $^1$H NMR spectrum (400 MHz, $CDCl_3$) δ (ppm): 1.36 (3H, t, $CH_2CH_3$), 1.70 (2H, m, piperidine), 1.88 (1H, m, piperidine), 2.01 (1H, dddd, piperidine), 3.05 (1H, dd, piperidine), 3.19 (1H, ddd, piperidine), 3.49 (1H, m, piperidine), 3.81 (1H, dd, piperidine), 3.99 (1H, ddddd, piperidine), 4.32 (2H, q, $CH_2CH_3$), 6.40 (1H, d, pyridine), 6.58 (1H, m, pyridine), 6.90 (2H, br d, $C_6H_4$), 7.41 (1H, ddd, pyridine), 7.91 (2H, br d, $C_6H_4$), 8.11 (1H, dd, pyridine)

Intermediate 25: 4-{(3S)-(Pyridin-2-ylamino)piperidin-1-yl}benzoic acid

Tetrahydrofuran (0.9 ml) and 0.3 ml of methanol were added to intermediate 24 (20.0 mg, 0.0615 mmol) to prepare a solution, and 0.3 ml of a 1.0 mol/liter aqueous sodium hydroxide solution was added to the solution. The mixture was stirred at 40°C for 3 hr and was then concentrated under the reduced pressure. Water (2 ml) was added to the residue to prepare a solution, and the solution was adjusted to pH 4 by the addition of 1.0 mol/liter hydrochloric acid. The precipitated solid was collected by filtration through a glass filter, was washed twice with water, and was then dried to give the title compound (17.3 mg, 95%).

Physicochemical properties of intermediate 25

(1) Color and form: Colorless solid
(2) Molecular formula: $C_{17}H_{19}N_3O_2$
(3) Mass spectrum (TSPMS): m/z 298 $(M+H)^+$

Example 26: t-Butyl (2S)-benzenesulfonylamino-3-[4-{(3S)-(pyridin-2-ylamino)piperidin-1-yl}-benzoylamino]-propionate

Dimethylformamide (1.0 ml) was added to intermediate 25 (17.3 mg, 0.0582 mmol) to prepare a solution, and t-butyl (2S)-N-benzenesulfonyl-2,3-diaminopropionate (19.2 mg, 0.0640 mmol) was added to the solution. Further, diisopropylethylamine (12 µl, 0.0698 mmol) and benzotriazol-1-yloxytri(dimethylamino)phosphonium hexafluorophosphate (BOP) (30.9 mg, 0.0698 mmol) were added thereto, and the mixture was stirred at room temperature for 7.5 hr. Water (15 ml) and 15 ml of a saturated aqueous sodium hydrogencarbonate solution were added to the reaction solution, and the mixture was extracted three times with ethyl acetate. The organic layers were combined, and the combined organic layers were washed with a mixed solution composed of 20 ml of saturated brine and 20 ml of water, were dried over anhydrous sodium sulfate, and were then concentrated under the reduced pressure. The residue was purified by preparative thin-layer chromatography on silica gel (development system: hexane : ethyl acetate = 1 : 2) to

give the title compound (32.0 mg, 95%).

Physicochemical properties of compound prepared in Example 26

(1) Color and form: Colorless amorphous form
(2) Molecular formula: $C_{30}H_{37}N_5O_5S$
(3) Mass spectrum (TSPMS): m/z 580 $(M+H)^+$
(4) Specific rotation: $[\alpha]_D^{24}$ +66° (c 1.0, $CH_2Cl_2$)
(5) $^1H$ NMR spectrum (400 MHz, $CDCl_3$) δ (ppm): 1.28 (9H, s, t-Bu), 1.65 (1H, m, piperidine), 1.74 (1H, m, piperidine), 1.88 (1H, m, piperidine), 1.99 (1H, m, piperidine), 3.01 (1H, dd, piperidine), 3.15 (1H, ddd, piperidine), 3.45 (1H, br ddd, piperidine), 3.61 (1H, ddd, CONHC$\underline{H}_2$CH), 3.76 (1H, dd, piperidine), 3.85 (1H, m, CONHC$\underline{H}_2$CH), 3.93 (1H, m, $CONHCH_2C\underline{H}$), 3.97 (1H, ddddd, piperidine), 6.41 (1H, d, pyridine), 6.59 (1H, ddd, pyridine), 6.91 (2H, br d, $C_6H_4$), 7.42 (1H, ddd, pyridine), 7.48 (2H, m, $C_6H_5$), 7.56 (1H, tt, $C_6H_5$), 7.67 (2H, br d, $C_6H_4$), 7.86 (2H, m, $C_6H_5$), 8.11 (1H, m, pyridine)

Example 27: (2S) -Benzenesulfonylamino-3-[4-{(3S)-(pyridin-2-ylamino)piperidin-1-yl}-benzoylamino]propionic acid

Methylene chloride (0.5 ml) was added to the compound (32.0 mg, 0.0552 mmol) prepared in Example 26 to prepare a solution. Trifluoroacetic acid (0.2 ml) was added to the solution, and the mixture was stirred at room temperature for 6.5 hr. The reaction solution was concentrated under the reduced pressure, and the residue was purified by preparative thin-layer chromatography on silica gel (development system: chloroform : methanol : concentrated aqueous ammonia = 30 : 10 : 1) to give the title compound (28.9 mg, 100%).

Physicochemical properties of compound prepared in Example 27

(1) Color and form: Colorless solid
(2) Molecular formula: $C_{26}H_{29}N_5O_5S$
(3) Mass spectrum (FABMS): m/z 524 $(M+H)^+$
(4) Specific rotation: $[\alpha]_D^{25}$ +39° (c 0.36, MeOH-conc. $NH_4OH$ (10 : 1))
(5) $^1H$ NMR spectrum (400 MHz, $CD_3OD$) δ (ppm): 1.58 (1H, dddd, piperidine), 1.75 (1H, ddddd, piperidine), 1.90 (1H, m, piperidine), 2.05 (1H, dddd, piperidine), 2.91 (1H, dd, piperidine), 3.03 (1H, ddd, piperidine), 3.53 (1H, dd, CONHC$\underline{H}_2$CH), 3.61 (1H, m, piperidine), 3.65 (1H, dd, CONHC$\underline{H}_2$CH), 3.84 (2H, m, $CONHCH_2C\underline{H}$ and piperidine), 3.94 (1H, dddd, piperidine), 6.62 (1H, m, pyridine), 6.67 (1H, d, pyridine), 6.96 (2H, br d, $C_6H_4$), 7.43 (2H, br t, $C_6H_5$), 7.52 (2H, m, pyridine and $C_6H_5$), 7.65 (2H, br d, $C_6H_4$), 7.84 (2H, m, $C_6H_5$), 7.92 (1H, m, pyridine)

Intermediate 26: Ethyl 4-{(3S)-aminopiperidin-1-yl}-2-fluorobenzoate

Intermediate 8 (630 mg, 3.7 mmol) and sodium hydrogencarbonate (1.01 g, 12 mmol) were added to ethyl 2,4-difluorobenzoate (230 mg, 1.2 mmol), and the mixture was stirred in a sealed tube at 110°C for 19 hr. Water (50 ml) was added to the reaction solution, and the mixture was extracted three times with ethyl acetate. The organic layer was extracted three times with 0.5 mol/liter hydrochloric acid, and the aqueous layer was adjusted to pH 8 by the addition of a 1.0 mol/liter aqueous sodium hydroxide solution and was then extracted three times with ethyl acetate. The organic layer was dried over anhydrous magnesium sulfate and was then concentrated under the reduced pressure. The residue was purified by column chromatography on silica gel (development system: chloroform : methanol = 9 : 1) to give the title compound (84 mg, 26%).

Physicochemical properties of intermediate 26

(1) Color and form: Brown liquid
(2) Molecular formula: $C_{14}H_{19}N_2O_2F$
(3) Mass spectrum (TSPMS): m/z 267$(M+H)^+$
(4) Specific rotation: $[\alpha]_D^{26}$ +45° (c 0.28, $CHCl_3$)

(5) $^1$H NMR spectrum (400 MHz, $CDCl_3$) δ (ppm): 1.30 (1H, m, piperidine), 1.36 (3H, t, $CH_2\underline{CH_3}$), 1.61 (1H, m, piperidine), 1.81 (1H, m, piperidine), 1.93 (1H, m, piperidine), 2.71 (1H, dd, piperidine), 2.92 (2H, m, piperidine), 3.60 (1H, dt, piperidine), 3.71 (1H, m, piperidine), 4.32 (2H, q, $CH_2\underline{CH_3}$), 6.49 (1H, dd, $C_6H_3$), 6.61 (1H, dd, $C_6H_3$), 7.78 (1H, dd, $C_6H_3$)

Intermediate 27: Ethyl 2-fluoro-4-{(3S)-(pyrimidin-2-ylamino)piperidin-1-yl}benzoate

N-Methylpyrrolidone (1.0 ml) was added to intermediate 26 (144 mg, 0.54 mmol) to prepare a solution. N,N-Diisopropylethylamine (0.47 ml, 2.7 mmol) and 2-bromopyrimidine (100 mg, 0.65 mmol) were added to the solution, and the mixture was stirred at 110°C for 20 hr. Water (50 ml) was then added to the reaction solution, and the mixture was extracted three times with ethyl acetate. The organic layers were combined, and the combined organic layers were dried over anhydrous magnesium sulfate and were then concentrated under the reduced pressure. The residue was purified by column chromatography on silica gel (development system: hexane : ethyl acetate = 1 : 1) to give the title compound (60 mg, 33%).

Physicochemical properties of intermediate 27

(1) Color and form: Colorless solid
(2) Molecular formula: $C_{18}H_{21}N_4O_2F$
(3) Mass spectrum (EIMS): m/z 344 ($M^+$)
(4) Specific rotation: $[\alpha]_D^{26}$ +35° (c 1.05, $CHCl_3$)
(5) $^1$H NMR spectrum (400 MHz, $CDCl_3$) δ (ppm): 1.36 (3H, t, $CH_2\underline{CH_3}$), 1.63 (1H, m, piperidine), 1.75 (1H, m, piperidine), 1.84 (1H, m, piperidine), 2.04 (1H, m, piperidine), 3.00 (1H, dd, piperidine), 3.15 (1H, ddd, piperidine), 3.57 (1H, dt, piperidine), 3.92 (1H, dd, piperidine), 4.08 (1H, m, piperidine), 4.32 (2H, q, $\underline{CH_2}CH_3$), 5.23 (1H, d, NH), 6.57 (1H, t, pyrimidine), 6.61 (1H, dd, $C_6H_3$), 6.67 (1H, dd, $C_6H_3$), 7.79 (1H, dd, $C_6H_3$), 8.29 (2H, d, pyrimidine)

Intermediate 28: 2-Fluoro-4-{(3S)-(pyrimidin-2-ylamino)piperidin-1-yl}benzoic acid

Tetrahydrofuran (2.7 ml) and 0.9 ml of methanol were added to intermediate 27 (60 mg, 0.17 mmol) to prepare a solution, and 0.9 ml of a 1 mol/liter aqueous sodium hydroxide solution was added to the solution. The mixture was stirred at 40°C for 16 hr and was then concentrated under the reduced pressure. Water (20 ml) was added to the residue to prepare a solution which was then washed twice with ethyl acetate. The aqueous layer was adjusted to pH 4 by the addition of 1 mol/liter hydrochloric acid. The precipitated solid was separated by centrifugation and was then dried to give the title compound (45 mg, 84%).

Physicochemical properties of intermediate 28

(1) Color and form: Colorless solid
(2) Molecular formula: $C_{16}H_{17}N_4O_2F$
(3) Mass spectrum (EIMS): m/z 316 ($M^+$)
(4) Specific rotation: $[\alpha]_D^{26}$ +17° (c 0.26, MeOH)
(5) $^1$H NMR spectrum (400 MHz, $CDCl_3$) δ (ppm): 1.77 (2H, m, piperidine), 1.92 (1H, m, piperidine), 2.09 (1H, m, piperidine), 3.16 (2H, m, piperidine), 3.69 (1H, m, piperidine), 3.92 (1H, m, piperidine), 4.17 (1H, m, piperidine), 6.71 (1H, dd, $C_6H_3$), 6.79 (1H, dd, $C_6H_3$), 6.99 (1H, t, pyrimidine), 7.78 (1H, dd, $C_6H_3$), 8.58 (2H, m, pyrimidine)

Example 28: t-Butyl (2S)-Benzenesulfonylamino-3-[2-fluoro-4-{(3S)-(pyrimidin-2-ylamino)piperidin-1-yl}benzoylamino]propionate

Dimethylformamide (5.0 ml) was added to intermediate 28 (45 mg, 0.14 mmol) to prepare a solution. N,N-Diisopropylethylamine (0.073 ml, 0.42 mmol) and BOP reagent (76 mg, 0.17 mmol) were added to the solution, and the mixture was stirred at room temperature for 10 min. t-Butyl (2S)-N-benzenesulfonyl-2,3-diaminopropionate (50 mg, 0.17 mmol) was then added thereto, and the mixture was stirred at room temperature for 14 hr. Water was added to the reaction solution, and the mixture was extracted three times with ethyl acetate. The organic layers were combined, and the combined organic layers were dried over anhydrous magnesium sulfate and were then concentrated under

the reduced pressure. The residue was purified by column chromatography on silica gel (development system: chloroform : methanol = 8 : 2) to give the title compound (43 mg, 51%).

Physicochemical properties of compound prepared in Example 28

(1) Color and form: Colorless solid
(2) Molecular formula: $C_{29}H_{35}N_6O_5FS$
(3) Mass spectrum (TSPMS): m/z 599 $(M+H)^+$
(4) Specific rotation: $[\alpha]_D^{25}+40°$ (c 0.26, $CHCl_3$)
(5) $^1H$ NMR spectrum (400 MHz, $CDCl_3$) δ (ppm): 1.29 (9H, s, t-Bu), 1.65 (1H, m, piperidine), 1.73 (1H, m, piperidine), 1.84 (1H, m, piperidine), 2.05 (1H, m, piperidine), 2.98 (1H, dd, piperidine), 3.14 (1H, ddd, piperidine), 3.56 (1H, m, piperidine), 3.74 (2H, ddd, CONH$\underline{CH_2}$CH), 3.90 (1H, dd, piperidine), 4.00 (1H, m, $CONHCH_2\underline{CH}$), 4.07 (1H, m, piperidine), 5.20 (1H, d, NH), 5.82 (1H, d, NH), 6.57 (1H, t, pyrimidine), 6.58 (1H, d, $C_6H_3$), 6.73 (1H, dd, $C_6H_3$), 6.87 (1H, m, NH), 7.44 (2H, t, $C_6H_5$), 7.51 (1H, d, $C_6H_5$), 7.83 (2H, d, $C_6H_5$), 7.87 (1H, t, $C_6H_3$), 8.30 (2H, d, pyrimidine)

Example 29: (2S)-Benzenesulfonylamino-3-[2-fluoro-4-{(3S)-(pyrimidin-2-ylamino)piperidin-1-yl}benzoylamino]-propionic acid

Methylene chloride (1.3 ml) was added to the compound (72 mg, 0.13 mmol) prepared in Example 28 to prepare a solution. Trifluoroacetic acid (1.3 ml) was added to the solution, and the mixture was stirred at room temperature for 5 hr. The reaction solution was concentrated under the reduced pressure to give a trifluoroacetate of the title compound.

Physicochemical properties of compound prepared in Example 29 (as trifluoroacetate)

(1) Color and form: Colorless solid
(2) Molecular formula: $C_{25}H_{27}N_6O_5FS$
(3) Mass spectrum (TSPMS): m/z 543 $(M+H)^+$
(4) $^1H$ NMR spectrum (400 MHz, $CD_3OD$) δ (ppm): 1.77 (2H, m, piperidine), 1.92 (1H, m, piperidine), 2.10 (1H, m, piperidine), 3.15 (2H, m, piperidine), 3.44 (1H, dd, piperidine), 3.69 (1H, m, piperidine), 3.77 (1H, dd, CONH$\underline{CH_2}$CH), 3.92 (1H, m, piperidine), 4.16 (2H, m, CONH$\underline{CH_2CH}$), 6.71 (1H, dd, $C_6H_3$), 6.83 (1H, dd, $C_6H_3$), 6.93 (1H, t, pyrimidine), 7.41 (2H, d, $C_6H_5$), 7.45 (1H, m, $C_6H_5$), 7.66 (1H, dd, $C_6H_3$), 7.80 (2H, m, $C_6H_5$), 8.55 (2H, br s, pyrimidine)

Example 30: (2S)-Benzenesulfonylamino-3-[2-fluoro-4-{(3S)-(1,4,5,6-tetrahydropyrimidin-2-ylamino)piperidin-1-yl} benzoylamino]propionic acid

Dioxane (2.0 ml) and 0.2 ml of water were added to the trifluoroacetate of the crude compound (0.056 mmol) prepared in Example 29 to prepare a solution. To the solution was added 20 mg of 10% palladium-carbon. The mixture was vigorously stirred under a hydrogen pressure of 1 atm at room temperature for 16 hr. The reaction solution was filtered through Celite, followed by washing with dioxane and water. The filtrate and the washings were combined, and the combined solution was concentrated under the reduced pressure. The residue was purified by preparative thin-layer chromatography on silica gel (development system: methylene chloride : ethanol : water : concentrated aqueous ammonia = 8 : 8 : 1 : 1) and was then purified by Sephadex LH-20 (development system: methanol) to give the title compound (6.0 mg, 20%).

Physicochemical properties of compound prepared in Example 30

(1) Color and form: Colorless solid
(2) Molecular formula: $C_{25}H_{31}N_6O_5FS$
(3) Mass spectrum (TSPMS): m/z 547 $(M+H)^+$

(4) Specific rotation: $[\alpha]_D^{28}$ +35° (c 0.17, MeOH)
(5) $^1$H NMR spectrum (400 MHz, $CD_3OD$) δ (ppm): 1.63 (1H, m, piperidine), 1.70 (1H, m, piperidine), 1.86 (1H, m, piperidine), 1.97 (3H, m, piperidine and tetrahydropyrimidine), 3.12 (1H, m, piperidine), 3.20 (1H, m, piperidine), 3.36 (4H, t, tetrahydropyrimidine), 3.42 (1H, m, piperidine), 3.65 (4H, m, CONH$\underline{CH_2}$CH and piperidine), 3.85 (1H, m, CONHCH$_2\underline{CH}$), 6.67 (1H, d, $C_6H_3$), 6.80 (1H, d, $C_6H_3$), 7.46 (2H, m, $C_6H_5$), 7.50 (1H, m, $C_6H_5$), 7.72 (1H, dd, $C_6H_3$), 7.83 (2H, m, $C_6H_5$)

Intermediate 29: Ethyl 4-{(3S)-aminopiperidin-1-yl}-2-chlorobenzoate

Intermediate 8 (346 mg, 2.0 mmol) and sodium hydrogencarbonate (530 mg, 6.4 mmol) were added to ethyl 2-chloro-4-fluorobenzoate (200 mg, 1.0 mmol), and the mixture was stirred in a sealed tube at 110°C overnight. Water (50 ml) was added to the reaction solution, and the mixture was extracted three times with ethyl acetate. The organic layer was extracted three times with 0.5 mol/liter hydrochloric acid. The aqueous layer was neutralized and adjusted to pH 8 by the addition of a 1.0 mol/lilter aqueous sodium hydroxide solution and was then extracted three times with ethyl acetate. The organic layer was dried over anhydrous magnesium sulfate and was then concentrated under the reduced pressure to give the title compound (113 mg, 40%).

Physicochemical properties of intermediate 29

(1) Color and form: Brown liquid
(2) Molecular formula: $C_{14}H_{19}N_2O_2Cl$
(3) Mass spectrum (EIMS): m/z 282 ($M^+$)
(4) Specific rotation: $[\alpha]_D^{26}$+47° (c 0.50, $CHCl_3$)
(5) $^1$H NMR spectrum (400 MHz, $CDCl_3$) δ (ppm): 1.29 (1H, m, piperidine), 1.37 (3H, t, CH$_2\underline{CH_3}$), 1.63 (1H, m, piperidine), 1.81 (1H, m, piperidine), 1.99 (1H, m, piperidine), 2.69 (1H, dd, piperidine), 2.92 (2H, m, piperidine), 3.60 (1H, m, piperidine), 3.70 (1H, m, piperidine), 4.33 (2H, q, $\underline{CH_2}$CH$_3$), 6.72 (1H, dd, $C_6H_3$), 6.86 (1H, d, $C_6H_3$), 7.80 (1H, d, $C_6H_3$)

Intermediate 30: Ethyl 2-chloro-4-{(3S)-(pyrimidin-2-ylamino)piperidin-1-yl}benzoate

N-Methylpyrrolidone (0.35 ml) was added to intermediate 29 (100 mg, 0.35 mmol) to prepare a solution. N, N-Diisopropylethylamine (0.30 ml, 1.7 mmol) and 2-bromopyrimidine (68 mg, 0.43 mmol) were added to the solution, and the mixture was stirred at 110°C for 20 hr. Water (50 ml) was then added to the reaction solution, and the mixture was extracted three times with ethyl acetate. The organic layers were combined, and the combined organic layers were dried over anhydrous magnesium sulfate and were then concentrated under the reduced pressure. The residue was purified by preparative thin-layer chromatography on silica gel (development system: hexane : ethyl acetate = 1 : 1) to give the title compound (58 mg, 46%).

Physicochemical properties of intermediate 30

(1) Color and form: Colorless solid
(2) Molecular formula: $C_{18}H_{21}N_4O_2Cl$
(3) Mass spectrum (TSPMS): m/z 361 $(M+H)^+$
(4) $^1$H NMR spectrum (400 MHz, $CDCl_3$) δ (ppm): 1.37 (3H, t, CH$_2\underline{CH_3}$), 1.69 (2H, m, piperidine), 1.85 (1H, m, piperidine), 2.05 (1H, m, piperidine), 2.98 (1H, dd, piperidine), 3.15 (1H, ddd, piperidine), 3.56 (1H, m, piperidine), 3.92 (1H, m, piperidine), 4.07 (1H, m, piperidine), 4.33 (2H, q, $\underline{CH_2}$CH$_3$), 5.11 (1H, d, NH), 6.56 (1H, t, pyrimidine), 6.78 (1H, dd, $C_6H_3$), 7.01 (1H, d, $C_6H_3$), 7.81 (1H, d, $C_6H_3$), 8.30 (2H, d, pyrimidine)

Intermediate 31: 2-Chloro-4-{(3S)-(pyrimidin-2-ylamino)piperidin-1-yl}benzoic acid

Tetrahydrofuran (1.7 ml) and 0.6 ml of methanol were added to intermediate 30 (41 mg, 0.11 mmol) to prepare a solution, and 0.6 ml of a 1 mol/liter aqueous sodium hydroxide solution was added to the solution. The mixture was stirred at 40°C for 16 hr and was then concentrated under the reduced pressure. Water (20 ml) was added to the residue to prepare a solution which was then washed twice with ethyl acetate. The aqueous layer was adjusted to pH

4 by the addition of 1 mol/lilter hydrochloric acid and was then extracted three times with ethyl acetate. The organic layer was dried over anhydrous magnesium sulfate and was then concentrated under the reduced pressure to give the title compound (32 mg, 88%).

Physicochemical properties of intermediate 31

(1) Color and form: Colorless solid
(2) Molecular formula: $C_{16}H_{17}N_4O_2Cl$
(3) Mass spectrum (EIMS): m/z 332 ($M^+$)

Example 31: t-Butyl (2S)-benzenesulfonylamino-3-[2-chloro-4-{(3S)-(pyrimidin-2-ylamino)piperidin-1-yl}-benzoylamino]propionate

Dimethylformamide (5.0 ml) was added to intermediate 31 (30 mg, 0.09 mmol) to prepare a solution. N,N-Diisopropylethylamine (0.047 ml, 0.27 mmol) and BOP reagent (47 mg, 0.11 mmol) were added to the solution, and the mixture was stirred at room temperature for 10 min. t-Butyl (2S)-N-benzenesulfonyl-2,3-diaminopropionate (32 mg, 0.11 mmol) was then added thereto, and the mixture was stirred at room temperature for 14 hr. Water was added to the reaction solution, and the mixture was extracted three times with ethyl acetate. The organic layers were combined, and the combined organic layers were dried over anhydrous magnesium sulfate and were then concentrated under the reduced pressure. The residue was purified by preparative thin-layer chromatography on silica gel (development system: chloroform : methanol = 8 : 2) to give the title compound (39 mg, 70%).

Physicochemical properties of compound prepared in Example 31

(1) Color and form: Colorless solid
(2) Molecular formula: $C_{29}H_{35}N_6O_5ClS$
(3) Mass spectrum (EIMS): m/z 615 ($M^+$)
(4) Specific rotation: $[\alpha]_D^{25}+68°$ (c 0.05, $CHCl_3$)
(5) $^1H$ NMR spectrum (400 MHz, $CDCl_3$) δ (ppm): 1.29 (9H, s, t-Bu), 1.65 (2H, m, piperidine), 1.85 (1H, m, piperidine), 2.06 (1H, m, piperidine), 2.96 (1H, dd, piperidine), 3.14 (1H, ddd, piperidine), 3.54 (1H, m, piperidine), 3.80 (3H, m, $CONH\underline{CH_2}CH$ and piperidine), 3.98 (1H, m, $CONHCH_2\underline{CH}$), 4.08 (1H, m, piperidine), 5.19 (1H, d, NH), 5.65 (1H, d, NH) , 6.57 (1H, t, pyrimidine), 6.81 (1H, m, NH),6.83 (1H, dd, $C_6H_3$), 6.97 (1H, d, $C_6H_3$), 7.50 (3H, m, $C_6H_5$), 7.69 (1H, d, $C_6H_3$), 7.84 (2H, d, $C_6H_5$), 8.30 (2H, d, pyrimidine)

Example 32: (2S)-Benzenesulfonylamino-3-[2-chloro-4-{(3S)-(pyrimidin-2-ylamino)piperidin-1-yl}benzoylamino]-propionic acid

Methylene chloride (2.0 ml) was added to the compound (30 mg, 0.049 mmol) prepared in Example 31 to prepare a solution. Trifluoroacetic acid (2.0 ml) was added to the solution, and the mixture was stirred at room temperature for 5 hr. The reaction solution was then concentrated under the reduced pressure to give a trifluoroacetate of the title compound.

Physicochemical properties of compound prepared in Example 32 (as trifluoroacetate)

(1) Color and form: Colorless solid
(2) Molecular formula: $C_{25}H_{27}N_6O_5ClS$
(3) Mass spectrum (EIMS): m/z 558 ($M^+$)
(4) $^1H$ NMR spectrum (400 MHz, $CD_3OD$) δ (ppm): 1.76 (2H, m, piperidine), 1.92 (1H, m, piperidine), 2.07 (1H, m, piperidine), 3.13 (2H, m, piperidine), 3.46 (1H, dd, $CONH\underline{CH_2}CH$), 3.60 (1H, m, piperidine), 3.72 (1H, dd, $CONH\underline{CH_2}CH$), 3.80 (1H, m, piperidine), 4.18 (1H, m, $CONHCH_2\underline{CH}$), 6.93 (1H, dd, $C_6H_3$), 6.97 (1H, t, pyrimidine), 7.04 (1H, d, $C_6H_3$), 7.44 (1H, d, $C_6H_3$), 7.52 (3H, m, $C_6H_5$), 7.84 (2H, m, $C_6H_5$), 8.58 (2H, m, pyrimidine)

Example 33: (2S)-Benzenesulfonylamino-3-[2-chloro-4-{(3S)-(1,4,5,6-tetrahydropyrimidin-2-ylamino)piperidin-1-yl}benzoylamino]propionic acid

Acetic acid (10 ml) and 1.5 ml of concentrated hydrochloric acid were added to the trifluoroacetate of the crude compound (0.030 mmol) prepared in Example 32 to prepare a solution. To the solution was added 15 mg of 10% palladium-carbon. The mixture was vigorously stirred under a hydrogen pressure of 1 atm at room temperature for 16 hr. The reaction solution was filtered through Celite and was then washed with dioxane and water. The filtrate and the washings were combined, and the combined solution was then concentrated under the reduced pressure. The residue was purified by preparative thin-layer chromatography on silica gel (development system: methylene chloride : ethanol : water : concentrated aqueous ammonia = 8 : 8 : 1 : 1) and was then purified by Sephadex LH-20 (development system: methanol) to give the title compound (8.0 mg, 47%).

Physicochemical properties of compound prepared in Example 33

(1) Color and form: Colorless solid
(2) Molecular formula: $C_{25}H_{31}N_6O_5ClS$
(3) Mass spectrum (FABMS): m/z 563 $(M+H)^+$
(4) Specific rotation: $[\alpha]_D^{26}+12°$ (c 0.086, MeOH)
(5) $^1H$ NMR spectrum (400 MHz, $CD_3OD$) δ (ppm): 1.64 (1H, m, piperidine), 1.75 (1H, m, piperidine), 1.88 (2H, m, piperidine), 1.97 (2H, m, tetrahydropyrimidine), 3.13 (3H, m, piperidine), 3.18 (1H, m, piperidine), 3.36 (4H, t, tetrahydropyrimidine), 3.50 (2H, m, $CONH\underline{CH_2}CH$), 3.75 (2H, m, $CONHCH_2\underline{CH}$ and piperidine), 6.90 (1H, dd, $C_6H_3$), 6.96 (1H, d, $C_6H_3$), 7.51 (4H, m, $C_6H_5$ and $C_6H_3$), 7.85 (2H, d, $C_6H_5$)

Intermediate 32: Ethyl 4-{(4R)-azido-(5R)-hydroxy-(3S) methoxyhexylamino}benzoate

Acetonitrile (200 ml) and 50 ml of ethanol were added to oleandomycin phosphate (25.4 g, 32.4 mmol) to prepare a solution. p-Toluenesulfonic acid (12.3 g, 64.9 mmol) was added to the solution, and the mixture was stirred at room temperature for 3 hr. A saturated aqueous sodium carbonate solution was added to the reaction solution, and the mixture was extracted three times with chloroform. The extract was dried over anhydrous sodium sulfate and was then concentrated under the reduced pressure. The residue was purified by column chromatography on silica gel (development system: ethyl acetate) to give ethyloleandroside (3.75 g, 61%).

Methylene chloride (180 ml) was added to ethyloleandroside (3.75 g, 18.4 mmol) to prepare a solution which was then ice cooled. Triethylamine (4.00 ml, 28.9 mmol) and methanesulfonyl chloride (1.85 ml, 23.9 mmol) were added to the solution, and the mixture was stirred at 0°C for 2 hr. Ice was added to the reaction solution, and the mixture was extracted once with chloroform. The organic layer was washed with water, was dried over anhydrous magnesium sulfate, and was then concentrated under the reduced pressure to give a methanesulfonyl compound (5.91 g, 100%).

Dimethylformamide (100 ml) was added to the crude methanesulfonyl compound (18.4 mmol) to prepare a solution. Sodium azide (1.41 g, 21.7 mmol) was added to the solution, and the mixture was stirred at 80°C for 18 hr. Water was added to the reaction solution, and the mixture was extracted once with ethyl acetate. The organic layer was dried over anhydrous magnesium sulfate and was then concentrated under the reduced pressure. The residue was purified by column chromatography on silica gel (development system: hexane : ethyl acetate = 2 : 1) to give an azide compound (5.22 g, 99%).

Dioxane (50 ml) was added to the azide compound (2.07 g, 9.62 mmol) to prepare a solution, and 50 ml of 1.0 mol/liter hydrochloric acid was added to the solution. The mixture was stirred at 60°C for 3 hr and was then ice cooled. The mixture was adjusted to pH 8 by the addition of a 5.0 mol/liter aqueous sodium hydroxide solution and was then extracted three times with chloroform. The organic layers were combined, and the combined organic layers were dried over anhydrous sodium sulfate and were then concentrated under the reduced pressure to give a hemiacetal compound (1.55 g, 86%).

Methylene chloride (35 ml) and 35 ml of methanol were added to the crude hemiacetal compound (1.35 g, 7.21 mmol) to prepare a solution. Ethyl 4-aminobenzoate (899 mg, 5.44 mmol), acetic acid (1.50 ml, 26.2 mmol), and sodium borocyanide (988 mg, 15.7 mmol) were added to the solution, and the mixture was stirred at room temperature for 24 hr. Ethyl 4-aminobenzoate (232 mg, 1.41 mmol), acetic acid (1.25 ml, 21.8 mmol), and sodium borocyanide (848 mg, 13.5 mmol) were added again, and the mixture was stirred at room temperature for 18 hr. Water was added to the reaction solution, and the mixture was extracted twice with chloroform. The extract was dried over anhydrous magnesium sulfate and was then concentrated under the reduced pressure. The residue was purified by column chromatography on silica gel (development system: hexane : ethyl acetate : concentrated aqueous ammonia = 5 : 5 : 0.3) to give

the title compound (1.58 g, 65%).

Physicochemical properties of intermediate 32

(1) Color and form: Light yellow syrup
(2) Molecular formula: $C_{16}H_{24}N_4O_4$
(3) Mass spectrum (TSPMS): m/z 337 $(M+H)^+$
(4) Specific rotation: $[\alpha]_D^{27}$ +0.40° (c 1.3, $CHCl_3$)
(5) $^1H$ NMR spectrum (400 MHz, $CDCL_3$) δ (ppm): 1.27 (3H, d, H-6'), 1.36 (3H, t, $CH_2CH_3$), 1.93 (2H, m, H-2'), 3.35 (2H, m, H-1' and H-4'), 3.48 (3H, s, $OCH_3$), 3.55 (1H, dt, H-3'), 3.94 (1H, br, H-5'), 4.32 (2H, q, $CH_2CH_3$), 6.56 (2H, m, $C_6H_4$), 7.88 (2H, m, $C_6H_4$)

Intermediate 33: Ethyl 4-{(4R)-azido-(5R)-methanesulfonyloxy-(3S)-methoxyhexylamino}benzoate

Methylene chloride (50 ml) was added to intermediate 32 (1.63 g, 4.83 mmol) to prepare a solution which was then ice cooled. Triethylamine (2.00 ml, 14.4 mmol) and methanesulfonyl chloride (0.560 ml, 7.23 mmol) were added to the cooled solution. The temperature of the mixture was raised to room temperature, and the mixture was stirred for 2 hr. Ice was added to the reaction solution, and the mixture was extracted once with chloroform. The organic layer was washed with water, was dried over anhydrous magnesium sulfate, and was then concentrated under the reduced pressure. The residue was purified by column chromatography on silica gel (development system: hexane : ethyl acetate : concentrated aqueous ammonia = 5 : 5 : 0.3) to give the title compound (1.68 g, 84%).

Physicochemical properties of intermediate 33

(1) Color and form: Colorless syrup
(2) Molecular formula: $C_{17}H_{26}N_4O_6S$
(3) Mass spectrum (FABMS): m/z 415 $(M+H)^+$
(4) Specific rotation: $[\alpha]_D^{27}$ -16° (c 1.2, $CHCl_3$)
(5) $^1H$ NMR spectrum (400 MHz, $CDCl_3$) δ (ppm): 1.36 (3H, t, $CH_2CH_3$), 1.50 (3H, d, H-6'), 1.97 (2H, m, H-2'), 3.08 (3H, s, $SO_2CH_3$), 3.36 (2H, t, H-1'), 3.42 (1H, dd, H-4'), 3.46 (3H, s, $OCH_3$), 3.55 (1H, dt, H-3'), 4.32 (2H, q, $CH_2CH_3$), 4.97 (1H, dq, H-5'), 6.61 (2H, brd, $C_6H_4$), 7.89 (2H, m, $C_6H_4$)

Intermediate 34: Ethyl 4-{(3R)-azido-(4S)-methoxy-(2R)-methylpiperidin-1-yl}benzoate

Toluene (45 ml) was added to intermediate 33 (1.87 g, 4.51 mmol) to prepare a solution. Diisopropylethylamine (1.60 ml, 9.19 mmol) was added to the solution, and the mixture was stirred under reflux for 18 hr. The reaction solution was concentrated under the reduced pressure, and the residue was purified by column chromatography on silica gel (development system: hexane : ethyl acetate : concentrated aqueous ammonia = 5 : 5 : 0.3) to give the title compound (1.16 g, 81%).

Physicochemical properties of intermediate 34

(1) Color and form: White solid
(2) Molecular formula: $C_{16}H_{22}N_4O_3$
(3) Mass spectrum (FABMS): m/z 319 $(M+H)^+$
(4) Specific rotation: $[\alpha]_D^{28}$ +62° (c 1.2, $CHCl_3$)
(5) $^1H$ NMR spectrum (400 MHz, $CDCl_3$) δ (ppm): 1.14 (3H, d, $CH_3$), 1.36 (3H, t, $CH_2CH_3$), 2.01 (2H, m, piperidine), 3.04 (1H, m, piperidine), 3.46 (3H, s, $OCH_3$), 3.70 (2H, m, piperidine), 3.92 (1H, m, piperidine), 4.32 (1H, m, piperidine), 4.32 (2H, q, $CH_2CH_3$), 6.85 (2H, m, $C_6H_4$), 7.91 (2H, m, $C_6H_4$)

Intermediate 35: Ethyl 4-{(3R)-amino-(4S)-methoxy-(2R)-methylpiperidin-1-yl}benzoate

Ethanol (24 ml) was added to intermediate 34 (858 mg, 2.70 mmol) to prepare a solution. To the solution was added 76.9 mg of 10% palladium-carbon. The mixture was vigorously stirred under a hydrogen pressure of 1 atm at room temperature for 18 hr. The insolubles were collected by filtration and were then washed with ethanol. The filtrate and the washings were combined, and the combined solution was concentrated under the reduced pressure. The residue was purified by column chromatography on silica gel (development system: ethyl acetate : concentrated aqueous ammonia = 10 : 0.3) to give the title compound (846 mg, 100%).

Physicochemical properties of intermediate 35

(1) Color and form: Light yellow syrup
(2) Molecular formula: $C_{16}H_{24}N_2O_3$
(3) Mass spectrum (TSPMS): m/z 293 $(M+H)^+$
(4) Specific rotation: $[\alpha]_D^{26}$ +47° (c 1.1, $CHCl_3$)
(5) $^1H$ NMR spectrum (400 MHz, $CDCl_3$) $\delta$ (ppm): 1.11 (3H, d, $CH_3$), 1.37 (3H, t, $CH_2CH_3$), 1.84 (2H, m, piperidine), 3.04 (1H, ddd, piperidine), 3.20(1H, m, piperidine), 3.40 (3H, s, $OCH_3$), 3.58 (2H, m, piperidine), 4.29 (1H, m, piperidine), 4.32 (2H, q, $CH_2CH_3$), 6.89 (2H, m, $C_6H_4$), 7.91 (2H, m, $C_6H_4$)

Intermediate 36: Ethyl 4-{(4S)-methoxy-(2R)-methyl-(3R)-(pyrimidin-2-ylamino)piperidin-1-yl}benzoate

N-Methylpyrrolidone (32 ml) was added to intermediate 35 (924 mg, 3.16 mmol) to prepare a solution. Diisopropylethylamine (2.80 ml, 16.1 mmol) and bromopyrimidine (508 mg, 3.19 mmol) were added to the solution, and the mixture was stirred at 120°C for 18 hr. Water was added to the reaction solution, and the mixture was extracted twice with ethyl acetate. The organic layers were combined, and the combined organic layers were dried over anhydrous magnesium sulfate and were then concentrated under the reduced pressure. The residue was purified by column chromatography on silica gel (development system: ethyl acetate) to give the title compound (502 mg, 43%).

Physicochemical properties of intermediate 36

(1) Color and form: Yellow syrup
(2) Molecular formula: $C_{20}H_{26}N_4O_3$
(3) Mass spectrum (FABMS): m/z 371 $(M+H)^+$
(4) Specific rotation: $[\alpha]_D^{26}$ -28° (c 1.1, $CHCl_3$)
(5) $^1H$ NMR spectrum (400 MHz, $CDCl_3$) $\delta$ (ppm): 1.19 (3H, d, $CH_3$), 1.35 (3H, t, $CH_2CH_3$), 1.93 (2H, m, piperidine), 3.13 (1H, ddd, piperidine), 3.38 (3H, s, $OCH_3$), 3.66 (1H, m, piperidine), 3.78 (1H, ddd, piperidine), 4.31 (2H, q, $CH_2CH_3$), 4.51 (2H, m, piperidine), 5.64(1H, d, NH), 6.54 (1H, t, pyrimidine), 6.80 (2H, m, $C_6H_4$), 7.86 (2H, m, $C_6H_4$), 8.27 (2H, d, pyrimidine)

Intermediate 37: 4-{(4S)-Methoxy-(2R)-methyl-(3R)-(pyrimidin-2-ylamino)piperidin-1-yl}benzoic acid

Methanol (4.0 ml) and 2.8 ml of water were added to intermediate 36 (491 mg, 1.33 mmol) to prepare a suspension, and 1.35 ml of a 1.0 mol/liter aqueous sodium hydroxide solution was added to the suspension. The mixture was stirred at 50°C for 6 hr and was then ice cooled. The reaction solution was adjusted to pH 4 by the addition of 5.0 mol/liter hydrochloric acid. The precipitated solid was collected by filtration, was washed twice with water, and was then dried to give the title compound (275 mg, 61%).

Physicochemical properties of intermediate 37

(1) Color and form: Yellow solid
(2) Molecular formula: $C_{18}H_{22}N_4O_3$
(3) Mass spectrum (EIMS): m/z 342 $(M)^+$

(4) Specific rotation: $[\alpha]_D^{26}$ +191° (c 1.1, $CHCl_3$)
(5) $^1H$ NMR spectrum (400 MHz, $CDCl_3$) δ (ppm): 1.25 (3H, d, $CH_3$), 2.03 (1H, m, piperidine), 2.14 (1H, dddd, piperidine), 3.18 (1H, ddd, piperidine), 3.42 (3H, s, $OCH_3$), 3.79 (1H, ddd, piperidine), 3.87 (1H, m, piperidine), 4.37 (1H, m, piperidine), 4.72 (1H, m, piperidine), 6.54 (1H, t, pyrimidine), 6.65 (2H, brd, $C_6H_4$), 7.32 (1H, brd, NH), 7.56 (2H, brd, $C_6H_4$), 8.27 (2H, br, pyrimidine)

Example 34: t-Butyl (2S)-benzenesulfonylamino-3-[4-{(4S)-methoxy-(2R)-methyl-(3R)-(pyrimidin-2-ylamino)-piperidin-1-yl}benzoylamino]propionate

Dimethylformamide (4.0 ml) was added to intermediate 37 (150 mg, 438 mmol) to prepare a solution, and t-butyl (2S)-N-benzenesulfonyl-2,3-diaminopropionate (148 mg, 493 mmol) was added to the solution. Further, benzotriazol-1-yloxytri(dimethylamino)phosphonium hexafluorophosphate (BOP) (241 mg, 545 mmol) and diisopropylethylamine (0.0920 ml, 0.528 mmol) were added thereto, and the mixture was stirred at room temperature for 18 hr. Water and a saturated aqueous sodium hydrogencarbonate solution were added to the reaction solution, and the mixture was extracted twice with ethyl acetate. The organic layers were combined, and the combined organic layers were washed with a mixed solution composed of saturated brine and water, were dried over anhydrous sodium sulfate, and were then concentrated under the reduced pressure. The residue was purified by column chromatography on silica gel (development system: methylene chloride : methanol = 7 : 1) to give the title compound (250 mg, 91%).

Physicochemical properties of compound prepared in Example 34

(1) Color and form: Yellow syrup
(2) Molecular formula: $C_{31}H_{40}N_6O_6S$
(3) Mass spectrum (FABMS): m/z 625 $(M+H)^+$
(4) Specific rotation: $[\alpha]_D^{27}$ +46° (c 0.99, $CHCl_3$)
(5) $^1H$ NMR spectrum (400 MHz, $CHCl_3$) δ (ppm): 1.18 (3H, d, $CH_3$), 1.26 (9H, s, t-Bu), 1.89 (1H, m, piperidine), 2.00 (1H, m, piperidine), 3.11 (1H, ddd, piperidine), 3.39 (3H, s, $OCH_3$), 3.58 (2H, m, piperidine), 3.78 (1H, ddd, $CONHC\underline{H}_2CH$), 3.85 (1H, ddd, $CONHC\underline{H}_2CH$), 3.92 (1H, ddd, $CONHCH_2C\underline{H}$), 4.49 (2H, m, piperidine), 5.72 (1H, d, NH), 5.98 (1H, d, NH), 6.54 (1H, t, pyrimidine), 6.58 (1H, dd, NH), 6.81 (2H, m, $C_6H_4$), 7.46 (2H, m, $C_6H_5$), 7.55 (1H, m, $C_6H_5$), 7.62 (2H, m, $C_6H_4$), 7.85 (2H, m, $C_6H_5$), 8.29 (2H, d, pyrimidine)

Example 35: (2S)-Benzenesulfonylamino-3-[4-{(4S)-methoxy-(2R)-methyl-(3R)-(pyrimidin-2-ylamino)piperidin 1-yl}benzoylamino]propionic acid

Methylene chloride (3.0 ml) was added to the compound (103 mg, 0.166 mmol) prepared in Example 34 to prepare a solution. Trifluoroacetic acid (3.0 ml) was added to the solution, and the mixture was stirred at room temperature for 3 hr. The reaction solution was concentrated under the reduced pressure to give a trifluoroacetate of the title compound.

Physicochemical properties of compound prepared in Example 35

(1) Color and form: Colorless liquid
(2) Molecular formula: $C_{27}H_{32}N_6O_6S$
(3) Mass spectrum (TSPMS): m/z 569 $(M+H)^+$
(4) $^1H$ NMR spectrum (400 MHz, $CD_3OD$)(as trifluoroacetate) δ (ppm): 1.21 (3H, d, $CH_3$), 2.03 (1H, m, piperidine), 2.15 (1H, m, piperidine), 3.44 (3H, s, $OCH_3$), 3.49 (1H, m), 3.76 (2H, m), 3.90 (1H, ddd), 4.20 (2H, m), 4.40 (1H, m), 4.73 (1H, m), 6.96 (1H, t, pyrimidine), 7.20 (2H, d, $C_6H_4$), 7.44 (2H, m, $C_6H_5$), 7.51 (1H, m, $C_6H_5$), 7.76 (2H, d, $C_6H_4$), 7.83 (2H, m, $C_6H_5$), 8.57 (2H, br, pyrimidine)

Example 36: (2S)-Benzenesulfonylamino-3-[4-{(4S)-methoxy-(2R)-methyl-(3R)-(1,4,5,6-tetrahydropyrimidin-2-ylamino)piperidin-1-yl}benzoylamino]propionic acid

Dioxane (3.0 ml) and 0.30 ml of water were added to the trifluoroacetate of the crude compound (0.166 mmol) prepared in Example 35 to prepare a solution. To the solution was added 18.1 mg of 10% palladium-carbon. The

mixture was vigorously stirred under a hydrogen pressure of 1 atm at room temperature for 18 hr. The insolubles were collected by filtration and were then washed with ethanol. The filtrate and the washings were combined, and the combined solution was concentrated under the reduced pressure. The residue was purified by column chromatography on silica gel (development system: chloroform : methanol : concentrated aqueous ammonia = 9 : 3 : 0.3) and was then purified by Sephadex LH-20 (development system: methanol) to give the title compound (62.6 mg, 66%).

Physicochemical properties of compound prepared in Example 36

(1) Color and form: White solid
(2) Molecular formula: $C_{27}H_{36}N_6O_6S$
(3) Mass spectrum (TSPMS): m/z 573 $(M+H)^+$
(4) Specific rotation: $[\alpha]_D^{25}$ +136° (c 0.15, MeOH)
(5) $^1H$ NMR spectrum (400 MHz, $CD_3OD$) δ (ppm): 1.15 (3H, d, $CH_3$), 1.70 (1H, ddd, piperidine), 1.94 (2H, quintet, tetrahydropyrimidine), 2.10 (1H, m, piperidine), 3.08 (1H, m, piperidine), 3.36 (4H, t, tetrahydropyrimidine), 3.41 (3H, s, $OCH_3$), 3.47 (1H, m), 3.55 (1H, m), 3.64 (2H, m), 3.81 (1H, m), 3.87 (1H, m), 4.26 (1H, m), 6.95 (2H, d, $C_6H_4$), 7.47 (2H, m, $C_6H_5$), 7.54 (1H, dd, $C_6H_5$), 7.68 (2H, d, $C_6H_4$), 7.85 (2H, m, $C_6H_5$)

Intermediate 38: Ethyl 4-{(3S)-aminopiperidin-1-yl}-2-trifluoromethylbenzoate

Intermediate 8 (1.1 g, 6.4 mmol), sodium hydrogencarbonate (1.6 g, 20 mmol), and 0.35 ml of N-methylpyrrolidone were added to ethyl 4-fluoro-2-trifluoromethylbenzoate (750 mg, 3.2 mmol), and the mixture was stirred in a sealed tube at 110°C overnight. Water (50 ml) was added to the reaction solution, and the mixture was extracted three times with ethyl acetate. The organic layer was extracted three times with 0.5 mol/liter hydrochloric acid. The aqueous layer was neutralized and adjusted to pH 8 by the addition of a 1.0 mol/liter aqueous sodium hydroxide solution and was then extracted three times with ethyl acetate. The organic layer was dried over anhydrous magnesium sulfate and was then concentrated under the reduced pressure to give the title compound (579 mg, 58%).

Physicochemical properties of intermediate 38

(1) Color and form: Brown liquid
(2) Molecular formula: $C_{15}H_{19}N_2O_2F_3$
(3) Mass spectrum (EIMS): m/z 316$(M^+)$
(4) Specific rotation: $[\alpha]_D^{26}$+21° (c 0.50, $CHCl_3$)
(5) $^1H$ NMR spectrum (400 MHz, $CDCl_3$) δ (ppm): 1.32 (1H, m, piperidine), 1.36 (3H, t, $CH_2\underline{CH_3}$), 1.64 (1H, m, piperidine), 1.84 (1H, m, piperidine), 2.02 (1H, m, piperidine), 2.73 (1H, dd, piperidine), 2.95 (2H, m, piperidine), 3.62 (1H, m, piperidine), 3.73 (1H, m, piperidine), 4.33 (2H, q, $\underline{CH_2}CH_3$), 6.93 (1H, dd, $C_6H_3$), 7.13 (1H, d, $C_6H_3$), 7.79 (1H, d, $C_6H_3$)

Intermediate 39: Ethyl 4-{(3S)-(pyrimidin-2-ylamino)piperidin-1-yl}-2-trifluoromethylbenzoate

N-Methylpyrrolidone (1.3 ml) was added to intermediate 38 (200 mg, 0.63 mmol) to prepare a solution. N,N-Diisopropylethylamine (0.54 ml, 3.2 mmol) and 2-bromopyrimidine (120 mg, 0.76 mmol) were added to the solution, and the mixture was stirred at 110°C for 20 hr. Water (50 ml) was then added to the reaction solution, and the mixture was extracted three times with ethyl acetate. The organic layers were combined, and the combined organic layers were dried over anhydrous magnesium sulfate and were then concentrated under the reduced pressure. The residue was purified by column chromatography on silica gel (development system: hexane : ethyl acetate = 1 : 1) to give the title compound (140 mg, 54%).

Physicochemical properties of intermediate 39

(1) Color and form: Colorless solid
(2) Molecular formula: $C_{19}H_{21}N_4O_2F_3$

(3) Mass spectrum (TSPMS): m/z 395 $(M+H)^+$
(4) $^1$H NMR spectrum (400 MHz, $CDCl_3$) δ (ppm): 1.36 (3H, t, $CH_2\underline{CH_3}$), 1.73 (2H, m, piperidine), 1.88 (1H, m, piperidine), 2.10 (1H, m, piperidine), 3.01 (1H, dd, piperidine), 3.14 (1H, ddd, piperidine), 3.71 (1H, m, piperidine), 4.03 (1H, m, piperidine), 4.33 (2H, q, $\underline{CH_2}CH_3$), 6.66 (1H, t, pyrimidine), 7.00 (1H, dd, $C_6H_3$), 7.32 (1H, d, $C_6H_3$), 7.80 (1H, d, $C_6H_3$), 8.34 (2H, brs, pyrimidine)

Intermediate 40: 4-{(3S)-(Pyrimidin-2-ylamino)piperidin-1-yl}-2-trifluoromethylbenzoic acid

Tetrahydrofuran (6.0 ml) and 2.0 ml of methanol were added to intermediate 39 (120 mg, 0.30 mmol) to prepare a solution, and 2.0 ml of a 1 mol/liter aqueous sodium hydroxide solution was added to the solution. The mixture was stirred at 40°C for 5 hr and was then concentrated under the reduced pressure. Water (20 ml) was added to the residue to prepare a solution which was then washed twice with ethyl acetate. The aqueous layer was adjusted to pH 4 by the addition of 1 mol/liter hydrochloric acid and was then extracted three times with ethyl acetate. The organic layer was dried over anhydrous magnesium sulfate and was then concentrated under the reduced pressure to give the title compound (90 mg, 82%).

Physicochemical properties of intermediate 40

(1) Color and form: Colorless solid
(2) Molecular formula: $C_{17}H_{17}N_4O_2F_3$
(3) $^1$H NMR spectrum (400 MHz, $CDCl_3$) δ (ppm): 1.80 (2H, m, piperidine), 1.94 (1H, m, piperidine), 2.11 (1H, m, piperidine), 3.23 (2H, m, piperidine), 3.72 (1H, m, piperidine), 3.94 (1H, m, piperidine), 4.20 (1H, m, piperidine), 7.02 (1H, t, pyrimidine), 7.18 (1H, dd, $C_6H_3$), 7.33 (1H, d, $C_6H_3$), 7.85 (1H, d, $C_6H_3$), 8.50 (2H, br s, pyrimidine)

Example 37: t-Butyl (2S)-benzenesulfonylamino-3-[4-{(3S)-(pyrimidin-2-ylamino)piperidin-1-yl}-2-trifluoromethylbenzoylamino]propionate

Dimethylformamide (5.0 ml) was added to intermediate 40 (80 mg, 0.22 mmol) to prepare a solution. N,N-Diisopropylethylamine (0.11 ml, 0.66 mmol) and BOP reagent (110 mg, 0.26 mmol) were added to the solution, and the mixture was stirred at room temperature for 10 min. t-Butyl (2S)-N-benzenesulfonyl-2,3-diaminopropionate (77 mg, 0.26 mmol) was then added thereto, and the mixture was stirred at room temperature for 14 hr. Water was added to the reaction solution, and the mixture was extracted three times with ethyl acetate. The organic layers were combined, and the combined organic layers were dried over anhydrous magnesium sulfate and were then concentrated under the reduced pressure. The residue was purified by preparative thin-layer chromatography on silica gel (development system: chloroform : methanol = 8 : 2) to give the title compound (115 mg, 81%).

Physicochemical properties of compound prepared in Example 37

(1) Color and form: Colorless solid
(2) Molecular formula: $C_{30}H_{35}N_6O_5F_3S$
(3) Mass spectrum (TSPMS): m/z 649 $(M+H)^+$
(4) Specific rotation: $[\alpha]_D^{26}+54°$ (c 0.12, $CHCl_3$)
(5) $^1$H NMR spectrum (400 MHz, $CDCl_3$) δ (ppm): 1.24 (9H, s, t-Bu), 1.64 (1H, m, piperidine), 1.68 (1H, m, piperidine), 1.85 (1H, m, piperidine), 2.04 (1H, m, piperidine), 3.00 (1H, m, piperidine), 3.15 (1H, m, piperidine), 3.54 (1H, m, piperidine), 3.60 (1H, m, $CONH\underline{CH_2}CH$), 3.84 (1H, m, piperidine), 3.91 (1H, m, $CONH\underline{CH_2CH}$), 4.09 (1H, m, piperidine), 5.19 (1H, brs, NH), 5.55 (1H, brs, NH),6.21 (1H, brs, NH), 6.57 (1H, t, pyrimidine), 7.04 (1H, dd, $C_6H_3$), 7.29 (1H, d, $C_6H_3$), 7.48 (1H, d, $C_6H_3$), 7.50 (2H, m, $C_6H_5$), 7.56 (1H, m, $C_6H_5$), 7.84 (2H, m, $C_6H_5$), 8.29 (2H, d, pyrimidine)

Example 38: (2S)-Benzenesulfonylamino-3-[4-{(3S)-(pyrimidin-2-ylamino)piperidin-1-yl}-2-trifluoromethylbenzoylamino]propionic acid

Methylene chloride (2.0 ml) was added to the compound (60 mg, 0.093 mmol) prepared in Example 37 to prepare a solution. Trifluoroacetic acid (2.0 ml) was added to the solution, and the mixture was stirred at room temperature for 5 hr. The reaction solution was concentrated under the reduced pressure to give a trifluoroacetate of the title compound.

Physicochemical properties of compound prepared in Example 38 (as trifluoroacetate)

(1) Color and form: Colorless solid
(2) Molecular formula: $C_{26}H_{27}N_6O_5F_3S$
(3) Mass spectrum (TSPMS): m/z 593 $(M+H)^+$

<u>Example 39: (2S)-Benzenesulfonylamino-3-[4-{(3S)-(1,4,5,6-tetrahydropyrimidin-2-ylamino)piperidin-1-yl}-2-trifluoromethylbenzoylamino]propionic acid</u>

Dioxane (4.0 ml) and 0.4 ml of water were added to the trifluoroacetate of the crude compound (0.075 mmol) prepared in Example 38 to prepare a solution. To the solution was added 40 mg of 10% palladium-carbon. The mixture was vigorously stirred under a hydrogen pressure of 1 atm at room temperature for 16 hr. The reaction solution was filtered through Celite, followed by washing with dioxane and water. The filtrate and the washings were combined, and the combined solution was concentrated under the reduced pressure. The residue was purified by preparative thin-layer chromatography on silica gel (development system: methylene chloride : ethanol : water : concentrated aqueous ammonia = 8 : 8 : 1 : 1) and was then purified by Sephadex LH-20 (development system: methanol) to give the title compound (10 mg, 24%).

Physicochemical properties of compound prepared in Example 39

(1) Color and form: Colorless solid
(2) Molecular formula: $C_{26}H_{31}N_6O_5F_3S$
(3) Mass spectrum (TSPMS): m/z 597 $(M+H)^+$
(4) Specific rotation: $[\alpha]_D^{26}$ +50° (c 0.12, MeOH)
(5) $^1H$ NMR spectrum (400 MHz, $CD_3OD$) δ (ppm): 1.63 (1H, m, piperidine), 1.74 (1H, m, piperidine), 1.90 (2H, m, piperidine), 1.96 (2H, m, tetrahydropyrimidine), 3.19 (3H, m, piperidine), 3.29 (1H, m, piperidine), 3.36 (4H, t, tetrahydropyrimidine), 3.58 (3H, m, CONH$\underline{CH_2}$CH and piperidine), 3.72 (1H, dd, $CONHCH_2\underline{CH}$), 7.17 (1H, dd, $C_6H_3$), 7.19 (1H, d, $C_6H_3$), 7.53 (3H, m, $C_6H_5$), 7.60 (1H, d, $C_6H_3$), 7.86 (1H, d, $C_6H_5$)

<u>Intermediate 41: Ethyl 4-{(3S)-(Pyrimidin-2-ylamino)piperidin-1-yl}benzoate</u>

<u>N</u>-Methylpyrrolidone (0.40 ml) was added to intermediate 23 (50 mg, 0.20 mmol) to prepare a solution. <u>N</u>,<u>N</u>-Diisopropylethylamine (0.18 ml, 1.0 mmol) and 2-bromopyrimidine (35 mg, 0.24 mmol) were added to the solution, and the mixture was stirred at 110°C for 20 hr. Water (50 ml) was then added to the reaction solution, and the mixture was extracted three times with ethyl acetate. The organic layers were combined, and the combined organic layers were dried over anhydrous magnesium sulfate and were then concentrated under the reduced pressure. The residue was purified by column chromatography on silica gel (development system: hexane : ethyl acetate = 7 : 3) to give the title compound (38 mg, 58%).

Physicochemical properties of intermediate 41

(1) Color and form: Colorless solid
(2) Molecular formula: $C_{18}H_{22}N_4O_2$
(3) Mass spectrum (TSPMS): m/z 327 $(M+H)^+$
(4) $^1H$ NMR spectrum (400 MHz, $CDCl_3$) δ (ppm): 1.36 (3H, t, $CH_2\underline{CH_3}$), 1.69 (1H, m, piperidine), 1.74 (1H, m, piperidine), 1.85 (1H, m, piperidine), 2.02 (1H, m, piperidine), 3.06 (1H, dd, piperidine), 3.40 (1H, ddd, piperidine), 3.50 (1H, m, piperidine), 3.84 (1H, dd, piperidine), 4.14 (1H, m, piperidine), 4.32 (2H, q, $\underline{CH_2}CH_3$), 5.24 (1H, d, NH), 6.55 (1H, t, pyrimidine), 6.91 (2H, d, $C_6H_4$), 7.89 (2H, d, $C_6H_4$), 8.28 (2H, d, pyrimidine)

Example 40: t-Butyl (2S)-(benzyloxycarbonyl)amino-3-[4-{(3S)-(pyrimidin-2-ylamino)piperidin-1-yl}benzoylamino]-propionate

Dimethylformamide (5.0 ml) was added to intermediate 41 (60 mg, 0.20 mmol) to prepare a solution. N,N-Diisopropylethylamine (0.10 ml, 0.60 mmol) and BOP reagent (100 mg, 0.24 mmol) were added to the solution, and the mixture was stirred at room temperature for 30 min. t-Butyl (2S)-N-benzyloxycarbonyl-2,3-diaminopropionate hydrochloride (70 mg, 0.24 mmol) was then added thereto, and the mixture was stirred at room temperature for 4 hr. Water was added to the reaction solution, and the mixture was extracted three times with ethyl acetate. The organic layers were combined, and the combined organic layers were dried over anhydrous magnesium sulfate and were then concentrated under the reduced pressure. The residue was purified by column chromatography on silica gel (development system: hexane : ethyl acetate = 1 : 3) to give the title compound (140 mg, 100%).

Physicochemical properties of compound prepared in Example 40

(1) Color and form: Colorless solid
(2) Molecular formula: $C_{31}H_{38}N_6O_5$
(3) Mass spectrum (TSPMS): m/z 575 $(M+H)^+$
(4) Specific rotation: $[\alpha]_D^{25}$+10° (c 0.17, $CHCl_3$)
(5) $^1$H NMR spectrum (400 MHz, $CDCl_3$) δ (ppm): 1.46 (9H, s, t-Bu), 1.66 (1H, m, piperidine),1.75 (1H, m, piperidine), 1.86 (1H, m, piperidine), 2.00 (1H, m, piperidine), 3.03 (1H, dd, piperidine), 3.16 (1H, ddd, piperidine), 3.45 (1H, m, piperidine), 3.78 (3H, m, piperidine and CONH$CH_2$CH), 4.15 (1H, m, piperidine), 4.43 (1H, m, CONH$CH_2$CH), 5.10 (2H, s, $CH_2C_6H_5$), 5.27 (1H, d, NH), 5.87 (1H, brs, NH), 6.54 (1H, t, pyrimidine), 6.66 (1H, brs, NH), 6.91 (2H, d, $C_6H_4$), 7.32 (5H, m, $C_6H_5$), 7.63 (2H, d, $C_6H_4$), 8.287 (2H, d, pyrimidine)

Example 41: t-Butyl (2S)-amino-3-[4-{(3S)-(pyrimidin-2-ylamino)piperidin-1-yl}benzoylamino]propionate

Tetrahydrofuran was added to the compound (85 mg, 0.15 mmol) prepared in Example 40 to prepare a solution. To the solution was added 34 mg of 5% palladium-carbon. The mixture was vigorously stirred under a hydrogen pressure of 1 atm at room temperature for 12 hr. The reaction solution was filtered through Celite and was then washed with tetrahydrofuran and ethanol. The filtrate and the washings were combined, and the combined solution was concentrated under the reduced pressure. The residue was purified by column chromatography on silica gel (development system: chloroform : methanol = 8 : 2) to give the title compound (48 mg, 74%).

Physicochemical properties of compound prepared in Example 41

(1) Color and form: Colorless solid
(2) Molecular formula: $C_{23}H_{32}N_6O_3$
(3) Mass spectrum (TSPMS): m/z 441 $(M+H)^+$
(4) Specific rotation: $[\alpha]_D^{25}$+30° (c 0.48, $CHCl_3$)
(5) $^1$H NMR spectrum (400 MHz, $CDCl_3$) δ (ppm): 1.26 (9H, s, t-Bu), 1.76 (2H, m, piperidine), 1.86 (1H, m, piperidine), 2.00 (1H, m, piperidine), 3.03 (1H, dd, piperidine), 3.17 (1H, ddd, piperidine), 3.45 (2H, m, piperidine), 3.60 (1H, m, CONH$CH_2$CH), 3.80 (2H, m, CONH$CH_2$CH), 4.15 (1H, m, piperidine), 5.29 (1H, d, NH), 6.54 (1H, t, pyrimidine), 6.67 (1H, brs, NH), 6.92 (2H, d, $C_6H_4$), 7.66 (2H, d, $C_6H_4$), 8.28 (2H, d, pyrimidine)

Example 42: t-Butyl 3-[4-{(3S)-(pyrimidin-2-ylamino)piperidin-1-yl}benzoylamino]-(2S)-{(thiophene-2-sulfonyl)amino}propionate

Dimethylformamide (3.0 ml) was added to the compound (40.0 mg, 0.0900 mmol) prepared in Example 41 to prepare a solution. N,N-Diisopropylethylamine (0.032 ml, 0.180 mmol) and 2-thiophenesulfonyl chloride (16.0 mg, 0.0900 mmol) were added to the solution, and the mixture was stirred at room temperature for 16 hr. Piperidine was added to the reaction solution, water and a saturated aqueous sodium hydrogencarbonate solution were then added thereto, and the mixture was extracted three times with ethyl acetate. The organic layers were combined, and the combined organic layers were dried over anhydrous magnesium sulfate and were then concentrated under the reduced pressure. The residue was purified by column chromatography on silica gel (development system: chloroform : methanol = 9 : 1) to give the title compound (27.0 mg, 51%).

Physicochemical properties of compound prepared in Example 42

(1) Color and form: Colorless solid
(2) Molecular formula: $C_{27}H_{34}N_6O_5S_2$
(3) Mass spectrum (TSPMS): m/z 587 $(M+H)^+$
(4) $^1H$ NMR spectrum (400 MHz, $CDCl_3$) δ (ppm): 1.35 (9H, s, t-Bu), 1.65 (1H, m, piperidine), 1.75 (1H, m, piperidine), 1.86 (1H, m, piperidine), 2.00 (1H, m, piperidine), 3.00 (1H, dd, piperidine), 3.14 (1H, ddd, piperidine), 3.46 (1H, m, piperidine), 3.67 (1H, ddd, CONH$\underline{CH_2}$CH), 3.80 (1H, m, piperidine), 3.86 (1H, m, CONH$\underline{CH_2}$CH), 4.04 (1H, m, CONH$CH_2$$\underline{CH}$), 4.13 (1H, m, piperidine), 5.35 (1H, bd, NH), 5.98 (1H, bd, NH), 6.49 (1H, m, NH), 6.55 (1H, t, pyrimidine), 6.92 (2H, d, $C_6H_4$), 7.05 (1H, dd, thiophene), 7.57 (1H, dd, thiophene), 7.60 (1H, dd, thiophene), 7.65 (2H, d, $C_6H_4$), 8.29 (2H, d, pyrimidine)

Example 43: 3-[4-{(3S)-(Pyrimidin-2-ylamino)piperidin-1-yl}benzoylamino]-(2S)-{(thiophene-2-sulfonyl)amino}-propionic acid

Methylene chloride (5.0 ml) was added to the compound (100 mg, 0.170 mmol) prepared in Example 42 to prepare a solution. Trifluoroacetic acid (5.0 ml) was added to the solution, and the mixture was stirred at room temperature for 4 hr. The reaction solution was concentrated under the reduced pressure to give the title compound.

Physicochemical properties of compound prepared in Example 43

(1) Color and form: Colorless solid
(2) Molecular formula: $C_{23}H_{26}N_6O_5S_2$
(3) Mass spectrum (FABMS): m/z 531 $(M+H)^+$
(4) $^1H$ NMR spectrum (400 MHz, $CD_3OD$) δ (ppm): 1.77 (2H, m, piperidine), 1.93 (1H, m, piperidine), 2.09 (1H, m, piperidine), 3.14 (2H, m, piperidine), 3.49 (1H, dd, CONH$\underline{CH_2}$CH), 3.62 (1H, m, piperidine), 3.74 (1H, dd, CONH$\underline{CH_2}$CH), 3.85 (1H, m, piperidine), 4.23 (2H, m, piperidine and CONH$CH_2$$\underline{CH}$), 6.95 (1H, t, pyrimidine), 7.01 (3H, m, $C_6H_4$ and thiophene), 7.56 (1H, dd, thiophene), 7.64 (1H, dd, thiophene), 7.68 (2H, d, $C_6H_4$), 8.57 (2H, brs, pyrimidine)

Example 44: 3-[4-{(3S)-(1,4,5,6-Tetrahydropyrimidin-2-ylamino)piperidin-1-yl}benzoylamino]-(2S)-{(thiophene-2-sulfonyl)amino}propionic acid

Tetrahydrofuran (5.0 ml) and 0.5 ml of water were added to the compound prepared in Example 43 to prepare a solution. To the solution was added 100 mg of 10% palladium-carbon. The mixture was vigorously stirred under a hydrogen pressure of 1 atm at room temperature for 15 hr. The reaction solution was filtered through Celite and was then washed with tetrahydrofuran and water. The filtrate and the washings were combined, and the combined solution was concentrated under the reduced pressure. The residue was purified by preparative thin-layer chromatography on silica gel (development system: chloroform : methanol : concentrated aqueous ammonia =8 : 2 : 0.2) and was then purified by Sephadex LH-20 (development system: methanol) to give the title compound.

Physicochemical properties of compound prepared in Example 44

(1) Color and form: Colorless solid
(2) Molecular formula: $C_{23}H_{30}N_6O_5S_2$
(3) Mass spectrum (TSPMS): m/z 535 $(M+H)^+$
(4) $^1H$ NMR spectrum (400 MHz, $CDCl_3$) δ (ppm): 1.58 (1H, m, piperidine), 1.70 (1H, m, piperidine), 1.86 (1H, m, piperidine), 1.94 (3H, m, tetrahydropyrimidine and piperidine), 3.02 (1H, dd, piperidine), 3.08 (1H, m, piperidine), 3.34 (4H, t, tetrahydropyrimidine), 3.40 (1H, m, piperidine), 3.54 (2H, m, CONH$\underline{CH_2}$CH and piperidine), 3.63 (1H, m, piperidine), 3.67 (1H, dd, CONH$\underline{CH_2}$CH), 3.97 (1H, dd, CONH$CH_2$$\underline{CH}$), 6.94 (2H, d, $C_6H_4$), 7.02 (1H, dd, thiophene), 7.57 (1H, dd, thiophene), 7.66 (3H, m, thiophene and $C_6H_4$)

Example 51: t-Butyl (2S)-(pyridine-3-sulfonyl)amino-3-[4-{(3S)-(pyrimidin-2-ylamino)piperidin-1-yl}benzoylamino] propionate

Dimethylformamide (3.0 ml) was added to the compound (105 mg, 0.238 mmol) prepared in Example 41 to prepare a solution. Diisopropylethylamine (100 µl, 0.574 mmol) and 3-pyridinesulfonyl chloride prepared by a method described in a literature (J. Med. Chem., 42, 2403 (1999)) were added in that order to the solution at room temperature, and a reaction was allowed to proceed for 12 hr. Water (100 ml) and 20 ml of a saturated aqueous sodium hydrogencarbonate solution were added to the reaction solution, and the mixture was extracted twice with 100 ml of ethyl acetate. The organic layers were combined, and the combined organic layers were washed twice with 100 ml of water and once with 100 ml of saturated brine, were dried over anhydrous magnesium sulfate, and were then concentrated under the reduced pressure. The residue was purified by chromatography on silica gel (development system: hexane : ethyl acetate = 1 : 8) to give the title compound (28.7 mg, 21%).

Physicochemical properties of compound prepared in Example 51

(1) Color and form: Colorless solid
(2) Molecular formula: $C_{28}H_{35}N_7O_5S$
(3) Mass spectrum (TSPMS): m/z 582 $(M+H)^+$
(4) Specific rotation: $[\alpha]_D^{24}$ +39° (c 1.3, $CHCl_3$)
(5) $^1H$ NMR spectrum (400 MHz, $CDCl_3$) δ (ppm): 1.31 (9H, s, t-Bu), 1.50 - 1.80 (2H, m, piperidine), 1.82 - 1.93 (1H, m, piperidine), 1.97 - 2.06 (1H, m, piperidine), 2.99 (1H, dd, piperidine), 3.14 (1H, ddd, piperidine), 3.45 - 3.53 (1H, m, piperidine), 3.73 (1H, dd, $CONHCH_2$), 3.77- 3.87 (2H, m, piperidine and $CONHCH_2$), 4.04 (1H, dd, $CONHCH_2CH$), 4.07 - 4.18 (1H, m, piperidine), 6.57 (1H, t, pyrimidine), 6.91 (2H, d, $C_6H_4$), 7.40 (1H, dd, pyridine), 7.62 (2H, d, $C_6H_4$), 8.13 (1H, ddd, pyridine), 8.31 (2H, d, pyrimidine), 8.76 (1H, dd, pyridine), 9.06 (1H, d, pyridine)

Example 52: (2S)-(Pyridine-3-sulfonyl)amino-3-[4-{(3S)-(pyrimidin-2-ylamino)piperidin-1-yl}benzoylamino]-propionic acid

Methylene chloride (1.0 ml) was added to the compound (26.0 mg, 0.0447 mmol) prepared in Example 51 to prepare a solution, and 1.0 ml of trifluoroacetic acid was added to the solution at room temperature. A reaction was allowed to proceed for 7 hr, and the reaction solution was then concentrated under the reduced pressure to give the title compound (37.6 mg, 92% (as tritrifluoroacetate)).

Physicochemical properties of compound prepared in Example 52 (as tritrifluoroacetate)

(1) Color and form: Light yellow solid
(2) Molecular formula: $C_{24}H_{27}N_7O_5S$
(3) Mass spectrum (FABMS): m/z 526 $(M+H)^+$

Example 53: (2S)-(Pyridine-3-sulfonyl)amino-3-[4-{(3S)-(1,4,5,6-tetrahydropyrimidin-2-ylamino)piperidin-1-yl} benzoylamino]propionic acid

1,4-Dioxane (2.0 ml) and 1.0 ml of water were added in that order to 32.2 mg of the compound prepared in Example 52 to prepare a solution. To the solution was added 9.0 mg of 10% palladium-carbon. The mixture was stirred under a hydrogen atmosphere at room temperature for 5 hr. The insolubles were collected by filtration and were washed with 90 ml of a solvent having the same composition as the mixed solvent used in the reaction. The filtrate and the washings were combined, and the combined solution was concentrated under the reduced pressure. The residue was purified by column chromatography on silica gel (development system: methylene chloride : ethanol : water : concentrated aqueous ammonia = 8 : 8 : 1 : 1) and was then purified by Sephadex LH-20 (methanol) to give the title compound (8.6 mg, 44%).

Physicochemical properties of compound prepared in Example 53

(1) Color and form: Colorless solid
(2) Molecular formula: $C_{24}H_{31}N_7O_5S$
(3) Mass spectrum (FABMS): m/z 530 $(M+H)^+$
(4) $^1H$ NMR spectrum (400 MHz, $CD_3OD$) δ (ppm): 1.61 (1H, dddd, piperidine), 1.67 - 1.77 (1H, m, piperidine), 1.81 - 2.00 (2H, m, piperidine), 1.96 (2H, quintet, tetrahydropyrimidine), 3.06 (1H, dd, piperidine), 3.14 (1H, ddd, piperidine), 3.34 - 3.42 (5H, m, tetrahydropyrimidine and piperidine), 3.53 (1H, br d, piperidine), 3.57 (1H, dd, $CONHCH_2$), 3.64 (1H, dd, $CONHCH_2$), 3.60 - 3.71 (1H, m, piperidine), 3.85 (1H, dd, $CONHCH_2CH$), 6.95 (2H, d, $C_6H_4$), 7.50 (1H, ddd, pyridine), 7.68 (2H, d, $C_6H_4$), 8.24 (1H, ddd, pyridine), 8.65 (1H, dd, pyridine), 8.97 (1H, dd, pyridine)

Example 54: t-Butyl (2S)-(Butane-1-sulfonyl)amino-3-[4-{(3S)-(pyrimidin-2-ylamino)piperidin-1-yl}benzoylamino]-propionate

Dimethylformamide (4.7 ml) was added to the compound (104 mg, 0.235 mmol) prepared in Example 41 to prepare a solution. Diisopropylethylamine (82 µl, 0.471 mmol) and 1-butanesulfonyl chloride (49 µl, 0.376 mmol) were added in that order to the solution at room temperature, and a reaction was allowed to proceed for 6 hr. Water (100 ml) and 20 ml of a saturated aqueous sodium hydrogencarbonate solution were added thereto, and the mixture was extracted twice with 100 ml of ethyl acetate. The organic layers were combined, and the combined organic layers were washed twice with 100 ml of water and once with 100 ml of saturated brine, were dried over anhydrous magnesium sulfate, and were then concentrated under the reduced pressure. The residue was purified by chromatography on silica gel (development system: hexane : ethyl acetate = 1 : 2) to give the title compound (86.7 mg, 70%).

Physicochemical properties of compound prepared in Example 54

(1) Color and form: Colorless solid
(2) Molecular formula: $C_{27}H_{40}N_6O_5S$
(3) Mass spectrum (EIMS): m/z 249 $(M)^+$
(4) Specific rotation: $[\alpha]_D^{24}$ +30° (c 0.57, $CHCl_3$)
(5) $^1H$ NMR spectrum (400 MHz, $CDCl_3$) δ (ppm): 0.91 (3H, t, n-Bu), 1.42 (2H, tq, n-Bu), 1.49 (9H, s, t-Bu), 1.57 - 1.82 (2H, m, piperidine), 1.79 (2H, tt, n-Bu), 1.83 - 1.92 (1H, m, piperidine), 1.96 - 2.06 (1H, m, piperidine), 2.90 - 3.09 (3H, m, piperidine and n-Bu), 3.14 (1H, ddd, piperidine), 3.43 - 3.50 (1H, m, piperidine), 3.73 (1H, ddd, $CONHCH_2$), 3.80 (1H, dd, piperidine), 3.87 (1H, ddd, $CONHCH_2$), 4.10 - 4.16 (1H, m, piperidine), 4.19 (1H, ddd, $CONHCH_2CH$), 6.57 (1H, t, pyrimidine), 6.92 (2H, d, $C_6H_4$), 7.67 (2H, d, $C_6H_4$), 8.30 (2H, d, pyrimidine)

Example 55: (2S)-(Butane-1-sulfonyl)amino-3-[4-{(3S)-(pyrimidin-2-ylamino)piperidin-1-yl}benzoylamino]-propionic acid

Methylene chloride (1.5 ml) was added to the compound (80.2 mg, 0.152 mmol) prepared in Example 54 to prepare a solution, and 1.5 ml of trifluoroacetic acid was added to the solution at room temperature. A reaction was allowed to proceed for 3.5 hr, and the reaction solution was then concentrated under the reduced pressure to give the title compound (115 mg, 89% (as tritrifluoroacetate)).

Physicochemical properties of compound prepared in Example 55 (as tritrifluoroacetate)

(1) Color and form: Light yellow solid
(2) Molecular formula: $C_{23}H_{32}N_6O_5S$
(3) Mass spectrum (TSPMS): m/z 505 $(M+H)^+$

Example 56: (2S)-(Butane-1-sulfonyl)amino-3-[4-{(3S)-(1,4,5,6-tetrahydropyrimidin-2-ylamino)piperidin-1-yl}-benzoylamino]propionic acid

1,4-Dioxane (3.0 ml) and 1.5 ml of water were added in that order to 112 mg of the compound prepared in Example 55 to prepare a solution. To the solution was added 29.2 mg of 10% palladium-carbon. The mixture was stirred under a hydrogen atmosphere at room temperature for 4.5 hr. The insolubles were collected by filtration and were washed with 90 ml of a solvent having the same composition as the mixed solvent used in the reaction. The filtrate and the washings were combined and were then concentrated under the reduced pressure. The residue was purified by column chromatography on silica gel (development system: methylene chloride : ethanol : water : concentrated aqueous ammonia = 8 : 8 : 1 : 1) and was then purified by Sephadex LH-20 (methanol) to give the title compound (4.3 mg, 6.2%).

Physicochemical properties of compound prepared in Example 56

(1) Color and form: Colorless solid
(2) Molecular formula: $C_{23}H_{36}N_6O_5S$
(3) Mass spectrum (TSPMS): m/z 509 $(M+H)^+$
(4) Specific rotation: $[\alpha]_D^{24}$ +8.5° (c 0.12, $CH_3OH$)
(5) $^1H$ NMR spectrum (400 MHz, $CD_3OD$) $\delta$ (ppm): 0.92 (3H, t, n-Bu), 1.41 (2H, tq, n-Bu), 1.57 - 1.94 (6H, m, piperidine and n-Bu), 1.97 (2H, quintet, tetrahydropyrimidine), 3.06 (1H, ddd, piperidine), 3.04 - 3.15 (2H, m, n-Bu), 3.19 (1H, ddd, piperidine), 3.36 (5H, m, tetrahydropyrimidine and piperidine), 3.53 (1H, dd, piperidine), 3.57 (1H, dd, $CONHCH_2$), 3.62 - 3.68 (1H, m, piperidine), 3.72 (1H, dd, $CONHCH_2$), 3.95 (1H, dd, $CONHCH_2CH$), 6.98 (2H, d, $C_6H_4$), 7.73 (2H, d, $C_6H_4$)

Example 57: t-Butyl (2S)-benzoylamino-3-[4-{(3S)-(pyrimidin-2-ylamino)piperidin-1-yl}benzoylamino]-propionate

Dimethylformamide (5.0 ml) was added to the compound (100 mg, 0.227 mmol) prepared in Example 41 to prepare a solution. Diisopropylethylamine (79 µl, 0.454 mmol), benzoic acid (27.7 mg, 0.227 mmol), and benzotriazol-1-yloxytri(dimethylamino)phosphonium hexafluorophosphate (122 mg, 0.275 mmol) were added in that order to the solution at room temperature, and a reaction was allowed to proceed for 3.5 hr. Water (100 ml) and 20 ml of a saturated aqueous sodium hydrogencarbonate solution were added to the reaction mixture, and the mixture was extracted twice with 100 ml of ethyl acetate. The organic layers were combined, and the combined organic layers were washed twice with 100 ml of water and once with 100 ml of saturated brine, were dried over anhydrous magnesium sulfate, and were concentrated under the reduced pressure. The residue was purified by chromatography on silica gel (development system: hexane : ethyl acetate = 1 : 6) to give the title compound (112 mg, 91%).

Physicochemical properties of compound prepared in Example 57

(1) Color and form: Colorless solid
(2) Molecular formula: $C_{30}H_{36}N_6O_4$
(3) Mass spectrum (TSPMS): m/z 545 $(M+H)^+$
(4) Specific rotation: $[\alpha]_D^{24}$ -22° (c 0.52, $CHCl_3$)
(5) $^1H$ NMR spectrum (400 MHz, $CDCl_3$) $\delta$ (ppm): 1.49 (9H, s, t-Bu), 1.60 - 1.80 (2H, m, piperidine), 1.83 - 1.93 (1H, m, piperidine), 1.95 - 2.04 (1H, m, piperidine), 3.03 (1H, dd, piperidine), 3.16 (1H, ddd, piperidine), 3.42 - 3.50 (1H, m, piperidine), 3.80 (1H, dd, piperidine), 3.86 (1H, ddd, $CONHCH_2$), 3.94 (1H, ddd, $CONHCH_2$), 4.15 (1H, dddd, piperidine), 4.82 (1H, ddd, $CONHCH_2CH$), 6.56 (1H, t, pyrimidine), 6.93 (2H, d, $C_6H_4$), 7.41 - 7.47 (2H, m, $C_6H_5$), 7.48 - 7.53 (1H, m, $C_6H_5$), 7.68 (2H, d, $C_6H_4$), 7.82 - 7.87 (2H, m, $C_6H_5$), 8.29 (2H, d, pyrimidine)

Example 58: (2S)-Benzoylamino-3-[4-{(3S)-(pyrimidin-2-ylamino)piperidin-1-yl}benzoylamino]propionic acid

Methylene chloride (2.0 ml) was added to the compound (106 mg, 0.194 mmol) prepared in Example 57 to prepare a solution, and 2.0 ml of trifluoroacetic acid was added to the solution at room temperature. A reaction was allowed to proceed for 2.5 hr, and the reaction solution was then concentrated under the reduced pressure to give the title compound (142 mg, 88%) (as tritrifluoroacetate)).

Physicochemical properties of compound prepared in Example 58 (as tritrifluoroacetate)

(1) Color and form: Light yellow solid
(2) Molecular formula: $C_{26}H_{28}N_6O_4$
(3) Mass spectrum (TSPMS): m/z 489 $(M+H)^+$

Example 59: (2S)-benzoylamino-3-[4-{(3S)-(1,4,5,6-tetrahydropyrimidin-2-ylamino)piperidin-1-yl}benzoylamino] propionic acid

1,4-Dioxane (3.4 ml) and 1.7 ml of water were added in that order to 141 mg of the compound prepared in Example 58 to prepare a solution. To the solution was added 36.2 mg of 10% palladium-carbon. The mixture was stirred under a hydrogen atmosphere at room temperature for 4 hr. The insolubles were collected by filtration and were washed with 90 ml of a solvent having the same composition as the mixed solvent used in the reaction. The filtrate and the washings were combined, and the combined solution was concentrated under the reduced pressure. The residue was purified by column chromatography on silica gel (development system: methylene chloride : ethanol : water : concentrated aqueous ammonia = 8 : 8 : 1 : 1) and was then purified by Sephadex LH-20 (methanol) to give the title compound (71.4 mg, 85%).

Physicochemical properties of compound prepared in Example 59

(1) Color and form: Colorless solid
(2) Molecular formula: $C_{26}H_{32}N_6O_4$
(3) Mass spectrum (FABMS): m/z 493 $(M+H)^+$
(4) $^1H$ NMR spectrum (400 MHz, $CDCl_3$) $\delta$ (ppm): 1.57 (1H, ddt, piperidine), 1.63 - 1.75 (1H, m, piperidine), 1.79 - 1.96 (2H, m, piperidine), 1.93 (2H, quintet, tetrahydropyrimidine), 3.01 (1H, dd, piperidine), 3.08 (1H, ddd, piperidine), 3.32 - 3.40 (5H, m, tetrahydropyrimidine and piperidine), 3.49 (1H, dd, piperidine), 3.60 (1H, dddd, piperidine), 3.79 (1H, dd, $CONHCH_2$), 3.83 (1H, dd, $CONHCH_2$), 4.63 (1H, dd, $CONHCH_2CH$), 6.92 (2H, d, $C_6H_4$), 7.41 - 7.46 (2H, m, $C_6H_5$), 7.48 - 7.53 (1H, m, $C_6H_5$), 7.68 (2H, d, $C_6H_4$), 7.82 - 7.86 (2H, m, $C_6H_5$)

Example 60: t-Butyl (2S)-(3-acetoxypropane-1-sulfonyl)amino-3-[4-{(3S)-(pyrimidin-2-ylamino)-piperidin-1-yl} benzoylamino]propionate

Dimethylformamide (5.0 ml) was added to the compound (100 mg, 0.227 mmol) prepared in Example 41 to prepare a solution. Diisopropylethylamine (79 μl, 0.454 mmol) and 3-acetoxy-1-propanesulfonyl chloride (126 mg, 0.630 mmol) prepared by a method described in EP 0562440 A1 were added in that order to the solution at room temperature, and a reaction was then allowed to proceed for 11.5 hr. Water (100 ml) and 20 ml of a saturated aqueous sodium hydrogencarbonate solution were added to the reaction solution, and the mixture was extracted twice with 100 ml of ethyl acetate. The organic layers were combined, and the combined organic layers were washed twice with 100 ml of water and once with 100 ml of saturated brine, were dried over anhydrous magnesium sulfate, and were then concentrated under the reduced pressure. The residue was purified by chromatography on silica gel (development system: hexane : ethyl acetate = 1 : 8) to give the title compound (168 mg, 56%).

Physicochemical properties of compound prepared in Example 60

(1) Color and form: Colorless solid
(2) Molecular formula: $C_{28}H_{40}N_6O_7S$
(3) Mass spectrum (TSPMS): m/z 605 $(M+H)^+$
(4) Specific rotation: $[\alpha]_D^{24}$ +23° (c 0.50, $CHCl_3$)
(5) $^1H$ NMR spectrum (400 MHz, $CDCl_3$) $\delta$ (ppm): 1.49 (9H, s, t-Bu), 1.54 (2H, m, piperidine), 1.82- 1.93 (1H, m, piperidine), 1.96 - 2.10 (1H, m, piperidine), 2.03 (3H, s, Ac), 2.10 - 2.24 (2H, m, Pr), 3.00 (1H, dd, piperidine), 3.09 - 3.19 (3H, m, piperidine and Pr), 3.44 - 3.52 (1H, m, piperidine), 3.76 (1H, dd, $CONHCH_2$), 3.73 - 3.89 (1H, m, piperidine), 3.85 (1H, ddd, $CONHCH_2$), 4.14 (3H, m, piperidine and Pr), 4.23 (1H, ddd, $CONHCH_2CH$), 6.56 (1H,

t, pyrimidine), 6.92 (2H, t, $C_6H_4$), 7.65 (2H, d, $C_6H_4$), 8.30 (2H, d, pyrimidine)

Example 61: (2S)-(3-Acetoxypropane-1-sulfonyl)amino-3-[4-{(3S)-(pyrimidin-2-ylamino)piperidin-1-yl}benzoylamino] propionic acid

Methylene chloride (3.0 ml) was added to the compound (163 mg, 0.270 mmol) prepared in Example 60 to prepare a solution, and 3.0 ml of trifluoroacetic acid was added to the solution at room temperature. A reaction was allowed to proceed for 6 hr, and the reaction mixture was then concentrated under the reduced pressure to give the title compound (243 mg, 100% (as tritrifluoroacetate)).

Physicochemical properties of compound prepared in Example 61 (as tritrifluoroacetate)

(1) Color and form: Brown solid
(2) Molecular formula: $C_{24}H_{32}N_6O_7S$
(3) Mass spectrum (TSPMS): m/z 549 $(M+H)^+$

Example 62: (2S)-(3-Acetoxypropane-1-sulfonyl)amino-3-[4-{(3S)-(1,4,5,6-tetrahydropyrimidin-2-ylamino)-piperidin-1-yl}benzoylamino]propionic acid

1,4-Dioxane (3.0 ml) and 1.5 ml of water were added in that order to 61.5 mg of the compound prepared in Example 61 to prepare a solution. To the solution was added 14.8 mg of 10% palladium-carbon. The mixture was stirred under a hydrogen atmosphere at room temperature for 5 hr. The insolubles were collected by filtration and were washed with 90 ml of a solvent having the same composition as the mixed solvent used in the reaction. The filtrate and the washings were combined, and the combined solution was concentrated under the reduced pressure. The residue was purified by column chromatography on silica gel (development system: methylene chloride : ethanol : water : concentrated aqueous ammonia = 8 : 8 : 1 : 1) and was then purified by Sephadex LH-20 (methanol) to give the title compound (17.7 mg, 46%).

Physicochemical properties of compound prepared in Example 62

(1) Color and form: Colorless solid
(2) Molecular formula: $C_{24}H_{36}N_6O_7S$
(3) Mass spectrum (FABMS): m/z 553 $(M+H)^+$
(4) $^1H$ NMR spectrum (400 MHz, $CD_3OD$) $\delta$ (ppm): 1.59 (1H, dddd, piperidine), 1.65 - 1.76 (1H, m, piperidine), 1.81 - 2.00 (2H, m, piperidine), 1.96 (2H, quintet, tetrahydropyrimidine), 2.02 (3H, s, Ac), 2.02 (2H, m, Pr), 3.04 (1H, dd, piperidine), 3.07 - 3.20 (3H, m, piperidine and Pr), 3.33 - 3.40 (5H, m, tetrahydropyrimidine and piperidine), 3.52 (1H, dd, piperidine), 3.58 (1H, dd, $CONHCH_2$), 3.59 - 3.68 (1H, m, piperidine), 3.70 (1H, dd, $CONHCH_2$), 3.99 (1H, dd, $CONHCH_2CH$), 4.09 (2H, t, Pr), 6.94 (2H, d, $C_6H_4$), 7.71 (2H, d, $C_6H_4$)

Example 63: t-Butyl (2S)-(3-hydroxypropane-1-sulfonyl)amino-3-[4-{(3S)-(pyrimidin-2-ylamino)-piperidin-1-yl} benzoylamino]propionate

Tetrahydrofuran (0.8 ml) and 0.2 ml of methanol were added to the compound (50.0 mg, 0.0828 mmol) prepared in Example 60 to prepare a solution. Lithium hydroxide (3.97 mg, 0.166 mmol) was added to the solution, and the mixture was stirred at room temperature for 3 hr. The reaction solution was adjusted to pH 5 by the addition of 0.1 mol/liter hydrochloric acid and was then concentrated under the reduced pressure. The residue was purified by preparative thin-layer chromatography on silica gel (development system: hexane : ethyl acetate = 1 : 10) to give the title compound (34.0 mg, 73%).

Physicochemical properties of compound prepared in Example 63

(1) Color and form: Colorless solid

(2) Molecular formula: $C_{26}H_{38}N_6O_6S$
(3) Mass spectrum (FABMS): m/z 563 $(M+H)^+$

Example 64: (2S)-(3-Hydroxypropane-1-sulfonyl)amino-3-[4-{(3S)-(pyrimidin-2-ylamino)piperidin-1-yl}benzoylamino] propionic acid

Tetrahydrofuran (0.24 ml) and 0.6 ml of methanol were added to the compound (4.0 mg, 0.00730 mmol) prepared in Example 61 to prepare a solution. Lithium hydroxide (0.4 mg, 0.0146 mmol) was added to the solution, and the mixture was stirred at room temperature for one hr. The reaction solution was adjusted to pH 5 by the addition of 0.1 mol/liter hydrochloric acid and was concentrated under the reduced pressure to give 4.0 mg of the title compound.

Physicochemical properties of compound prepared in Example 64

(1) Color and form: Colorless solid
(2) Molecular formula: $C_{22}H_{30}N_6O_6S$
(3) Mass spectrum (FABMS): m/z 507 $(M+H)^+$

Example 65: (2S)-(3-Hydroxypropane-1-sulfonyl)amino-3-[4-{(3S)-(1,4,5,6-tetrahydropyrimidin-2-ylamino)-piperidin-1-yl}benzoylamino]propionic acid

Tetrahydrofuran (0.24 ml) and 0.06 ml of methanol were added to the compound (16.0 mg, 0.0289 mmol) prepared in Example 62 to prepare a solution. Lithium hydroxide (1.40 mg, 0.0579 mmol) was added to the solution, and the mixture was stirred at room temperature for one hr. The reaction solution was adjusted to pH 5 by the addition of 0.1 mol/liter hydrochloric acid and was concentrated under the reduced pressure. The residue was purified by preparative thin-layer chromatography on silica gel (development system: methylene chloride : ethanol : concentrated aqueous ammonia : water = 8 : 8 : 1 : 1) to give the title compound (14.0 mg, 95%).

Physicochemical properties of compound prepared in Example 65

(1) Color and form: Colorless solid
(2) Molecular formula: $C_{22}H_{34}N_6O_6S$
(3) Mass spectrum (FABMS): m/z 511 $(M+H)^+$
(4) $^1H$ NMR spectrum (400 MHz, $CD_3OD$) $\delta$ (ppm): 1.62 (1H, m, piperidine), 1.73 (1H, m, piperidine), 1.95 (6H, m, piperidine, tetrahydropyrimidine and Pr), 3.10 (1H, dd, piperidine), 3.15 (2H, t, Pr), 3.18 (1H, m, piperidine), 3.35 (1H, m, piperidine), 3.37 (4H, t, tetrahydropyrimidine), 3.54 (1H, br dd, piperidine), 3.57 (1H, dd, $CONHC\underline{H_2}CH$), 3.60 (2H, t, Pr), 3.65 (1H, m, piperidine), 3.73 (1H, dd, $CONHC\underline{H_2}CH$), 3.98 (1H, dd, $CONHCH_2C\underline{H}$), 6.98 (2H, d, $C_6H_4$), 7.73 (2H, d, $C_6H_4$)

Example 66: t-Butyl (2S)-(morpholine-4-sulfonyl)amino-3-[4-{(3S)-(pyrimidin-2-ylamino)piperidin-1-yl}benzoyl amino] propionate

To the compound (66.1 mg, 0.150 mmol) prepared in Example 41 was added 0.5 ml of a 3.0 mol/liter aqueous sodium hydroxide solution to prepare a solution. A solution (3.0 ml) of morpholinosulfonyl chloride (J. Med. Chem, 40, 3820 (1997)) (28.6 mg, 0.150 mmol) in tetrahydrofuran was added to the solution, and the mixture was stirred at room temperature for 18 hr. The reaction solution was adjusted to pH 5 by the addition of 1.0 mol/liter hydrochloric acid and was extracted three times with ethyl acetate. The organic layers were combined, and the combined organic layers were dried over anhydrous magnesium sulfate and were then concentrated under the reduced pressure to give the title compound (78.8 mg).

(1) Molecular formula: $C_{27}H_{39}N_7O_6S$
(2) Mass spectrum (TSPMS): m/z 590 $(M+H)^+$

Example 69: Benzhydryl (2S)-hydroxy-3-[4-{(3S)-(pyrimidin-2-ylamino)piperidin-1-yl}benzoylamino]-propionate

Methanol (50 ml) was added to 1.00 g of 3-amino-(2S)-hydroxypropionic acid (L-isoserine) and 1.81 g of p-toluenesulfonic acid monohydrate to prepare a solution. Diphenyldiazomethane (16.1 g) was added to the solution at room temperature over a period of one hr. The reaction solution was concentrated under the reduced pressure. The residue was extracted with 100 ml of chloroform, followed by washing with a saturated aqueous sodium hydrogencarbonate solution. The chloroform layer was dried over anhydrous sodium sulfate and was concentrated under the reduced pressure. The residue was purified by column chromatography on silica gel (100 g, chloroform : methanol = 20 : 1) to give 1.25 g of benzhydryl 3-amino-(2S)-hydroxypropionate.

Physicochemical properties of benzhydryl 3-amino-(2S)-hydroxypropionate

(1) Color and form: Colorless acicular crystal
(2) Molecular formula: $C_{16}H_{17}NO_3$
(3) m.p.: 65 - 66°C
(4) Mass spectrum (TSPMS): m/z 272 $(M+H)^+$
(5) Specific rotation: $[\alpha]_D^{25}$ -20° (c 1.0, MeOH)
(6) $^1$H NMR spectrum (400 MHz, $CDCl_3$) δ (ppm): 3.04 (1H, dd, CONHC$\underline{H_2}$CH), 3.11 (1H, dd, CONHC$\underline{H_2}$CH), 4.28 (1H, dd, CONHCH$_2$C$\underline{H}$), 6.96 (1H, s, CHPh$_2$), 7.30 - 7.38 (10H, m, Ph)

Subsequently, 2.0 ml of dimethylformamide was added to intermediate 22 (50.0 mg, 0.168 mmol) to prepare a solution, and benzhydryl 3-amino-(2S)-hydroxypropionate (50.0 mg, 0.184 mmol) was added to the solution. Further, diisopropylethylamine (35 μl, 0.202 mmol) and benzotriazol-1-yloxytri(dimethylamino)-phosphonium hexafluorophosphate (BOP) (89.2 mg, 0.202 mmol) were added thereto, and the mixture was stirred at room temperature for 15 hr. A saturated aqueous sodium hydrogencarbonate solution (20 ml) was added to the reaction solution, and the mixture was extracted three times with ethyl acetate. The organic layers were combined, and the combined organic layers were washed with a mixed solution composed of 20 ml of saturated brine and 20 ml of water, were dried over anhydrous sodium sulfate, and were then concentrated under the reduced pressure. The residue was purified by preparative thin-layer chromatography on silica gel (development system: hexane : ethyl acetate =1 : 3) to give the title compound (80.0 mg, 86%).

Physicochemical properties of compound prepared in Example 69

(1) Color and form: Colorless amorphous form
(2) Molecular formula: $C_{32}H_{33}N_5O_4$
(3) Mass spectrum (TSPMS): m/z 552 $(M+H)^+$
(4) Specific rotation: $[\alpha]_D^{25}$ -8° (c 1.1, $CHCl_3$)
(5) $^1$H NMR spectrum (400 MHz, $CDCl_3$) δ (ppm): 1.65 (1H, m, piperidine), 1.75 (1H, m, piperidine), 1.88 (1H, m, piperidine), 2.00 (1H, m, piperidine), 3.01 (1H, dd, piperidine), 3.15 (1H, ddd, piperidine), 3.47 (1H, ddd, piperidine), 3.78 (2H, m, piperidine and CONHC$\underline{H_2}$CH), 3.97 (1H, dd, CONHC$\underline{H_2}$CH), 4.00 (1H, dd, CONHCH$_2$C$\underline{H}$), 4.14 (1H, m, piperidine), 6.56 (1H, t, pyrimidine), 6.88 (2H, d, $C_6H_4$), 6.92 (1H, s, C$\underline{H}$Ph$_2$), 7.30 (10H, m, CH$\underline{Ph_2}$), 7.52 (2H, d, $C_6H_4$), 8.30 (2H, d, pyrimidine)

Example 70: (2S)-Hydroxy-3-[4-{(3S)-(pyrimidin-2-ylamino)piperidin-1-yl}benzoylamino]propionic acid

Methylene chloride (1.5 ml) and anisole (16 μl, 0.145 mmol) were added to the compound (80.0 mg, 0.145 mmol) prepared in Example 69 to prepare a solution. Trifluoroacetic acid (1.0 ml) was added to the solution, and the mixture was stirred at room temperature for 3 hr. The reaction solution was concentrated under the reduced pressure to give a trifluoroacetate of the title compound.

Physicochemical properties of compound prepared in Example 70

(1) Color and form: Colorless solid
(2) Molecular formula: $C_{19}R_{23}N_5O_4$
(3) Mass spectrum (TSPMS): m/z 386 $(M+H)^+$

Example 71: (2S)-Hydroxy-3-[4-{(3S}-(1,4,5,6-tetrahydropyrimidin-2-ylamino)piperidin-1-yl}benzoylamino]-propionic acid

Dioxane (1.5 ml) and 0.15 ml of water were added to the trifluoroacetate of the crude compound (0.145 mmol) prepared in Example 70 to prepare a solution. To the solution was added 10.0 mg of 10% palladium-carbon. The mixture was vigorously stirred under a hydrogen pressure of 1 atm at room temperature for 15 hr. The insolubles were collected by filtration and were then washed with a mixed solvent composed of 10 ml of dioxane and 1 ml of water and, further, 10 ml of methanol. The filtrate and the washings were combined, and the combined solution was concentrated under the reduced pressure. The residue was dissolved in a mixed solvent composed of 5 ml of dioxane and 1 ml of water. Further, 15 ml of water was added, and the mixture was washed twice with ethyl acetate. The aqueous layer was concentrated under the reduced pressure, and the residue was purified by preparative thin-layer chromatography on silica gel (development system: methylene chloride : ethanol : water : concentrated aqueous ammonia = 8 : 8 : 1 : 1) and was then purified by Sephadex LH-20 (development system: methanol : concentrated aqueous ammonia = 10 : 1) to give the title compound (33.9 mg, 60%).

Physicochemical properties of compound prepared in Example 71

(1) Color and form: Colorless solid
(2) Molecular formula: $C_{19}H_{27}N_5O_4$
(3) Mass spectrum (FABMS): m/z 390 $(M+H)^+$
(4) Specific rotation: $[\alpha]_D^{26}$ -2.8° (c 1.7, MeOH-conc. $NH_4OH$ (10 : 1))
(5) $^1H$ NMR spectrum (400 MHz, $CD_3OD$) δ (ppm): 1.59 (1H, m, piperidine), 1.70 (1H, m, piperidine), 1.85 (1H, m, piperidine), 1.94 (1H, m, piperidine), 1.96 (2H, quintet, tetrahydropyrimidine), 3.01 (1H, dd, piperidine), 3.08 (1H, m, piperidine), 3.34 (1H, m, piperidine), 3.36 (4H, t, tetrahydropyrimidine), 3.52 (1H, br d, piperidine), 3.61 (3H, m, piperidine and $CONHC\underline{H_2}CH$), 4.07 (1H, dd, $CONHCH_2C\underline{H}$), 6.94 (2H, d, $C_6H_4$), 7.71 (2H, d, $C_6H_4$)

Intermediate 42: (3S)-Aminoazepane

Tetrahydrofuran (270 ml) was added to aluminum lithium hydride (2.60 g, 68.3 mmol) to prepare a suspension which was then ice cooled. L-α-Amino-ε-caprolactam hydrochloride (4.5 g, 27.3 mmol) was gradually added to the suspension in the internal temperature range of 10°C to 20°C. The temperature of the mixture was raised to room temperature 10 min after the completion of the addition, and the mixture was vigorously stirred for 3 hr and was then ice cooled. Water (2.6 ml), 2.6 ml of a 5.0 mol/liter aqueous sodium hydroxide solution, and 7.8 ml of water were added in that order thereto. The temperature of the mixture was raised to room temperature, and the mixture was vigorously stirred for 1.5 hr. The precipitated inorganic material was collected by filtration through Celite and was washed with tetrahydrofuran. The filtrate and the washings were combined, were dried over anhydrous sodium sulfate and were then filtered. A solution of 4.0 mol/liter hydrochloric acid in ethyl acetate (14.0 ml, 54.6 mmol) was added to the filtrate, and the solvent was removed by distillation under the reduced pressure. The residue was subjected to azeotropic distillation with methanol to give a dihydrochloride of the title compound (4.60 g, 90%).

Physicochemical properties of intermediate 42

(1) Color and form: Brown solid
(2) Molecular formula: $C_6H_{14}N_2$
(3) Mass spectrum (EIMS): m/z 114 $(M)^+$
(4) $^1H$ NMR spectrum (400 MHz, $CD_3OD$) (as dihydrochloride) δ (ppm): 1.72 (1H, m, azepane), 1.93 (4H, m,

azepane), 2.21 (1H, m, azepane), 3.30 (2H, m, azepane), 3.61 (2H, m, azepane), 3.73 (1H, dddd, azepane)

Intermediate 43: 4-{(3S)-Aminoazepan-1-yl}benzonitrile

N-methylpyrrolidone (2.6 ml) was added to intermediate 42 (1.0 g, 5.34 mmol) to prepare a solution, and sodium hydrogencarbonate (1.35 g, 16.0 mmol) and 4-fluorobenzonitrile (323 mg, 2.67 mmol) were added to the solution. The mixture was stirred in a hermetically sealed container at 120°C for 18 hr. A saturated aqueous sodium hydrogencarbonate solution (100 ml) was then added to the reaction solution, and the mixture was extracted three times with ethyl acetate. The organic layers were combined, and the combined organic layers were washed twice with 200 ml of a saturated aqueous sodium hydrogencarbonate solution, were dried over anhydrous sodium sulfate, and were then concentrated under the reduced pressure. The residue was purified by column chromatography on silica gel (development system: methylene chloride : methanol = 7 : 1) to give the title compound (210.6 mg, 37%).

Physicochemical properties of intermediate 43

(1) Color and form: Colorless syrup
(2) Molecular formula: $C_{13}H_{17}N_3$
(3) $^1H$ NMR spectrum (400 MHz, $CD_3OD$) δ (ppm): 1.29 (1H, m, azepane), 1.44 (1H, m, azepane), 1.78 (3H, m, azepane), 2.02 (1H, m, azepane), 3.06 (2H, m, azepane), 3.35 (1H, dd, azepane), 3.74 (1H, ddd, azepane), 3.84 (1H, br d, azepane), 6.83 (2H, br d, $C_6H_4$), 7.46 (2H, br d, $C_6H_4$)

Intermediate 44: 4-{(3S)-(Pyrimidin-2-ylamino)azepan-1-yl}benzonitrile

Dimethyl sulfoxide (4.6 ml) was added to intermediate 43 (100 mg, 0.464 mmol) to prepare a solution, and 2-bromopyrimidine (81.2 mg, 0.510 mmol) was added to the solution. Further, 0.46 ml of diisopropylethylamine was added thereto, and the mixture was heated to 110°C and was stirred for 5 hr. The temperature of the reaction solution was returned to room temperature. A mixed solution composed of 30 ml of saturated brine and 30 ml of water was then added thereto, and the mixture was extracted three times with ethyl acetate. The organic layers were combined, and the combined organic layers were washed twice with 60 ml of brine, were dried over anhydrous sodium sulfate, and were then concentrated under the reduced pressure. The residue was purified by preparative thin-layer chromatography on silica gel (development system: hexane : ethyl acetate = 1 : 2) to give the title compound (95.0 mg, 70%).

Physicochemical properties of intermediate 44

(1) Color and form: Colorless solid
(2) Molecular formula: $C_{17}H_{19}N_5$
(3) Mass spectrum (TSPMS): m/z 294 $(M+H)^+$
(4) Specific rotation: $[\alpha]_D^{27}$ +122° (c 0.67, $CHCl_3$)
(5) $^1H$ NMR spectrum (400 MHz, $CDCl_3$) δ (ppm): 1.50 (2H, m, azepane), 1.81 (2H, m, azepane), 2.05 (2H, m, azepane), 3.21 (1H, dd, azepane), 3.36 (1H, ddd, azepane), 3.64 (1H, ddd, azepane), 4.04 (1H, dd, azepane), 4.20 (1H, ddddd, azepane), 6.60 (1H, t, pyrimidine), 7.01 (2H, d, $C_6H_4$), 7.47 (2H, d, $C_6H_4$), 8.33 (2H, d, pyrimidine)

Intermediate 45: 4-{(3S)-(Pyrimidin-2-ylamino)azepan-1-yl}benzoic acid

Intermediate 44 (95.0 mg, 0.324 mmol) was dissolved in 50% sulfuric acid (0.3 ml), and the solution was heated under reflux for 3 hr. The temperature of the reaction solution was returned to room temperature, and the reaction solution was then gradually poured into 20 ml of an aqueous sodium hydrogencarbonate solution under ice cooling. This mixed solution was adjusted to pH 4 by the addition of 5.0 mol/liter hydrochloric acid. The precipitate was collected by filtration through a glass filter, was washed with water, and was then dried to give the title compound (95.9 g, 95%).

Physicochemical properties of intermediate 45

(1) Color and form: Brown solid
(2) Molecular formula: $C_{17}H_{20}N_4O_2$
(3) Mass spectrum (TSPMS): m/z 313 $(M+H)^+$

Example 72: t-Butyl (2S)-benzenesulfonylamino-3-[4-{(3S)-(pyrimidin-2-ylamino)azepan-1-yl}benzoylamino]-propionate

Dimethylformamide (1.6 ml) was added to intermediate 45 (50.0 mg, 0.160 mmol) to prepare a solution, and t-butyl (2S)-N-benzenesulfonyl-2,3-diaminopropionate (52.9 mg, 0.176 mmol) was added to the solution. Further, diisopropylethylamine (33 μl, 0.192 mmol) and benzotriazol-1-yloxytri(dimethylamino)phosphonium hexafluorophosphate (BOP) (85.0 mg, 0.192 mmol) were added thereto, and the mixture was stirred at room temperature for 20.5 hr. A saturated aqueous sodium hydrogencarbonate solution (20 ml) was added to the reaction solution, and the mixture was extracted three times with ethyl acetate. The organic layers were combined, and the combined organic layers were washed with a mixed solution composed of 20 ml of saturated brine and 20 ml of water, were dried over anhydrous sodium sulfate, and were then concentrated under the reduced pressure. The residue was purified by preparative thin-layer chromatography on silica gel (development system: hexane : ethyl acetate = 1 : 3) to give the title compound (91.8 mg, 96%).

Physicochemical properties of compound prepared in Example 72

(1) Color and form: Colorless syrup
(2) Molecular formula: $C_{30}H_{38}N_6O_5S$
(3) Mass spectrum (FABMS): m/z 595 $(M+H)^+$
(4) Specific rotation: $[\alpha]_D^{25}$ +85° (c 0.55, MeOH)
(5) $^1$H NMR spectrum (400 MHz, $CDCl_3$) δ (ppm): 1.29 (9H, s, t-Bu), 1.50 (2H, m, azepane), 1.74 (2H, m, azepane), 2.02 (2H, m, azepane), 3.06 (1H, dd, azepane), 3.30 (1H, ddd, azepane), 3.61 (1H, ddd, azepane), 3.77 (2H, m, $CONHCH_2CH$), 3.92 (1H, dd, azepane), 3.97 (1H, dd, $CONHCH_2CH$), 4.22 (1H, ddddd, azepane), 6.60 (1H, t, pyrimidine), 6.97 (2H, d, $C_6H_4$), 7.48 (2H, br t, $C_6H_5$), 7.55 (1H, br t, $C_6H_5$), 7.63 (2H, d, $C_6H_4$), 7.86 (2H, m, $C_6H_5$), 8.36 (1H, d, pyrimidine)

Example 73: (2S)-Benzenesulfonylamino-3-[4-{(3S)-(pyrimidin-2-ylamino)azepan-1-yl}benzoylamino]propionic acid

Methylene chloride (1.5 ml) was added to the compound (91.8 mg, 0.154 mmol) prepared in Example 72 to prepare a solution. Trifluoroacetic acid (1.0 ml) was added to the solution, and the mixture was stirred at room temperature for 3 hr. The reaction solution was concentrated under the reduced pressure to give a trifluoroacetate of the title compound.

Physicochemical properties of compound prepared in Example 73

(1) Color and form: Colorless syrup
(2) Molecular formula: $C_{26}H_{30}N_6O_5S$
(3) Mass spectrum (TSPMS): m/z 539 $(M+H)^+$

Example 74: (2S)-Benzenesulfonylamino-3-[4-{(3S)-(1,4,5,6-tetrahydropyrimidin-2-ylamino)azepan-1-yl}-benzoylamino]propionic acid

Dioxane (1.5 ml) and 0.15 ml of water were added to the trifluoroacetate of the crude compound (0.154 mmol) prepared in Example 73 to prepare a solution. To the solution was added 10.0 mg of 10% palladium-carbon. The mixture was vigorously stirred under a hydrogen pressure of 1 atm at room temperature for 13 hr. The insolubles were collected by filtration and were then washed with 15 ml of methanol. The filtrate and the washings were combined, and

the combined solution was concentrated under the reduced pressure. The residue was purified by preparative thin-layer chromatography on silica gel (development system: methylene chloride : ethanol : water : concentrated aqueous ammonia = 8 : 8 : 1 : 1) and was then purified by Sephadex LH-20 (development system: methanol : concentrated aqueous ammonia = 10 : 1) to give the title compound (49.1 mg, 59%).

Physicochemical properties of compound prepared in Example 74

(1) Color and form: Colorless solid
(2) Molecular formula: $C_{26}H_{34}N_6O_5S$
(3) Mass spectrum (FABMS): m/z 543 $(M+H)^+$
(4) Specific rotation: $[\alpha]_D^{26}$ +100° (c 0.55, MeOH-conc. $NH_4OH$ (10 : 1))
(5) $^1H$ NMR spectrum (400 MHz, $CD_3OD$) δ (ppm): 1.57 (2H, m, azepane), 1.75 (3H, m, azepane), 1.95 (1H, m, azepane), 1.97 (2H, quintet, tetrahydropyrimidine), 3.37 (4H, dd, tetrahydropyrimidine), 3.39 (1H, m, azepane), 3.56 (1H, dd, $CONHC\underline{H_2}CH$), 3.70 (5H, m, azepane and $CONHC\underline{H_2}C\underline{H}$), 3.84 (1H, m, azepane), 6.75 (2H, br d, $C_6H_4$), 7.50 (2H, m, $C_6H_5$), 7.56 (1H, m, $C_6H_5$), 7.70 (2H, br d, $C_6H_4$), 7.87 (2H, m, $C_6H_5$)

Intermediate 46: 4-[(3S)-{(t-Butoxycarbonyl)amino}-piperidin-1-yl]benzoic acid

Intermediate 23 (100 mg, 0.403 mmol) was added to 4.0 ml of dimethylformamide to prepare a solution. Triethylamine (112 µl, 0.806 mmol) and di-t-butyl dicarbonate (111 µl, 0.483 mmol) were added to the solution, and the mixture was stirred at room temperature for 1.5 hr. Brine (50 ml) was added to the reaction solution, and the mixture was extracted three times with ethyl acetate. The organic layers were combined, and the combined organic layers were washed twice with a mixed solution composed of 50 ml of saturated brine and 50 ml of water, were dried over anhydrous sodium sulfate, and were then concentrated under the reduced pressure to give a crude compound. Subsequently, tetrahydrofuran (6.0 ml) and 2.0 ml of methanol were added to the crude compound to prepare a solution, and 2.0 ml of a 1.0 mol/liter aqueous sodium hydroxide solution was added to the solution. The mixture was stirred at 40°C for 18 hr and then at 60°C for 10 hr, and was then concentrated under the reduced pressure. Water (3 ml) was added to the residue to prepare a solution which was then adjusted to pH 7 by the addition of 1.0 mol/liter hydrochloric acid. The precipitated solid was collected by filtration through a glass filter, was washed twice with water, and was then dried to give the title compound (118.3 mg, 92%).

Physicochemical properties of intermediate 46

(1) Color and form: Colorless solid
(2) Molecular formula: $C_{17}H_{24}N_2O_4$
(3) Mass spectrum (EIMS): m/z 320 $(M)^+$
(4) $^1H$ NMR spectrum (400 MHz, DMSO-$\underline{d}_6$) δ (ppm): 1.40 (9H, s, t-Bu), 1.45 (2H, m, piperidine), 1.72 (1H, m, piperidine), 1.83 (1H, m, piperidine), 2.50 (1H, m, piperidine), 2.72 (1H, br dd, piperidine), 2.84 (1H, ddd, piperidine), 3.39 (1H, m, piperidine), 3.75 (1H, m, piperidine), 6.91 (2H, d, $C_6H_4$), 7.74 (2H, d, $C_6H_4$)

Intermediate 47: t-Butyl 3-[4-{(3S)-aminopiperidin-1-yl}benzoylamino]-(2S)-(benzenesulfonylamino)propionate

Dimethylformamide (2.5 ml) was added to intermediate 46 (80.0 mg, 0.250 mmol) to prepare a solution, and t-butyl (2S)-N-benzenesulfonyl-2,3-diaminopropionate (90.0 mg, 0.300 mmol) was added to the solution. Further, diisopropylethylamine (65 µl, 0.375 mmol) and benzotriazol-1-yloxytri(dimethylamino)phosphonium hexafluorophosphate (BOP) (166.0 mg, 0.375 mmol) were added thereto, and the mixture was stirred at room temperature for 15 hr. Water (15 ml) and 30 ml of a saturated aqueous potassium carbonate solution were added to the reaction solution, and the mixture was extracted three times with ethyl acetate. The organic layers were combined, and the combined organic layers were washed with a mixed solution composed of 50 ml of saturated brine and 50 ml of water, were dried over anhydrous sodium sulfate, and were then concentrated under the reduced pressure. The residue was purified by preparative thin-layer chromatography on silica gel (development system: hexane : ethyl acetate = 1 : 2) to give t-butyl (2S)-benzenesulfonylamino-3-[4-[(3S)-{(t-butoxycarbonyl)-amino}piperidin-1-yl]benzoylamino]propionate (150 mg, 100%).

Physicochemical properties of t-butyl (2S)-benzenesulfonylamino-3-[4-[(3S)-{(t-butoxycarbonyl)-amino}piperidin-1-yl] benzoylamino]propionate

(1) Color and form: Colorless syrup
(2) Molecular formula: $C_{30}H_{42}N_4O_7S$
(3) Mass spectrum (FABMS): m/z 602 (M+H)$^-$
(4) $^1$H NMR spectrum (400 MHz, $CDCl_3$) δ (ppm): 1.29 (9H, s, t-Bu), 1.47 (9H, s, Boc), 1.55 (1H, m, piperidine), 1.69 (1H, m, piperidine), 1.80 (1H, m, piperidine), 1.86 (1H, m, piperidine), 3.06 (1H, m, piperidine), 3.18 (1H, m, piperidine), 3.31 (1H, m, piperidine), 3.59 (2H, m, piperidine or CONHC$\underline{H_2}$CH), 3.81 (1H, m, piperidine), 3.90 (2H, m, piperidine or CONHC$\underline{H_2}$C$\underline{H}$), 6.91 (2H, d, $C_6H_4$), 7.49 (2H, br t, $C_6H_5$), 7.57 (1H, br t, $C_6H_5$), 7.68 (2H, d, $C_6H_4$), 7.86 (2H, m, $C_6H_5$)

Methylene chloride (2.0 ml) was added to the above compound (109.0 mg, 0.181 mmol) to prepare a solution which was then ice cooled. Trifluoroacetic acid (0.4 ml) was added to the cooled solution, and the mixture was stirred at that temperature for 5.5 hr. The reaction solution was poured into a mixed solvent composed of 10 ml of methanol and 10 ml of concentrated aqueous ammonia which had been cooled to 0°C. The mixture was concentrated under the reduced pressure. The residue was purified by column chromatography on silica gel (development system: chloroform : methanol : concentrated aqueous ammonia = 30 : 10 : 1) to give the title compound (90 mg, 100%).

Physicochemical properties of intermediate 47

(1) Color and form: Colorless amorphous form
(2) Molecular formula: $C_{25}H_{34}N_4O_5S$
(3) Mass spectrum (TSPMS): m/z 503 (M+H)$^+$
(4) $^1$H NMR spectrum (400 MHz, $CD_3OD$) δ (ppm): 1.23 (9H, s, t-Bu), 1.42 (1H, dddd, piperidine), 1.68 (1H, m, piperidine), 1.85 (1H, m, piperidine), 2.00 (1H, m, piperidine), 2.78 (1H, dd, piperidine), 2.96 (2H, m, piperidine), 3.49 (1H, dd, CONHC$\underline{H_2}$CH), 3.61 (1H, m, piperidine), 3.65 (1H, dd, CONHC$\underline{H_2}$CH), 3.75 (1H, br dd, piperidine), 4.11 (1H, dd, CONHCH$_2$C$\underline{H}$), 6.96 (2H, d, $C_6H_4$), 7.47 (2H, br t, $C_6H_5$), 7.54 (1H, br t, $C_6H_5$), 7.67 (2H, d, $C_6H_4$), 7.83 (2H, m, $C_6H_5$)

Example 75: t-Butyl (2S)-benzenesulfonylamino-3-[4-{(3S)-guanidinopiperidin-1-yl}benzoylamino]propionate

Dioxane (0.36 ml) and 0.36 ml of water were added to intermediate 47 (82.2 mg, 0.164 mmol) to prepare a solution. 1H-Pyrazole-1-carboxamidine hydrochloride (105 mg, 0.719 mmol) and diisopropylethylamine (0.120 ml, 0.689 mmol) were added to the solution, and the mixture was vigorously stirred at room temperature for 18 hr. The reaction solution was concentrated under the reduced pressure, and the residue was purified by column chromatography on silica gel (development system: chloroform : methanol : concentrated aqueous ammonia = 9 : 3 : 0.3) to give the title compound (50.1 mg, 56%).

Physicochemical properties of compound prepared in Example 75

(1) Color and form: Light yellow solid
(2) Molecular formula: $C_{26}H_{36}N_6O_5S$
(3) Mass spectrum (FABMS): m/z 545 (M+H)$^+$
(4) Specific rotation: $[\alpha]_D^{26}$ +22° (c 1.5, MeOH)
(5) $^1$H NMR spectrum (400 MHz, $CD_3OD$) δ (ppm): 1.23 (9H, s, t-Bu), 1.67 (1H, m, piperidine), 1.76 (1H, m, piperidine), 1.93 (2H, m, piperidine), 3.20 (2H, m, piperidine), 3.37 (1H, m, piperidine), 3.49 (1H, dd, CONHC$\underline{H_2}$CH), 3.57 (1H, m, piperidine), 3.65 (1H, dd, CONHC$\underline{H_2}$CH), 3.77 (1H, m, piperidine), 4.12 (1H, dd, CONHCH$_2$C$\underline{H}$), 7.00 (2H, m, $C_6H_4$), 7.48 (2H, m, $C_6H_5$), 7.55 (1H, m, $C_6H_5$), 7.69 (2H, m, $C_6H_4$), 7.84 (2H, m, $C_6H_5$)

Example 76: (2S)-Benzenesulfonylamino-3-[4-{(3S)-guanidinopiperidin-1-yl}benzoylamino]propionic acid

Methylene chloride (0.5 ml) was added to the compound (18.4 mg, 0.0338 mmol) prepared in Example 75 to prepare a solution. Trifluoroacetic acid (0.5 ml) was added to the solution, and the mixture was stirred at room temperature for 18 hr. The reaction solution was concentrated under the reduced pressure to give a trifluoroacetate of the title compound. The residue was purified by preparative thin-layer chromatography on silica gel (development system: methylene chloride : ethanol : concentrated aqueous ammonia : water = 8 : 8 : 1 : 1) and was then purified by Sephadex LH-20 (development system: methanol) to give the title compound (15.5 mg, 94%).

Physicochemical properties of compound prepared in Example 76

(1) Color and form: Colorless solid
(2) Molecular formula: $C_{22}H_{28}N_6O_5S$
(3) Mass spectrum (TSPMS): m/z 489 $(M+H)^+$
(4) Specific rotation: $[\alpha]_D^{26}$ +66° (c 0.097, MeOH)
(5) $^1$H NMR spectrum (400 MHz, $CD_3OD$) δ (ppm): 1.71 (2H, m, piperidine), 1.92 (2H, m, piperidine), 3.15 (1H, m, piperidine), 3.21 (1H, m, piperidine), 3.38 (1H, m, piperidine), 3.47 (2H, m, piperidine and CONHC$\underline{H_2}$CH), 3.62 (1H, m, CONHC$\underline{H_2}$CH), 3.75 (1H, m, piperidine), 3.92 (1H, m, $CONHCH_2C\underline{H}$), 6.99 (2H, d, $C_6H_4$), 7.44 (2H, m, $C_6H_5$), 7.51 (1H, m, $C_6H_5$), 7.69 (2H, d, $C_6H_4$), 7.83 (2H, d, $C_6H_5$)

Intermediate 48: 4-{(3S)-(6-Hydroxypyridin-2-ylamino)-piperidin-1-yl}benzonitrile

Under an argon atmosphere, 13 ml of toluene was added to a mixture of intermediate 20 (516 mg, 2.56 mmmol), 6-methoxy-2-chloropyridine (549 mg, 3.82 mmol), palladium acetate (58.3 mg, 0.260 mmol), (R)-(+)-2,2'-bis (diphenylphosphino)-1,1'-binaphthyl (BINAP) (161 mg, 0.259 mmol), and sodium t-butoxide (296 mg, 3.08 mmol) to prepare a suspension. Argon was blown into the suspension with ultrasonication for 5 min. The reaction mixture was stirred at 75°C for 18 hr. A saturated aqueous ammonium chloride solution was then added to the reaction solution, and the mixture was extracted three times with ethyl acetate. The organic layers were combined, and the combined organic layers were dried over anhydrous magnesium sulfate, and were then concentrated under the reduced pressure. The residue was purified by column chromatography on silica gel (development system: hexane : ethyl acetate = 2 : 1) to give the title compound (418 mg, 53%).

Physicochemical properties of intermediate 48

(1) Color and form: Light yellow syrup
(2) Molecular formula: $C_{18}H_{20}N_4O$
(3) Mass spectrum (EIMS): m/z 308 $(M)^+$
(4) Specific rotation: $[\alpha]_D^{25}$ +6.1° (c 1.1, $CHCl_3$)
(5) $^1$H NMR spectrum (400 MHz, $CDCl_3$) δ (ppm): 1.60 (1H, m, piperidine), 1.73 (1H, m, piperidine), 1.87 (1H, m, piperidine), 2.04 (1H, m, piperidine), 2.96 (1H, dd, piperidine), 3.12 (1H, ddd, piperidine), 3.56 (1H, ddd, piperidine), 3.85 (3H, s, $OCH_3$), 3.93 (2H, m, piperidine), 4.44 (1H, brd, NH), 5.96 (1H, dd, pyridine), 6.04 (1H, dd, pyridine), 6.88 (2H, d, $C_6H_4$), 7.33 (1H, m, pyridine), 7.45 (2H, m, $C_6H_4$)

Intermediate 49: 4-{(3S)-(6-Hydroxypyridin-2-ylamino)-piperidin-1-yl}benzoic acid

To intermediate 48 (203 mg, 0.657 mmmol) was added 10 ml of 5.0 mol/liter hydrochloric acid to prepare a suspension. The suspension was stirred under reflux for 18 hr, and the reaction solution was then concentrated under the reduced pressure to give the title compound as a crude compound (256 mg).

Physicochemical properties of intermediate 49

(1) Color and form: Purple syrup

(2) Molecular formula: $C_{17}H_{19}N_3O_3$
(3) Mass spectrum (TSPMS): m/z 314 $(M+H)^+$
(4) $^1H$ NMR spectrum (400 MHz, $CD_3OD$) δ (ppm): 1.55 (2H, m, piperidine), 1.77 (1H, m, piperidine), 1.99 (1H, m, piperidine), 2.72 (1H, m, piperidine), 2.92 (1H, m, piperidine), 3.82 (2H, m, piperidine), 4.06 (1H, m, piperidine), 5.88 (1H, d, pyridine), 6.05 (1H, d, pyridine), 6.96 (2H, d, $C_6H_4$), 7.29 (1H, dd, pyridine), 7.73(2H, d, $C_6H_4$)

Example 77: t-Butyl (2S)-benzenesulfonylamino-3-[4-{(3S)-(6-hydroxypyridin-2-ylamino)piperidin-1-yl}-benzoylamino] propionate

Dimethylformamide (8.0 ml) was added to intermediate 49 as the crude compound (256 mg) to prepare a solution, and t-butyl (2S)-N-benzenesulfonyl-2,3-diaminopropionate (234 mg, 0.780 mmol) was added to the solution. Further, benzotriazol-1-yloxytri(dimethylamino)phosphonium hexafluorophosphate (BOP) (416 mg, 0.941 mmol) and diisopropylethylamine (0.163 ml, 0.936 mmol) were added thereto, and the mixture was stirred at room temperature for 18 hr. Water and a saturated aqueous sodium hydrogencarbonate solution were added to the reaction solution, and the mixture was extracted twice with ethyl acetate. The organic layers were combined, and the combined organic layers were washed with a mixed solution composed of saturated brine and water, were dried over anhydrous sodium sulfate, and were then concentrated under the reduced pressure to give the title compound as a crude compound (287 mg).

Physicochemical properties of compound prepared in Example 77

(1) Color and form: Purple solid
(2) Molecular formula: $C_{30}H_{37}N_5O_6S$
(3) Mass spectrum (FABMS): m/z 596 $(M+H)^+$
(4) $^1H$ NMR spectrum (400 MHz, $CDCl_3$) δ (ppm): 1.27 (9H, s, t-Bu), 1.63 (2H, m, piperidine), 1.85 (1H, m, piperidine), 1.96 (1H, m, piperidine), 2.92 (1H, m, piperidine), 3.03 (1H, m, piperidine), 3.38 (1H, m), 3.48 (1H, m), 3.58 (1H, m), 3.67 (1H, m), 3.79 (1H, m), 4.01 (1H, m), 5.39 (1H, d, NH), 5.67 (1H, d, NH), 6.28 (1H, brd, pyridine), 6.81 (2H, d, $C_6H_4$), 7.12 (1H, m, pyridine), 7.24 (1H, dd, pyridine), 7.41 (2H, m, $C_6H_5$), 7.49 (1H, m, $C_6H_5$), 7.66 (2H, d, $C_6H_4$), 7.84 (2H, m, $C_6H_5$)

Example 78: (2S)-Benzenesulfonylamino-3-[4-{(3S)-(6-hydroxypyridin-2-ylamino)piperidin-1-yl}-benzoylamino]-propionic acid

Methylene chloride (1.0 ml) was added to the crude compound (67.3 mg) prepared in Example 77 to prepare a solution. Trifluoroacetic acid (1.0 ml) was added to the solution, and the mixture was stirred at room temperature for 18 hr. The reaction solution was concentrated under the reduced pressure to give a trifluoroacetate of the title compound. The residue was purified by preparative thin-layer chromatography on silica gel (development system: methylene chloride : ethanol : concentrated aqueous ammonia : water = 8 : 8 : 1 : 1) to give the title compound.

Physicochemical properties of compound prepared in Example 78

(1) Color and form: Colorless liquid
(2) Molecular formula: $C_{26}H_{29}N_5O_6S$
(3) Mass spectrum (TSPMS): m/z 540 $(M+H)^+$
(4) $^1H$ NMR spectrum (400 MHz, $CDCl_3$) δ (ppm): 1.73 (2H, m, piperidine), 1.93 (2H, m, piperidine), 3.20 (1H, dd, piperidine), 3.31 (2H, m, piperidine), 3.56 (2H, m), 3.66 (2H, m), 3.81 (1H, dd), 5.62(1H, d, pyridine), 5.70(1H, dd, pyridine), 6.96 (2H, m, $C_6H_4$), 7.32 (1H, m, pyridine), 7.44 (2H, m, $C_6H_5$), 7.51 (1H, m, $C_6H_5$), 7.68 (2H, d, $C_6H_4$), 7.85 (2H, m, $C_6H_5$)

Intermediate 52: (3S)-(Tritylamino)piperidin-2-one

Methylene chloride (100 ml) was added to intermediate 7 (5.70 g, 50.0 mmmol) to prepare a solution, and triethylamine (14 ml, 100 mmol) and triphenylmethyl chloride (16.7 g, 60.0 mmol) were added to the solution. The mixture was stirred at room temperature for 16 hr. Water (200 ml) was then added to the reaction solution, and the

mixture was extracted three times with methylene chloride, was dried over anhydrous sodium sulfate, and was then concentrated under the reduced pressure. The residue was purified by column chromatography on silica gel (development system: hexane : ethyl acetate = 1 : 1) to give the title compound (9.10 g, 51%).

Physicochemical properties of intermediate 52

(1) Color and form: Colorless solid
(2) Molecular formula: $C_{24}H_{24}N_2O$
(3) Mass spectrum (EIMS): m/z 356 $(M)^+$
(4) $^1H$ NMR spectrum (400 MHz, $CDCl_3$) $\delta$ (ppm): 1.14 (1H, m, piperidine), 1.36 (2H, m, piperidine), 1.38 (2H, m, piperidine), 2.88 (1H, m, piperidine), 3.13 (1H, m, piperidine), 3.19 (1H, m, piperidine), 3.93 (1H, brs, NH), 5.62 (1H, brs, NH), 7.17 (3H, m, $C_6H_5$), 7.25 (6H, m, $C_6H_5$), 7.58 (6H, m, $C_6H_5$)

Intermediate 53: (3S)-(Tritylamino)piperidine

Tetrahydrofuran (40 ml) was added to intermediate 52 (720 mg, 2.00 mmmol) to prepare a solution. Borane-dimethyl sulfide (2.5 ml, 5.00 mmol) was added to the solution under cooling at 0°C, and the mixture was stirred at room temperature for 16 hr. Methanol (50 ml) was added to the reaction solution, and the mixture was then concentrated under the reduced pressure. The residue was purified by column chromatography on silica gel (development system: chloroform : methanol : concentrated aqueous ammonia = 3 : 1 : 0.1) to give the title compound (150 mg, 22%).

Physicochemical properties of intermediate 53

(1) Color and form: Colorless solid
(2) Molecular formula: $C_{24}H_{26}N_2$
(3) Mass spectrum (TSPMS): m/z 343 $(M+H)^+$
(4) $^1H$ NMR spectrum (400 MHz, $CD_3OD$) $\delta$ (ppm): 1.10 (1H, m, piperidine), 1.20 (1H, m, piperidine), 1.28 (1H, m, piperidine), 1.50 (1H, m, piperidine), 2.03 (1H, dd, piperidine), 2.37 (2H, m, piperidine), 2.47 (1H, m, piperidine), 2.70 (1H, m, piperidine), 7.15 (3H, m, $C_6H_5$), 7.22 (6H, m, $C_6H_5$), 7.53 (6H, m, $C_6H_5$)

Intermediate 54: Ethyl 2-methyl-4-{(3S)-(tritylamino)piperidin-1-yl}benzoate

Toluene (2.0 ml) was added to intermediate 53 (80.0 mg, 0.230 mmol) to prepare a solution. $Pd_2(dba)_3$ (3.4 mg, 0.00400 mmol), BINAP (1.9 mg, 0.0030 mmol), and cesium carbonate (92 mg, 0.290 mmol) were added to the solution, and the mixture was stirred at 100°C for 2 days. Water (50 ml) was added to the reaction solution, and the mixture was extracted three times with ethyl acetate. The organic layers were combined, and the combined organic layers were dried over anhydrous magnesium sulfate, and were then concentrated under the reduced pressure. The residue was purified by column chromatography on silica gel (development system: hexane : ethyl acetate =1 : 1) to give the title compound (55.2 mg, 58%).

Physicochemical properties of intermediate 54

(1) Color and form: Colorless syrup
(2) Molecular formula: $C_{34}H_{36}N_2O_2$
(3) Mass spectrum (EIMS): m/z 506 $(M+H)^+$
(4) $^1H$ NMR spectrum (400 MHz, $CDCl_3$) $\delta$ (ppm): 1.34 (3H, t, $CH_2\underline{CH_3}$), 1.40 (2H, m, piperidine), 1.58 (1H, m, piperidine), 1.73 (1H, m, piperidine), 2.24 (1H, dd, piperidine), 2.52 (3H, s, $CH_3$), 2.73 (1H, m, piperidine), 2.88 (1H, m, piperidine), 2.96 (1H, dd, piperidine), 3.29 (1H, m, piperidine), 4.28 (2H, q, $CH_2\underline{CH_3}$), 6.28 (1H, d, $C_6H_3$), 6.33 (1H, dd, $C_6H_3$), 7.23 (9H, m, $C_6H_5$), 7.56 (6H, m, $C_6H_5$), 7.73 (1H, d, $C_6H_3$)

Intermediate 55: Ethyl 4-{(3S)-aminopiperidin-1-yl}-2-methylbenzoate

Methylene chloride (5.0 ml) and 1.0 ml of trifluoroacetic acid were added to intermediate 54 (50.0 mg, 0.10 mmol) to prepare a solution. The solution was stirred at room temperature for 16 hr and was then concentrated under the reduced pressure. Water (8.0 ml) was added to the residue to prepare a solution which was then washed twice with ethyl acetate. The aqueous layer was neutralized with sodium hydrogencarbonate and was then extracted with ethyl acetate. The extract was dried over anhydrous magnesium sulfate and was then concentrated under the reduced pressure to give the title compound (12.6 mg, 45%).

Physicochemical properties of intermediate 55

(1) Color and form: Colorless solid
(2) Molecular formula: $C_{15}H_{22}N_2O_2$
(3) Mass spectrum (TSPMS): m/z 263 $(M+H)^+$
(4) $^1H$ NMR spectrum (400 MHz, $CDCl_3$) $\delta$ (ppm): 1.22 (1H, m, piperidine), 1.34 (3H, t, $CH_2\underline{CH_3}$), 1.67 (2H, m, piperidine), 1.87 (1H, m, piperidine), 3.08 (1H, dd, piperidine), 3.19 (1H, dd, piperidine), 3.30 (2H, m, piperidine), 3.55 (1H, dd, piperidine), 4.27 (2H, q, $\underline{CH_2}CH_3$), 5.11 (2H, brs, $NH_2$), 6.68 (2H, m, $C_6H_3$), 7.82 (1H, d, $C_6H_3$)

Intermediate 56: Ethyl 2-methyl-4-{(3S)-(pyrimidin-2-ylamino)piperidin-1-yl}benzoate

N-Methylpyrrolidone (0.30 ml) was added to intermediate 55 (39.0 mg, 0.148 mmol) to prepare a solution. N,N-Diisopropylethylamine (0.096 ml, 0.740 mmol) and 2-bromopyrimidine (28.0 mg, 0.178 mmol) were added to the solution, and the mixture was stirred at 110°C for 20 hr. Water (50 ml) was then added to the reaction solution, and the mixture was extracted three times with ethyl acetate. The organic layers were combined, and the combined organic layers were dried over anhydrous magnesium sulfate, and were then concentrated under the reduced pressure. The residue was purified by preparative thin-layer chromatography on silica gel (development system: hexane : ethyl acetate = 1 : 1) to give the title compound (7.25 mg, 14%).

Physicochemical properties of intermediate 56

(1) Color and form: Colorless solid
(2) Molecular formula: $C_{19}H_{24}N_4O_2$
(3) Mass spectrum (FABMS): m/z 341 $(M+H)^+$
(4) $^1H$ NMR spectrum (400 MHz, $CDCl_3$) $\delta$ (ppm): 1.36 (3H, t, $CH_2\underline{CH_3}$), 1.74 (2H, m, piperidine), 1.85 (1H, m, piperidine), 2.00 (1H, m, piperidine), 2.58 (3H, s, $CH_3$), 2.62 (1H, m, piperidine), 3.04 (1H, dd, piperidine), 3.17 (1H, ddd, piperidine), 3.48 (1H, m, piperidine), 3.82 (1H, dd, piperidine), 4.13 (1H, m, piperidine), 4.29 (2H, q, $\underline{CH_2}CH_3$), 5.30 (1H, d, NH), 6.55 (1H, t, pyrimidine), 6.74 (2H, m, $C_6H_3$), 7.86 (1H, m, $C_6H_3$), 8.28 (2H, d, pyrimidine)

Intermediate 62: Ethyl 4-{2-(aminomethyl)pyrrolidin-1-yl}benzoate

Dioxane (220 ml) and 10 ml of acetic acid were added to the about 1 : 1 mixture (6.0 g, 21.9 mmol) of intermediate 3 with ethyl 4-{2-(azidomethyl)pyrrolidin-1-yl)benzoate to prepare a solution. To the solution was added 600 mg of 10% palladium-carbon. The mixture was vigorously stirred under a hydrogen pressure of 1 atm at room temperature for 23 hr. The insolubles were collected by filtration and were then washed twice with dioxane and twice with methanol. The filtrate and the washings were combined, and the combined solution was concentrated under the reduced pressure. The residue was purified by column chromatography on silica gel (development system: methylene chloride : methanol = 7 : 1) to give intermediate 4 (1.45 g, 27%) and the title compound (1.16 g, 21%).

Physicochemical properties of intermediate 62

(1) Color and form: Colorless solid
(2) Molecular formula: $C_{14}H_{20}N_2O_2$

(3) Mass spectrum (TSPMS): m/z 249 $(M+H)^+$
(4) $^1H$ NMR spectrum (400 MHz, $CDCl_3$) δ (ppm): 1.37 (3H, t, $CH_2CH_3$), 2.05 (4H, m, pyrrolidine), 2.67 (1H, dd, $NH_2CH_2$), 2.92 (1H, dd, $NH_2CH_2$), 3.25 (1H, m, pyrrolidine), 3.50 (1H, m, pyrrolidine), 3.81 (1H, m, pyrrolidine), 4.33 (2H, q, $CH_2CH_3$), 6.58 (2H, d, $C_6H_4$), 7.90 (2H, d, $C_6H_4$)

Intermediate 63: Ethyl 4-[2-{(pyrimidin-2-ylamino)-methyl}pyrrolidin-1-yl]benzoate

Dimethyl sulfoxide (20 ml) was added to intermediate 62 (500 mg, 2.01 mmol) to prepare a solution, and 2-bromopyrimidine (352 mg, 2.21 mmol) was added to the solution. Further, diisopropylethylamine (2.0 ml, 11.3 mmol) was added thereto, and the mixture was heated to 120°C and was stirred for 4.5 hr. The temperature of the reaction solution was returned to room temperature. Saturated brine (200 ml) was then added to the reaction solution, and the mixture was extracted three times with 150 ml of ethyl acetate. The organic layers were combined, and the combined organic layers were washed with saturated brine, were then dried over anhydrous sodium sulfate, and were concentrated under the reduced pressure. The residue was purified by column chromatography on silica gel (development system: hexane : ethyl acetate = 1 : 1) to give the title compound (569.1 mg, 87%).

Physicochemical properties of intermediate 63

(1) Color and form: Light yellow solid
(2) Molecular formula: $C_{18}H_{22}N_4O_2$
(3) Mass spectrum (EIMS): m/z 326 $(M)^+$
(4) $^1H$ NMR spectrum (400 MHz, $CDCl_3$) δ (ppm): 1.37 (3H, t, $CH_2CH_3$), 2.06 (4H, m, pyrrolidine), 3.12 (1H, ddd, $NHCH_2$), 3.25 (1H, ddd, $NHCH_2$), 3.54 (1H, ddd, pyrrolidine), 3.87 (1H, ddd, pyrrolidine), 4.12 (1H, dddd, pyrrolidine), 4.33 (2H, q, $CH_2CH_3$), 6.59 (1H, t, pyrimidine), 6.88 (2H, d, $C_6H_4$), 7.93 (2H, d, $C_6H_4$), 8.33 (2H, d, pyrimidine)

Example 89: t-Butyl (2S)-benzenesulfonylamino-3-[4-[2-{(pyrimidin-2-ylamino)methyl}pyrrolidin-1-yl]benzoylamino] propionate

Tetrahydrofuran (15 ml) and 5.0 ml of methanol were added to intermediate 63 (300 mg, 0.920 mmol) to prepare a solution, and 5.0 ml of a 1.0 mol/liter aqueous sodium hydroxide solution was added to the solution. The mixture was stirred at 40°C for 4 hr and was then concentrated under the reduced pressure. The residue was purified by column chromatography on silica gel (development system: methylene chloride : methanol = 7 : 1) to give 4-[2-{(pyrimidin-2-ylamino)methyl}pyrrolidin-1-yl]benzoic acid (119.6 mg, 44%).

Physicochemical properties of 4-[2-{(pyrimidin-2-ylamino)methyl}pyrrolidin-1-yl]benzoic acid

(1) Color and form: Colorless solid
(2) Molecular formula: $C_{16}H_{18}N_4O_2$
(3) Mass spectrum (EIMS): m/z 298 $(M)^+$
(4) $^1H$ NMR spectrum (400 MHz, DMSO-$d_6$) δ (ppm): 1.85 (1H, m, pyrrolidine), 2.02 (3H, m, pyrrolidine), 2.94 (1H, ddd, $NHCH_2$), 3.13 (1H, m, $NHCH_2$), 3.46 (1H, br t, pyrrolidine), 3.59 (1H, m, pyrrolidine), 3.94 (1H, m, pyrrolidine), 6.60 (1H, t, pyrimidine), 6.95 (2H, d, $C_6H_4$), 7.77 (2H, d, $C_6H_4$), 8.35 (2H, m, pyrimidine)

Subsequently, 3.0 ml of dimethylformamide was added to this compound (88.0 mg, 0.295 mmol) to prepare a solution, and t-butyl (2S)-N-benzenesulfonyl-2,3-diaminopropionate (97.5 mg, 0.324 mmol) was added to the solution. Further, 1-hydroxybenzotriazole (51.9 mg, 0.384 mmol), N-methylmorpholine (0.10 ml, 0.885 mmol), and 1-ethyl-3-(3-dimethylaminopropyl)carbodiimide hydrochloride (73.5 mg, 0.384 mmol) were added thereto, and the mixture was stirred at room temperature for 14 hr. Saturated brine (100 ml) and 50 ml of a saturated aqueous potassium carbonate solution were added to the reaction solution, and the mixture was extracted three times with 100 ml of ethyl acetate. The organic layers were combined, and the combined organic layers were washed twice with a mixed solution composed of 250 ml of saturated brine and 250 ml of water, were dried over anhydrous sodium sulfate, and were then concentrated under the reduced pressure. The residue was purified by column chromatography on silica gel (development system: methylene chloride : methanol = 30 : 1) to give the title compound (171 mg, 100%).

Physicochemical properties of compound prepared in Example 89

(1) Color and form: Colorless oil
(2) Molecular formula: $C_{29}H_{36}N_6O_5S$
(3) Mass spectrum (FABMS): m/z 581 $(M+H)^+$
(4) $^1H$ NMR spectrum (400 MHz, $CD_3OD$) δ (ppm): 1.24 (9H, s, t-Bu), 2.03 (4H, m, pyrrolidine), 3.10 (1H, m, $NHCH_2$), 3.21 (1H, ddd, $NHCH_2$), 3.52 (2H, m, pyrrolidine), 3.64 (1H, dd, $CONHC\underline{H}_2CH$), 3.75 (1H, dd, $CONHC\underline{H}_2CH$), 4.10 (2H, m, pyrrolidine and $CONHCH_2C\underline{H}$), 6.62 (1H, t, pyrimidine), 6.94 (2H, d, $C_6H_4$), 7.47 (2H, br t, $C_6H_5$), 7.54 (1H, m, $C_6H_5$), 7.65 (2H, d, $C_6H_4$), 7.84 (2H, m, $C_6H_5$), 8.32 (2H, m, pyrimidine)

Example 90: (2S)-Benzenesulfonylamino-3-[4-[2-{(pyrimidin-2-ylamino)methyl}pyrrolidin-1-yl]benzoylamino]propionic acid

Methylene chloride (1.5 ml) was added to the compound (96.0 mg, 0.165 mmol) prepared in Example 89 to prepare a solution. Trifluoroacetic acid (1.5 ml) was added to the solution at room temperature, and the mixture was stirred for 3 hr. The reaction solution was concentrated under the reduced pressure and was subjected to azeotropic distillation twice with toluene to give a trifluoroacetate of the title compound.

Physicochemical properties of compound prepared in Example 90

(1) Color and form: Light yellow syrup
(2) Molecular formula: $C_{25}H_{28}N_6O_5S$
(3) Mass spectrum (TSPMS): m/z 525 $(M+H)^+$

Example 91: (2S)-Benzenesulfonylamino-3-[4-[2-{(1,4,5,6-tetrahydropyrimidin-2-ylamino)methyl}pyrrolidin-1-yl]-benzoylamino]propionic acid

Dioxane (2.0 ml) and 0.2 ml of water were added to the trifluoroacetate of the crude compound (0.165 mmol) prepared in Example 90 to prepare a solution. To the solution was added 20 mg of 10% palladium-carbon. The mixture was vigorously stirred under a hydrogen pressure of 1 atm at room temperature for 22 hr. The insolubles were collected by filtration and were then washed with a mixed solution composed of 10 ml of dioxane and 1 ml of water. The filtrate and the washings were combined, and the combined solution was concentrated under the reduced pressure. The residue was purified by column chromatography on silica gel (development system: methylene chloride : ethanol : water : concentrated aqueous ammonia = 8 : 8 : 1 : 1) and was then purified by Sephadex LH-20 (development system: methanol) to give the title compound (67.2 mg, 77%).

Physicochemical properties of compound prepared in Example 91

(1) Color and form: Colorless solid
(2) Molecular formula: $C_{25}H_{32}N_6O_5S$
(3) Mass spectrum (TSPMS): m/z 529 $(M+H)^+$
(4) Specific rotation: $[\alpha]_D^{26}$ +83° (c 0.22, MeOH-conc. $NH_4OH$ (10 : 1))
(5) $^1H$ NMR spectrum (400 MHz, $CD_3OD$) δ (ppm): 1.83 (2H, quintet, tetrahydropyrimidine), 1.89 (1H, m, pyrrolidine), 2.10 (3H, m, pyrrolidine), 3.29 (7H, m, pyrrolidine, tetrahydropyrimidine and $NHCH_2$), 3.56 (2H, m, pyrrolidine and $CONHC\underline{H}_2CH$), 3.68 (2H, m, pyrrolidine and $CONHC\underline{H}_2CH$), 4.10 (1H, m, $CONHCH_2C\underline{H}$), 6.69 (2H, d, $C_6H_4$), 7.50 (2H, t, $C_6H_5$), 7.56 (1H, t, $C_6H_5$), 7.72 (2H, d, $C_6H_4$), 7.87 (2H, m, $C_6H_5$)

Pharmacological Test Example 1: $\alpha_v\beta_3$ Binding assay

Integrin $\alpha_v\beta_3$ antagonistic activity of compounds according to the present invention was measured in a vitronectin-vitronectin receptor binding assay system in accordance with the method of Kouns et al. (W. C. Kouns, D. Kirchhofer, P. Hadvary, A. Edenhofer, T. Weller, G. Pfenninger, H. R. Baumgartner, L. K. Jennings and B. Steiner, Blood,

80, 2539-2547 (1992)).

Specifically, a vitronectin receptor (protein content: 118 μg/ml) purified from the human placenta in accordance with the method of Pytela et al. (R. Pytela, M. D. Pierschbacher, S. Argraves, S. Suzuki, and E. Ruoslahti, Method in Enzymology, 144, 475-489 (1987)) was diluted 50 times with TBS (20 mM Tris-HCl, 150 mM NaCl, 1 mM $CaCl_2$, 1 mM $MgCl_2$, pH 7.4), and distributed and coated on wells (50 μl/well) of a plate (Maxisorp, Nunc, 96 well Immuno Plate). The plate was then allowed to stand at 4°C for one day, washed twice with TBS (200 μl/well), and then subjected to blocking with TBS (150 μl/well) containing 1% bovine serum albumin (SIGMA) at 4°C overnight. After washing twice with TBS (200 μl/well), 50 μl of vitronectin (CALBIOCHEM) adjusted to 0.2 μg/ml by the addition of TBS (TBS-Tween) containing 0.01% Tween-20 was mixed with 50 μl of each test compound adjusted to each concentration in wells, and a reaction was allowed to proceed at room temperature for 4 hr. After the completion of the reaction, the wells were washed three times with TBS-Tween. A solution prepared by diluting anti-vitronectin rabbit antiserum (CHEMICON) 500 times with TBS-Tween was added as a primary antibody in an amount of 50 μl/well, and a reaction was allowed to proceed at room temperature for 1.5 hr. After washing three times with 200 μl/well of TBS-Tween, a peroxidase (POD)-labeled anti-rabbit IgG antibody solution (CAPPEL) diluted 500 times with TBS-Tween was added as a secondary antibody in an amount of 50 μl/well, and a reaction was allowed to proceed at room temperature for 1.5 hr. After washing three times with TBS-Tween (200 μl/well), ABTS (2,2'-azino-bis(3-ethylbenzthiazoline-6-sulfonic acid), SIGMA) was adjusted to 1 mg/ml by the addition of a ten-fold diluted POD-buffer (ZYMED), and was added in an amount of 50 μl/well, and a reaction was allowed to proceed for 5 to 10 min. A 0.1 M citric acid buffer (pH 4.3) containing 0.05% $NaN_3$ was added in an amount of 50 μl/well to stop the reaction, followed by the measurement of the absorbance at 415 nm with a microplate reader (MTP 32, Corona Electric) (reference: 675 nm). The total binding was defined as the absorbance after a reaction using 50 μl of TBS-Tween instead of the test compound, and the non-specific binding (100% inhibition) was defined as the absorbance after a reaction using 50 μl of TBS-Tween containing 2 x $10^{-3}$ M RGDS. The inhibition was calculated by the following equation:

$$\text{Inhibition (\%)} = 100 - \frac{\text{(absorbance in the presence of test compound - non-specific binding)}}{\text{(total binding - non-specific binding)}} \times 100$$

$IC_{50}$ was determined from a primary regression line of the logarithm of each concentration of the test compound and the logarithm of (100 - inhibition)/inhibition.

As a result of the above test, all the compounds prepared in Examples 3, 6, 19, 25, 27, 30, and 44 had significant integrin $\alpha_v\beta_3$ antagonistic activity and had an $IC_{50}$ value of not more than 1.0 nM. The $IC_{50}$ value of the compound prepared in Example 6 was 0.13 nM.

Pharmacological Test Example 2: GP IIb/IIIa antagonistic activity and human platelet aggregation inhibitory activity

GP IIb/IIIa antagonistic activity was measured for compounds according to the present invention. The measurement of the GP IIb/IIIa antagonistic activity was carried out according to the method described in Pharmacological Test 2 in WO 94/21599. As a result, all the compounds prepared in Examples 2, 3, 6, 9, 19, 22, 25, 27, 30, and 44 had significant GP IIb/IIIa antagonistic activity and had an $IC_{50}$ value of not more than 1.1 nM. The $IC_{50}$ value of the compound prepared in Example 6 was 1.1 nM.

Human platelet aggregation inhibitory activity was measured for compounds according to the present invention. The measurement of the human platelet aggregation inhibitory activity was carried out according to the method described in Pharmacological Test 1 in WO 94/21599. As a result, the compound prepared in Example 25 very strongly inhibited human platelet aggregation and had an $IC_{50}$ value of 56 nM.

Pharmacological Test Example 3: Inhibitory activity against adhesion of human vascular smooth muscle cells to vitronectin

The adhesion of human vascular smooth muscle cells to immobilized human vitronectin was measured in accordance with the method of Liaw et al. (Liaw L, Almeida M, Hart CE, Schwartz SM, and Giachelli CM, Circulation Research, 74 (2), 214-224 (1994)).

A Dulbecco's phosphate buffer (Dulbecco's PBS(-), Nissui Pharmaceutical Co., Ltd.) solution of human plasma-derived vitronectin (CALBIOCHEM) adjusted to a concentration of 4 μg/ml was first added to wells (50 μl/well) of a microplate (Maxisorp, Nunc), and a reaction for immobilization was allowed to proceed at 4°C overnight. After washing twice with 150 μl of Dulbecco's phosphate buffer, a Dulbecco's phosphate buffer containing 10 mg/ml of bovine serum albumin (SIGMA) was added, followed by blocking at 37°C for one hr. The assay plate was then washed twice with 150 μl of Dulbecco's phosphate buffer.

Separately, human vascular smooth muscle cells cultivated at 37°C under 5% carbon dioxide in a medium

for vascular smooth muscle cells (Clonetics) were separated using a Dulbecco's phosphate buffer containing trypsin-EDTA (GIBCO BRL), washed with Dulbecco's phosphate buffer, and then suspended in a Dulbecco's modified Eagle's basal medium (Nissui Pharmaceutical Co., Ltd.) containing 0.1% bovine serum albumin to a concentration of 5 x $10^5$/ml.

Next, 50 µl of a Dulbecco's modified Eagle's basal medium containing 0.1% of bovine serum albumin with a medicament added thereto was added to the wells of the human vitronectin-coated assay microplate, followed by precultivation under 5% carbon dioxide at 37°C for 10 min. Thereafter, 50 µl of the medium with human vascular smooth muscle cells suspended therein was added thereto, and the plate was thoroughly stirred. A reaction was allowed to proceed under 5% carbon dioxide at 37°C for 90 min. The reaction solution containing non-adherent cells was then removed, followed by washing three times with Dulbecco's phosphate buffer. For the adhered cells, 100 µl of a Dulbecco's phosphate buffer containing 4% paraformaldehyde (Wako Pure Chemical Industries, Ltd.) was added, and immobilization was allowed to proceed at room temperature for 10 min. Next, 100 µl of a Dulbecco's phosphate buffer containing 0.5% Toluidine Blue (Croma) and 4% paraformaldehyde was added, and staining was allowed to proceed at room temperature for 5 min, followed by thorough washing with distilled water. The inside of the wells was then air-dried, and 1% aqueous sodium dodecylsulfate solution was added to perform cytolysis. The absorbance of the microplate thus obtained was measured at 595 nm. The total binding was defined as the absorbance of the well not containing the test compound, and the non-specific binding (100% inhibition) was defined as the absorbance of the well which does not contain vitronectin and has been subjected to blocking with bovine serum albumin. The inhibition was calculated by the following equation. $IC_{50}$ was determined from a primary regression line of the logarithm of each concentration of the test compound and the logarithm of (100 - inhibition)/inhibition.

$$\text{Inhibition (\%)} = 100 - \frac{\text{(absorbance in the presence of test compound - non-specific binding)}}{\text{(total binding - non-specific binding)}} \times 100$$

As a result, the compound of Example 30 had potent cell adhesion inhibitory activity, and, for this compound, the $IC_{50}$ value on the inhibitory activity against the adhesion of human vascular smooth muscle cells to vitronectin was 13 nM.

Thus, derivatives represented by formula (I), wherein a basic atomic group corresponding to group A has been attached to the 3-position of the piperidine ring, had potent integrin $\alpha_v\beta_3$ antagonistic activity. Further, as compared with corresponding derivatives wherein the basic atomic group corresponding to group A has been attached to the 4-position of the piperidine ring, these compounds according to the present invention had at least two-fold higher solubility in water and, in addition, had very high safety in a general pharmacological test using mice. Further, the present inventors have found compounds of which the balance between integrin $\alpha_v\beta_3$ antagonistic activity and integrin $\alpha_{IIb}\beta_3$ antagonistic activity has been improved by about ten times.

The compounds having potent integrin $\alpha_v\beta_3$ antagonistic activity according to the present invention are expected to be used as prophylactic or therapeutic agents for diseases in various fields, for example, acute myocardial infarction, neointima formation hypertrophy, restenosis after PTCA, unstable angina, cerebral infarction, cancers or metastasis thereof, immunological diseases, diabetic retinopathy, and osteopathy. Among others, regarding the administration of drugs through intraveneous injection, intraveneous drop infusion, instillation or the like, the compounds according to the present invention, by virtue of their high solubility in water, are expected to have excellent clinical application.

The structures of the compounds described in Examples 1 to 91 will be summarized below.

| | A | D | Z | p | q | $R^7$ | $R^8$ | Q | $R^9$ | J | $R^{10}$ | $R^{11}$ |
|---|---|---|---|---|---|---|---|---|---|---|---|---|
| 1 | 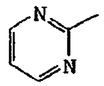 | 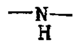 | N | 3 | 1 | m=0 | n=0 | 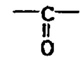 | H | $CH_2$ | $-NHSO_2Ph$ | t-Bu |
| 2 | 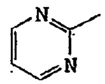 | 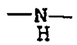 | N | 3 | 1 | m=0 | n=0 | 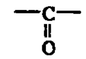 | H | $CH_2$ | $-NHSO_2Ph$ | H |
| 3 | 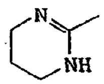 | 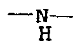 | N | 3 | 1 | m=0 | n=0 | 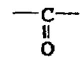 | H | $CH_2$ | $-NHSO_2Ph$ | H |
| 4 | 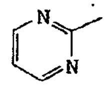 | 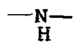 | N | 3 | 1 | m=0 | n=1(3-F) | 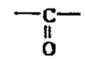 | H | $CH_2$ | $-NHSO_2Ph$ | t-Bu |
| 5 | 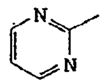 | 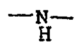 | N | 3 | 1 | m=0 | n=1(3-F) | 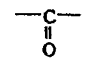 | H | $CH_2$ | $-NHSO_2Ph$ | H |
| 6 | 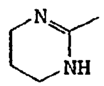 | 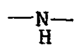 | N | 3 | 1 | m=0 | n=1(3-F) | 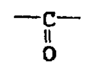 | H | $CH_2$ | $-NHSO_2Ph$ | H |
| 7 | 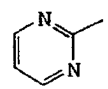 | 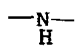 | N | 3 | 1 | m=0 | n=1(3-F) | 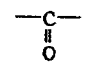 | H | $CH_2$ | $-NHSO_2Ph$ | t-Bu |
| 8 | 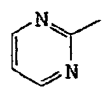 | 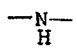 | N | 3 | 1 | m=0 | n=1(3-F) | 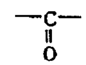 | H | $CH_2$ | $-NHSO_2Ph$ | H |
| 9 | 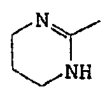 | 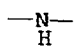 | N | 3 | 1 | m=0 | n=1(3-F) | 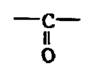 | H | $CH_2$ | $-NHSO_2Ph$ | H |
| 10 | 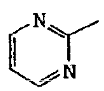 | 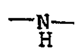 | N | 3 | 1 | m=0 | n=1(3-F) | 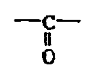 | H | $CH_2$ | $-NHCO_2Bn$ | t-Bu |

| | | | | | | | | | | | |
|---|---|---|---|---|---|---|---|---|---|---|---|
| 11 | 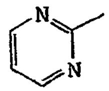 | 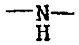 | N | 3 | 1 | m=0 | n=1(3-F) | 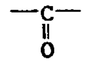 | H | $CH_2$ | $-NH_2$ | t-Bu |
| 12 | 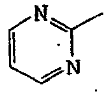 | 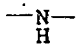 | N | 3 | 1 | m=0 | n=1(3-F) | 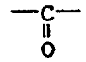 | H | $CH_2$ | $-NHSO_2Ph$*(4-MeO) | t-Bu |
| 13 | 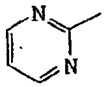 | 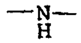 | N | 3 | 1 | m=0 | n=1(3-F) | 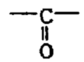 | H | $CH_2$ | $-NHSO_2Ph$*(4-MeO) | H |
| 14 | 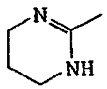 | 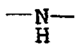 | N | 3 | 1 | m=0 | n=1(3-F) | 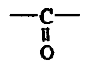 | H | $CH_2$ | $-NHSO_2Ph$*(4-MeO) | H |
| 15 | 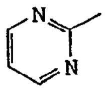 | 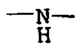 | N | 3 | 1 | m=0 | n=1(3-F) | 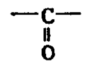 | H | $CH_2$ | $-NHSO_2Ph$*(4-OH) | H |
| 16 | 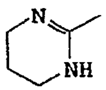 | 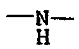 | N | 3 | 1 | m=0 | n=1(3-F) | 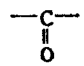 | H | $CH_2$ | $-NHSO_2Ph$*(4-OH) | H |
| 17 | 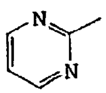 | 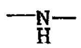 | N | 3 | 1 | m=0 | n=2(2,3-F) | 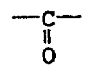 | H | $CH_2$ | $-NHSO_2Ph$ | t-Bu |
| 18 | 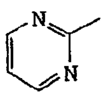 | 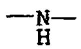 | N | 3 | 1 | m=0 | n=2(2,3-F) | 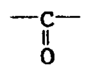 | H | $CH_2$ | $-NHSO_2Ph$ | H |
| 19 | 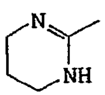 | 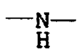 | N | 3 | 1 | m=0 | n=2(2,3-F) | 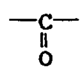 | H | $CH_2$ | $-NHSO_2Ph$ | H |
| 20 | 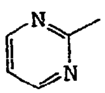 | 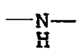 | N | 3 | 1 | m=0 | n=1(3-Cl) | 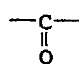 | H | $CH_2$ | $-NHSO_2Ph$ | t-Bu |

| | | | | | | | | | | | | |
|---|---|---|---|---|---|---|---|---|---|---|---|---|
| 21 | 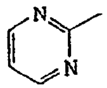 | 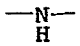 | N | 3 | 1 | m=0 | n=1(3-Cl) | 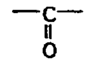 | H | $CH_2$ | $-NHSO_2Ph$ | H |
| 22 | 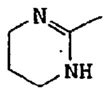 | 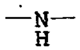 | N | 3 | 1 | m=0 | n=1(3-Cl) | 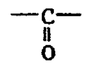 | H | $CH_2$ | $-NHSO_2Ph$ | H |
| 23 | 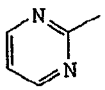 | 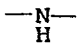 | N | 3 | 1 | m=0 | n=0 | 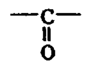 | H | $CH_2$ | $-NHSO_2Ph$ | t-Bu |
| 24 | 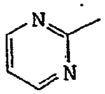 | 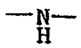 | N | 3 | 1 | m=0 | n=0 | 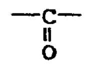 | H | $CH_2$ | $-NHSO_2Ph$ | H |
| 25 | 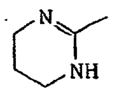 | 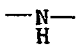 | N | 3 | 1 | m=0 | n=0 | 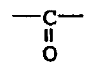 | H | $CH_2$ | $-NHSO_2Ph$ | H |
| 26 | 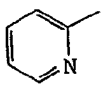 | 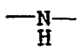 | N | 3 | 1 | m=0 | n=0 | 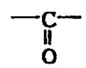 | H | $CH_2$ | $-NHSO_2Ph$ | t-Bu |
| 27 | 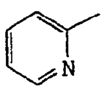 | 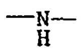 | N | 3 | 1 | m=0 | n=0 | 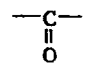 | H | $CH_2$ | $-NHSO_2Ph$ | H |
| 28 | 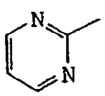 | 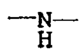 | N | 3 | 1 | m=0 | n=1(2-F) | 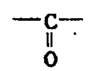 | H | $CH_2$ | $-NHSO_2Ph$ | t-Bu |
| 29 | 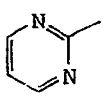 | 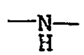 | N | 3 | 1 | m=0 | n=1(2-F) | 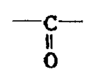 | H | $CH_2$ | $-NHSO_2Ph$ | H |
| 30 | 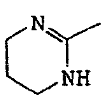 | 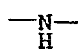 | N | 3 | 1 | m=0 | n=1(2-F) | 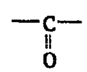 | H | $CH_2$ | $-NHSO_2Ph$ | H |

| | | | | | | | | | | | | |
|---|---|---|---|---|---|---|---|---|---|---|---|---|
| 31 | 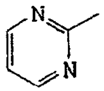 | 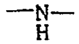 | N | 3 | 1 | m=0 | n=1(2-Cl) | 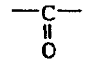 | H | $CH_2$ | $-NHSO_2Ph$ | t-Bu |
| 32 | 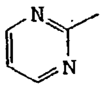 | 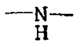 | N | 3 | 1 | m=0 | n=1(2-Cl) | 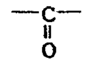 | H | $CH_2$ | $-NHSO_2Ph$ | H |
| 33 | 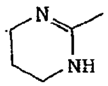 | 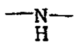 | N | 3 | 1 | m=0 | n=1(2-Cl) | 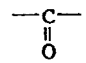 | H | $CH_2$ | $-NHSO_2Ph$ | H |
| 34 | 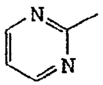 | 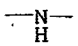 | N | 3 | 1 | m=2(4S-MeO,2R-Me) | n=0 | 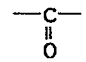 | H | $CH_2$ | $-NHSO_2Ph$ | t-Bu |
| 35 | 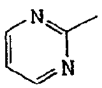 | 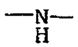 | N | 3 | 1 | m=2(4S-MeO,2R-Me) | n=0 | 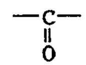 | H | $CH_2$ | $-NHSO_2Ph$ | H |
| 36 | 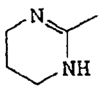 | 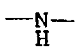 | N | 3 | 1 | m=2(4S-MeO,2R-Me) | n=0 | 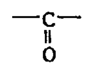 | H | $CH_2$ | $-NHSO_2Ph$ | H |
| 37 | 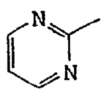 | 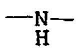 | N | 3 | 1 | m=0 | n=1(2-$CF_3$) | 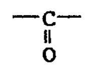 | H | $CH_2$ | $-NHSO_2Ph$ | t-Bu |
| 38 | 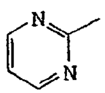 | 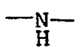 | N | 3 | 1 | m=0 | n=1(2-$CF_3$) | 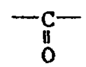 | H | $CH_2$ | $-NHSO_2Ph$ | H |
| 39 | 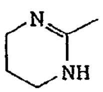 | 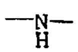 | N | 3 | 1 | m=0 | n=1(2-$CF_3$) | 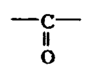 | H | $CH_2$ | $-NHSO_2Ph$ | H |
| 40 | 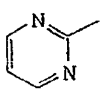 | 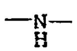 | N | 3 | 1 | m=0 | n=0 | 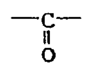 | H | $CH_2$ | $-NHCO_2Bn$ | t-Bu |

| | A | D | Z | p | q | $R^7$ | $R^8$ | Q | $R^9$ | J | $R^{10}$ | $R^{11}$ |
|---|---|---|---|---|---|---|---|---|---|---|---|---|
| 41 | 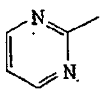 | 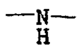 | N | 3 | 1 | m=0 | n=0 | 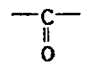 | H | $CH_2$ | $-NH_2$ | t-Bu |
| 42 | 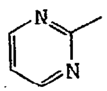 | 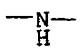 | N | 3 | 1 | m=0 | n=0 | 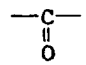 | H | $CH_2$ | $-NHSO_2Th$ | t-Bu |
| 43 | 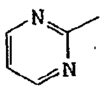 | 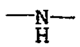 | N | 3 | 1 | m=0 | n=0 | 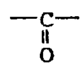 | H | $CH_2$ | $-NHSO_2Th$ | H |
| 44 | 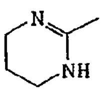 | 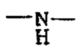 | N | 3 | 1 | m=0 | n=0 | 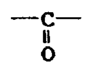 | H | $CH_2$ | $-NHSO_2Th$ | H |

| | A | D | Z | p | q | $R^7$ | $R^8$ | Q | $R^9$ | J | $R^{10}$ | $R^{11}$ |
|---|---|---|---|---|---|---|---|---|---|---|---|---|
| 51 | 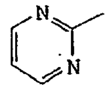 | 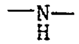 | N | 3 | 1 | m=0 | n=0 | 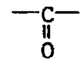 | H | $CH_2$ | $-NHSO_2Py(3)$ | t-Bu |
| 52 | 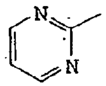 | 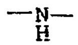 | N | 3 | 1 | m=0 | n=0 | 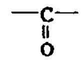 | H | $CH_2$ | $-NHSO_2Py(3)$ | H |
| 53 | 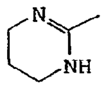 | 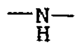 | N | 3 | 1 | m=0 | n=0 | 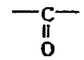 | H | $CH_2$ | $-NHSO_2Py(3)$ | H |
| 54 | 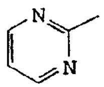 | 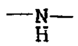 | N | 3 | 1 | m=0 | n=0 | 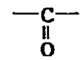 | H | $CH_2$ | $-NHSO_2nBu$ | t-Bu |
| 55 | 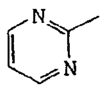 | 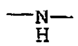 | N | 3 | 1 | m=0 | n=0 | 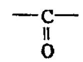 | H | $CH_2$ | $-NHSO_2nBu$ | H |
| 56 | 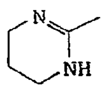 | 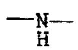 | N | 3 | 1 | m=0 | n=0 | 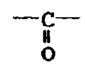 | H | $CH_2$ | $-NHSO_2nBu$ | H |
| 57 | 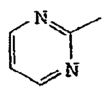 | 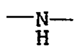 | N | 3 | 1 | m=0 | n=0 | 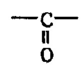 | H | $CH_2$ | -NHCOPh | t-Bu |
| 58 | 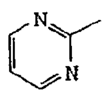 | 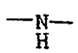 | N | 3 | 1 | m=0 | n=0 | 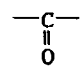 | H | $CH_2$ | -NHCOPh | H |
| 59 | 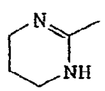 | 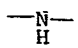 | N | 3 | 1 | m=0 | n=0 | 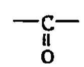 | H | $CH_2$ | -NHCOPh | H |
| 60 | 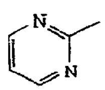 | 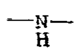 | N | 3 | 1 | m=0 | n=0 | 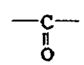 | H | $CH_2$ | $-NHSO_2(CH_2)_3OAc$ | t-Bu |

| | A | D | Z | p | q | $R^7$ | $R^8$ | Q | $R^9$ | J | $R^{10}$ | $R^{11}$ |
|---|---|---|---|---|---|---|---|---|---|---|---|---|
| 61 | 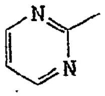 | 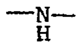 | N | 3 | 1 | m=0 | n=0 | 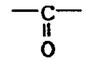 | H | $CH_2$ | $-NHSO_2(CH_2)_3OAc$ | H |
| 62 | 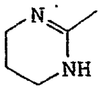 | 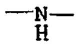 | N | 3 | 1 | m=0 | n=0 | 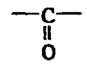 | H | $CH_2$ | $-NHSO_2(CH_2)_3OAc$ | H |
| 63 | 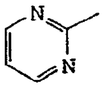 | 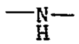 | N | 3 | 1 | m=0 | n=0 | 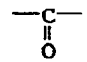 | H | $CH_2$ | $-NHSO_2(CH_2)_3OH$ | t-Bu |
| 64 | 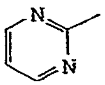 | 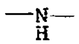 | N | 3 | 1 | m=0 | n=0 | 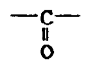 | H | $CH_2$ | $-NHSO_2(CH_2)_3OH$ | H |
| 65 | 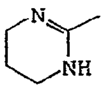 | 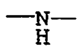 | N | 3 | 1 | m=0 | n=0 | 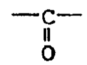 | H | $CH_2$ | $-NHSO_2(CH_2)_3OH$ | H |
| 66 | 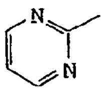 | 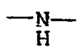 | N | 3 | 1 | m=0 | n=0 | 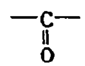 | H | $CH_2$ | $-NHSO_2Mo$ | t-Bu |
| 69 | 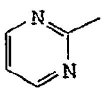 | 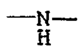 | N | 3 | 1 | m=0 | n=0 | 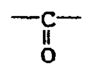 | H | $CH_2$ | -OH | $-CHPh_2$ |
| 70 | 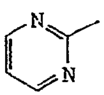 | 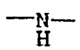 | N | 3 | 1 | m=0 | n=0 | 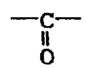 | H | $CH_2$ | -OH | H |

| | A | D | Z | p | q | $R^7$ | $R^8$ | Q | $R^9$ | J | $R^{10}$ | $R^{11}$ |
|---|---|---|---|---|---|---|---|---|---|---|---|---|
| 71 | 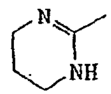 | 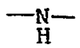 | N | 3 | 1 | m=0 | n=0 | 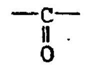 | H | $CH_2$ | -OH | H |
| 72 | 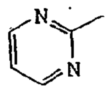 | 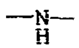 | N | 4 | 1 | m=0 | n=0 | 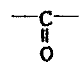 | H | $CH_2$ | $-NHSO_2Ph$ | t-Bu |
| 73 | 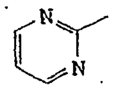 | 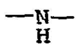 | N | 4 | 1 | m=0 | n=0 | 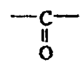 | H | $CH_2$ | $-NHSO_2Ph$ | H |
| 74 | 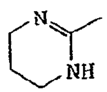 | 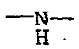 | N | 4 | 1 | m=0 | n=0 | 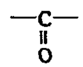 | H | $CH_2$ | $-NHSO_2Ph$ | H |
| 75 | 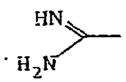 | 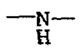 | N | 3 | 1 | m=0 | n=0 | 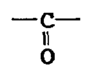 | H | $CH_2$ | $-NHSO_2Ph$ | t-Bu |
| 76 | 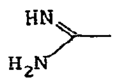 | 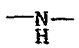 | N | 3 | 1 | m=0 | n=0 | 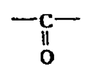 | H | $CH_2$ | $-NHSO_2Ph$ | H |
| 77 | 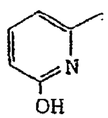 | 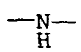 | N | 3 | 1 | m=0 | n=0 | 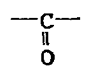 | H | $CH_2$ | $-NHSO_2Ph$ | t-Bu |
| 78 | 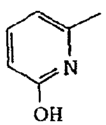 | 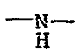 | N | 3 | 1 | m=0 | n=0 | 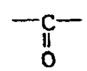 | H | $CH_2$ | $-NHSO_2Ph$ | H |

| A | D | Z | p | q | $R^7$ | $R^8$ | Q | $R^9$ | J | $R^{10}$ | $R^{11}$ |
|---|---|---|---|---|---|---|---|---|---|---|---|
| 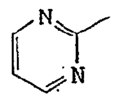 | 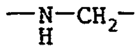 | N | 3 | 0 | m=0 | n=0 | 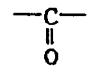 | H | $CH_2$ | $-NHSO_2Ph$ | t-Bu |
| 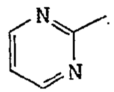 | 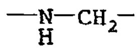 | N | 3 | 0 | m=0 | n=0 | 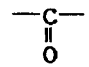 | H | $CH_2$ | $-NHSO_2Ph$ | H |
| 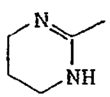 | 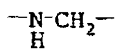 | N | 3 | 0 | m=0 | n=0 | 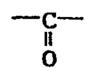 | H | $CH_2$ | $-NHSO_2Ph$ | H |

Me: methyl, Bu: butyl, Ac: acetyl, MeO: methoxy, Ph: phenyl, Ph*: substituted phenyl, Bn: benzyl, Th: thiophen-2-yl, Py: pyridine-3-yl, Mo: morpholin-4-yl

## Claims

**1.** A compound represented by formula (I) or a pharmaceutically acceptable salt or solvate thereof:

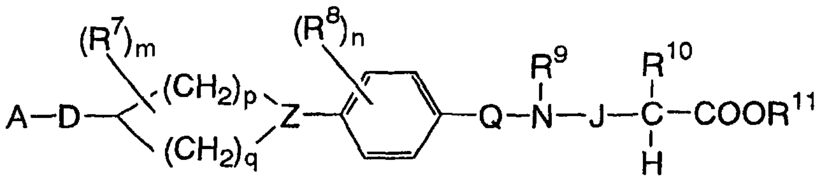

(I)

wherein

A represents a saturated or unsaturated five- to seven-membered heterocyclic group containing at least one nitrogen atom, which is optionally condensed with another saturated or unsaturated five- to seven-membered carbocyclic ring or heterocyclic ring to form a bicyclic group, wherein the heterocyclic group and the bicyclic group are optionally substituted by $C_{1-6}$ alkyl, amino, $C_{2-6}$ alkenyl, hydroxyl, a halogen atom, $C_{1-6}$ alkoxy, $C_{1-6}$ alkoxycarbonyl, or aralkyl and the $C_{1-6}$ alkyl, amino, $C_{2-6}$ alkenyl, hydroxyl, $C_{1-6}$ alkoxy, $C_{1-6}$ alkoxycarbonyl, and aralkyl groups are optionally substituted by $C_{1-6}$ alkyl, $C_{1-6}$ alkoxy, a halogen atom, amino, or hydroxyl, or a group represented by formula

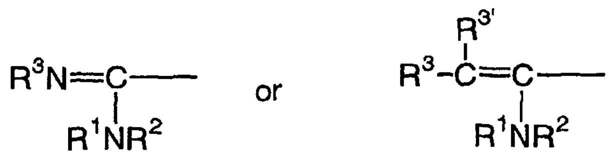

wherein $R^1$, $R^2$, $R^3$, and $R^{3'}$, which may be the same or different, represent a hydrogen atom, $C_{1-6}$ alkyl, $C_{2-6}$ alkenyl, $C_{2-6}$ alkynyl, aralkyl, or nitrile, or $R^1$ and $R^2$ may together form group $-(CH_2)i-$, wherein i represents 4 or 5, or group $-(CH_2)_2-O-(CH_2)_2-$, wherein the $C_{1-6}$ alkyl, $C_{2-6}$ alkenyl, $C_{2-6}$ alkynyl, and aralkyl groups are optionally substituted by a halogen atom, $C_{1-6}$ alkoxy, amino, or hydroxyl;

D represents $>NR^4$ wherein $R^4$ represents a hydrogen atom or $C_{1-6}$ alkyl and this alkyl group is optionally substituted by phenyl optionally substituted by $C_{1-6}$ alkoxy; $>CR^5R^6$ wherein $R^5$ and $R^6$ each independently represent a hydrogen atom or $C_{1-6}$ alkyl and this alkyl group is optionally substituted by phenyl optionally substituted by $C_{1-6}$ alkoxy; -O-; -S-; or $-NR^4-CR^5R^6-$ wherein $R^4$, $R^5$, and $R^6$ are as defined above;

Z represents CH or N;

$R^7$ represents $C_{1-6}$ alkyl, $C_{1-6}$ alkoxy, a halogen atom, amino, nitro, hydroxyl, or an oxygen atom, wherein the $C_{1-6}$ alkyl and $C_{1-6}$ alkoxy groups are optionally substituted by a halogen atom, $C_{1-6}$ alkoxy, amino, or hydroxyl;

$R^8$ represents $C_{1-6}$ alkyl, $C_{1-6}$ alkoxy, a halogen atom, amino, nitro, or hydroxyl, wherein the $C_{1-6}$ alkyl and $C_{1-6}$ alkoxy groups are optionally substituted by a halogen atom, $C_{1-6}$ alkoxy, amino, or hydroxyl;

Q represents $>C=O$, $>CHR^{13}$, or $>CHOR^{13}$ wherein $R^{13}$ represents a hydrogen atom or $C_{1-6}$ alkyl;

$R^9$ represents a hydrogen atom, $C_{1-6}$ alkyl, $C_{2-6}$ alkenyl, $C_{2-6}$ alkynyl, or aralkyl wherein the $C_{1-6}$ alkyl, $C_{2-6}$ alkenyl, $C_{2-6}$ alkynyl, and aralkyl groups are optionally substituted by a halogen atom, $C_{1-6}$ alkoxy, amino, or hydroxyl;

J represents a bond or an alkylene chain having 1 to 3 carbon atoms, wherein one or more hydrogen atoms on the alkylene chain are optionally substituted by the same or different substituent selected from $C_{1-6}$ alkyl, $C_{2-6}$ alkenyl, $C_{2-5}$ alkynyl, aralkyl, hydroxyl, or amino, the $C_{1-6}$ alkyl, $C_{2-6}$ alkenyl, $C_{2-6}$ alkynyl, and aralkyl groups are further optionally substituted by a halogen atom, $C_{1-6}$ alkoxy, amino, or hydroxyl, and the amino group is optionally substituted by $C_{1-6}$ alkyl; $C_{1-6}$ alkoxycarbonyl; benzenesulfonyl of which the phenyl portion is optionally substituted by $C_{1-6}$ alkyl; or benzyloxycarbonyl of which the phenyl portion is optionally substituted by $C_{1-6}$ alkyl;

$R^{10}$ represents a hydrogen atom, hydroxyl, $C_{1-6}$ alkyl, $C_{2-6}$ alkenyl, $C_{2-6}$ alkynyl, aralkyl, or amino, wherein the $C_{1-6}$ alkyl, $C_{2-6}$ alkenyl, $C_{2-6}$ alkynyl, and aralkyl groups are optionally substituted by a halogen atom, $C_{1-6}$ alkoxy, amino, or hydroxyl, the hydroxyl group is optionally substituted by $C_{1-6}$ alkyl, and the amino group is optionally substituted by carboxyl; sulfonyl; $C_{1-6}$ alkyl; $C_{1-6}$ alkylcarbonyl; $C_{1-6}$ alkoxycarbonyl; $C_{1-6}$ alkylsulfonyl; $-C(=O)-O-(CH_2)u-R^{14}$ wherein u is an integer of 0 to 4 and $R^{14}$ represents $-OR^{15}$ (wherein $R^{15}$ represents

a hydrogen atom or $C_{1-4}$ alkylcarbonyl) or a saturated or unsaturated five- to seven-membered carbocyclic or heterocyclic group and the carbocyclic group and the heterocyclic group are optionally substituted by $C_{1-6}$ alkyl, $C_{1-6}$ alkoxy, phenyl optionally condensed with the carbocyclic group or the heterocyclic group, carboxyl, hydroxyl, nitro, amino, $C_{1-6}$ alkylamino, or a halogen atom; -C(=O)-$R^{14}$ wherein $R^{14}$ is as defined above; or -$S(=O)_2$-$(CH_2)$v-$R^{14}$ wherein v is an integer of 0 to 4 and $R^{14}$ is as defined above;
$R^{11}$ represents a hydrogen atom or $C_{1-6}$ alkyl wherein the $C_{1-6}$ alkyl group is optionally substituted by one or two phenyl groups;
m is an integer of 0 to 5;
n is an integer of 0 to 4;
p is 3 or 4; and
q is an integer of 0 to 3.

2. The compound according to claim 1, wherein Z represents N.

3. The compound according to claim 1, wherein Z represents CH.

4. The compound according to any one of claims 1 to 3, wherein p is 3 to 4 and q is 0 or 1.

5. The compound according to any one of claims 1 to 4, wherein A represents a group of formula

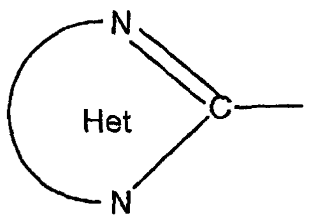
or
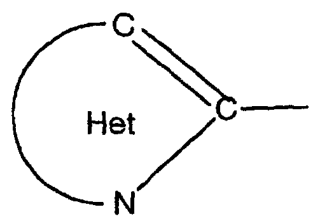

wherein
Het represents a saturated or unsaturated five- to seven-membered heterocyclic group containing at least one nitrogen atom, which is optionally condensed with another saturated or unsaturated five- to seven-membered carbocyclic ring or heterocyclic ring to form a bicyclic group, wherein the heterocyclic group and the bicyclic group are optionally substituted by $C_{1-6}$ alkyl, amino, $C_{2-6}$ alkenyl, hydroxyl, a halogen atom, $C_{1-6}$ alkoxy, $C_{1-6}$ alkoxycarbonyl, or aralkyl and the $C_{1-6}$ alkyl, amino, $C_{2-6}$ alkenyl, hydroxyl, $C_{1-6}$ alkoxy, $C_{1-6}$ alkoxycarbonyl, and aralkyl groups are optionally substituted by $C_{1-6}$ alkyl, $C_{1-6}$ alkoxy, a halogen atom, amino, or hydroxyl.

6. The compound according to any one of claims 1 to 4, wherein A represents a group of formula

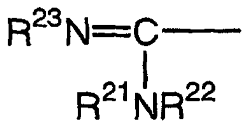
or
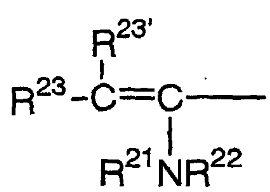

wherein

$R^{21}$, $R^{22}$, $R^{23}$, and $R^{23'}$, which may be the same or different, represent a hydrogen atom, $C_{1-6}$ alkyl, $C_{1-6}$ alkoxy, hydroxyl, $C_{1-6}$ alkoxycarbonyl, $C_{2-6}$ alkenyl, or aralkyl and the $C_{1-6}$ alkyl, $C_{1-6}$ alkoxy, hydroxyl, $C_{1-6}$ alkoxycarbonyl, $C_{2-6}$ alkenyl, and aralkyl groups are optionally substituted by a halogen atom, $C_{1-6}$ alkoxy, amino, $C_{1-6}$ alkyl, or hydroxyl, or
$R^{21}$ and $R^{23}$ may together form

group $-(CH_2)_4-$,
group $-(CH_2)_3-$,

group $-CHR_{24}CH_2CH_2-$ wherein $R^{24}$ represents $C_{1-6}$ alkyl, amino, or hydroxyl and the amino and hydroxyl groups are optionally substituted by $C_{1-6}$ alkyl, $C_{1-6}$ alkoxycarbonyl, aralkyl, or aralkyloxycarbonyl,
group $-CH_2CHR^{24}CH_2-$ wherein $R^{24}$ is as defined above,
group $-CH_2CH_2-$,
group $-CHR_{24}CH_2-$ wherein $R^{24}$ is as defined above,
group $-CR^{25}=CR^{26}-$ wherein $R^{25}$ and $R^{26}$, which may be the same or different, represent a hydrogen atom or $C_{1-6}$ alkyl, or $R^{25}$ and $R^{26}$ may together form -CH=CH-CH=CH-, $-CR^{24}=CH-CH=CH-$ wherein $R^{24}$ is as defined above, $-CH=CR^{24}$ -CH=CH- wherein $R^{24}$ is as defined above, -N=CH-CH=CH-, or -CH=N-CH=CH-, or

$R^{21}$ and $R^{23}$ may together form

=CH-CH=CH-,
=CH-CH=N-, or
=CH-N=CH-, and

$R^{22}$ may represent a single bond between $R^{21}$ and the nitrogen atom attached to $R^{21}$.

**7.** The compound according to any one of claims 1 to 6, wherein D represents >NH, $>CH_2$, or $-NH-CH_2-$.

**8.** The compound according to any one of claims 1 to 7, wherein Q represents >C=O or $>CH_2$.

**9.** The compound according to any one of claims 1 to 8, wherein m and n are each an integer of 0 to 2.

**10.** The compound according to claim 1, wherein A represents a group of formula

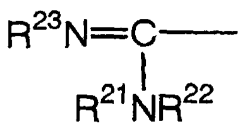

or

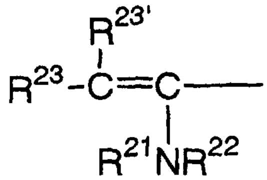

wherein

$R^{21}$, $R^{22}$, $R^{23}$, and $R^{23'}$ are as defined in claim 6;
D represents >NH, or $-NH-CH_2-$;
Z represents N;
Q represents >C=O or $>CH_2$;
$R^9$ represents a hydrogen atom, $C_{1-6}$ alkyl or aralkyl wherein the $C_{1-6}$ alkyl and aralkyl groups are optionally substituted by a halogen atom, $C_{1-6}$ alkoxy, amino, or hydroxyl;
J represents a methylene chain optionally substituted by $C_{2-6}$ alkynyl;
$R^{10}$ represents a hydrogen atom, hydroxyl, or amino, wherein the hydroxyl group is optionally substituted by $C_{1-6}$ alkyl and the amino group is optionally substituted by $C_{1-6}$ alkyl; acetyl; $C_{1-6}$ alkylcarbonyl; $C_{1-6}$ alkylsulfonyl; benzoyl or benzyloxycarbonyl of which the phenyl portion is optionally substituted by $C_{1-6}$ alkyl, $C_{1-6}$ alkoxy, carboxyl, hydroxyl, nitro, amino, or a halogen atom; or $-S(=O)_2-(CH_2)v-R^{14}$ wherein v is an integer of 0 to 4 and $R^{14}$ represents -OH, -OAc (wherein Ac represents acetyl), phenyl optionally substituted by $C_{1-6}$ alkyl, $C_{1-6}$ alkoxy, carboxyl, hydroxyl, nitro, amino, or a halogen atom, pyridyl, thiophenyl, or morpholyl;
m and n are each an integer of 0 to 2;
p is 3 or 4; and
q is 0 or 1.

**11.** The compound according to claim 1, which is selected from:

1. t-butyl (2S)-benzenesulfonylamino-3-[4-{3-(pyrimidin-2-ylamino)piperidin-1-yl}benzoylamino]-propionate;
2. (2S)-benzenesulfonylamino-3-[4-{3-(pyrimidin-2-ylamino)piperidin-1-yl}benzoylamino]propionic acid;
3. (2S)-benzenesulfonylamino-3-[4-{3-(1,4,5,6-tetrahydropyrimidin-2-ylamino)piperidin-1-yl}benzoylamino]propionic acid;
4. t-butyl (2S)-benzenesulfonylamino-3-[3-fluoro-4 -{(3S)-(pyrimidin-2-ylamino)piperidin-1-yl}benzoylamino] propionate;
5. (2S)-benzenesulfonylamino-3-[3-fluoro-4-{(3S)-(pyrimidin-2-ylamino)piperidin-1-yl}benzoylamino]-propionic acid;
6. (2S)-benzenesulfonylamino-3-[3-fluoro-4-{(3S)-(1,4,5,6-tetrahydropyrimidin-2-ylamino)piperidin-1-yl}-benzoylamino]propionic acid;
7. t-butyl (2S)-benzenesulfonylamino-3-[3-fluoro-4 -{(3R)-(pyrimidin-2-ylamino)piperidin-1-yl}benzoylamino] propionate;
8. (2S)-benzenesulfonylamino-3-[3-fluoro-4-{(3R)-(pyrimidin-2-ylamino)piperidin-1-yl}benzoylamino]-propionic acid;
9. (2S)-benzenesulfonylamino-3-[3-fluoro-4-{(3R)-(1,4,5,6-tetrahydropyrimidin-2-ylamino)piperidin-1-yl}-benzoylamino]propionic acid;
10. t-butyl (2S)-(benzyloxycarbonyl)amino-3-[3-fluoro-4-{(3S)-(pyrimidin-2-ylamino)piperidin-1-yl}-benzoylamino]propionate;
11. t-butyl (2S)-amino-3-[3-fluoro-4-{(3S)-(pyrimidin-2-ylamino)piperidin-1-yl}benzoylamino]-propionate;
12. t-butyl 3-[3-fluoro-4-{(3S)-(pyrimidin-2-ylamino)piperidin-1-yl}benzoylamino]-(2S)-{(4-methoxybenzenesulfonyl)amino}propionate;
13. 3-[3-fluoro-4-{(3S)-(pyrimidin-2-ylamino)-piperidin-1-yl}benzoylamino]-(2S)-{(4-methoxybenzenesulfonyl) amino}propionic acid;
14. 3-[3-fluoro-4-{(3S)-(1,4,5,6-tetrahydropyrimidin-2-ylamino)piperidin-1-yl}benzoylamino]-(2S)-{(4-methoxybenzenesulfonyl)amino}propionic acid;
15. 3-[3-fluoro-4-{(3S)-(pyrimidin-2-ylamino)-piperidin-1-yl}benzoylamino]-(2S)-{(4-hydroxybenzenesulfonyl) amino}propionic acid;
16. 3-[3-fluoro-4-{(3S)-(1,4,5,6-tetrahydropyrimidin-2-ylamino)piperidin-1-yl}benzoylamino]-(2S)-{(4-hydroxybenzenesulfonyl)amino}propionic acid;
17. t-butyl (2S)-benzenesulfonylamino-3-[2,3-difluoro-4-{(3S)-(pyrimidin-2-ylamino)piperidin-1-yl}-benzoylamino]propionate;
18. (2S)-benzenesulfonylamino-3-[2,3-difluoro-4-{(3S)-(pyrimidin-2-ylamino)piperidin-1-yl}benzoylamino]-propionic acid;
19. (2S)-benzenesulfonylamino-3-[2,3-difluoro-4-{(3S)-(1,4,5,6-tetrahydropyrimidin-2-ylamino)piperidin-1 -yl} benzoylamino]propionic acid;
20. t-butyl (2S)-benzenesulfonylamino-3-[3-chloro-4-{(3S)-(pyrimidin-2-ylamino)piperidin-1-yl}benzoylamino] propionate;
21. (2S)-benzenesulfonylamino-3-[3-chloro-4-{(3S)-(pyrimidin-2-ylamino)piperidin-1-yl}benzoylamino]-propionic acid;
22. (2S)-benzenesulfonylamino-3-[3-chloro-4-{(3S)-(1,4,5,6-tetrahydropyrimidin-2-ylamino)piperidin-1-yl}-benzoylamino]propionic acid;
23. t-butyl (2S)-benzenesulfonylamino-3-[4-{(3S)-(pyrimidin-2-ylamino)piperidin-1-yl}benzoylamino]-propionate;
24. (2S)-benzenesulfonylamino-3-[4-{(3S)-(pyrimidin-2-ylamino)piperidin-1-yl}benzoylamino]-propionic acid;
25. (2S)-benzenesulfonylamino-3-[4-{(3S)-(1,4,5,6-tetrahydropyrimidin-2-ylamino)piperidin-1-yl}benzoylamino]propionic acid;
26. t-butyl (2S)-benzenesulfonylamino-3-[4-{(3S)-(pyridin-2-ylamino)piperidin-1-yl}benzoylamino]-propionate;
27. (2S)-benzenesulfonylamino-3-[4-{(3S)-(pyridin-2-ylamino)piperidin-1-yl}benzoylamino]-propionic acid;
28. t-butyl (2S)-benzenesulfonylamino-3-[2-fluoro-4-{(3S)-(pyrimidin-2-ylamino)piperidin-1-yl}benzoylamino] propionate;
29. (2S)-benzenesulfonylamino-3-[2-fluoro-4-{(3S)-(pyrimidin-2-ylamino)piperidin-1-yl}benzoylamino]-propionic acid;
30. (2S)-benzenesulfonylamino-3-[2-fluoro-4-{(3S)-(1,4,5,6-tetrahydropyrimidin-2-ylamino)piperidin-1-yl}-benzoylamino]propionic acid;
31. t-butyl (2S)-benzenesulfonylamino-3-[2-chloro-4-{(3S)-(pyrimidin-2-ylamino)piperidin-1-yl}benzoylamino] propionate;
32. (2S)-benzenesulfonylamino-3-[2-chloro-4-{(3S)-(pyrimidin-2-ylamino)piperidin-1-yl}benzoylamino]-propionic acid;
33. (2S)-benzenesultonylamino-3-[2-chloro-4-{(3S)-(1,4,5,6-tetrahydropyrimidin-2-ylamino)piperidin-1-yl}-benzoylamino]propionic acid;

34. t-butyl (2S)-benzenesulfonylamino-3-[4-{(4S)-methoxy-(2R)-methyl-(3R)-(pyrimidin-2-ylamino) piperidin1-yl}benzoylamino]propionate;
35. (2S)-benzenesulfonylamino-3-[4-{(4S)-methoxy-(2R)-methyl-(3R)-(pyrimidin-2-ylamino)piperidin-1-yl}-benzoylamino]propionic acid;
36. (2S)-benzenesulfonylamino-3-[4-{(4S)-methoxy-(2R)-methyl-(3R)-(1,4,5,6-tetrahydropyrimidin-2-ylamino)piperidin-1-yl}benzoylamino]propionic acid;
37. t-butyl (2S)-benzenesulfonylamino-3-[4-{(3S)-(pyrimidin-2-ylamino)piperidin-1-yl}-2-trifluoromethyl-benzoylamino]propionate;
38. (2S)-benzenesulfonylamino-3-[4-{(3S)-(pyrimidin-2-ylamino)piperidin-1-yl}-2-trifluoromethylbenzoylamino]propionic acid;
39. (2S)-benzenesulfonylamino-3-[4-{(3S)-(1,4,5,6-tetrahydropyrimidin-2-ylamino)piperidin-1-yl}-2-trifluoromethylbenzoylamino]propionic acid;
40. t-butyl (2S)-(benzyloxycarbonyl)amino-3-[4-{(3S)-(pyrimidin-2-ylamino)piperidin-1-yl}benzoylamino]-propionate;
41. t-butyl (2S)-amino-3-[4-{(3S)-(pyrimidin-2-yl-amino)piperidin-1-yl}benzoylamino]propionate;
42. t-butyl 3-[4-{(3S)-(pyrimidin-2-ylamino)-piperidin-1-yl}benzoylamino]-(2S)-{(thiophene-2-sulfonyl)amino} propionate;
43. 3-[4-{(3S)-(pyrimidin-2-ylamino)piperidin-1-yl}benzoylamino]-(2S)-{(thiophene-2-sulfonyl)amino}-propionic acid;
44. 3-[4-{(3S)-(1,4,5,6-tetrahydropyrimidin-2-ylamino)piperidin-1-yl}benzoylamino]-(2S)-{(thiophene-2-sulfonyl)amino}propionic acid;
51. t-butyl (2S)-(pyridine-3-sulfonyl)amino-3-[4-{(3S)-(pyrimidin-2-ylamino)piperidin-1-yl}benzoylamino]-propionate;
52. (2S)-(pyridine-3-sulfonyl)amino-3-[4-{(3S)-(pyrimidin-2-ylamino)piperidin-1-yl}benzoylamino]-propionic acid;
53. (2S)-(pyridine-3-sulfonyl)amino-3-[4-{(3S)-(1,4,5,6-tetrahydropyrimidin-2-ylamino)piperidin-1-yl}-benzoylamino]propionic acid;
54. t-butyl (2S)-(butane-1-sulfonyl)amino-3-[4-{(3S)-(pyrimidin-2-ylamino)piperidin-1-yl}benzoylamino]-propionate;
55. (2S)-(butane-1-sulfonyl)amino-3-[4-{(3S)-(pyrimidin-2-ylamino)piperidin-1-yl}benzoylamino]-propionic acid;
56. (2S)-(butane-1-sulfonyl)amino-3-[4-{(3S)-(1,4, 5,6-tetrahydropyrimidin-2-ylamino)piperidin-1-yl}-benzoylamino]propionic acid;
57. t-butyl (2S)-benzoylamino-3-[4-{(3S)-(pyrimidin-2-ylamino)piperidin-1-yl}benzoylamino]-propionate;
58. (2S)-benzoylamino-3-[4-{(3S)-(pyrimidin-2-ylamino)piperidin-1-yl}benzoylamino]propionic acid;
59. (2S)-benzoylamino-3-[4-{(3S)-(1,4,5,6-tetrahydropyrimidin-2-ylamino)piperidin-1-yl}benzoylamino]-propionic acid;
60. t-butyl (2S)-(3-acetoxypropane-1-sulfonyl)-amino-3-[4-{(3S)-(pyrimidin-2-ylamino)piperidin-1-yl}-benzoylamino]propionate;
61. (2S)-(3-acetoxypropane-1-sulfonyl)amino-3-[4-{(3S)-(pyrimidin-2-ylamino)piperidin-1-yl}benzoylamino]-propionic acid;
62. (2S)-(3-acetoxypropane-1-sulfonyl)amino-3-[4-{(3S)-(1,4,5,6-tetrahydropyrimidin-2-ylamino)piperidin-1 -yl}benzoylamino]propionic acid;
63. t-butyl (2S)-(3-hydroxypropane-1-sulfonyl)-amino-3-[4-{(3S)-(pyrimidin-2-ylamino)piperidin-1-yl}-benzoylamino]propionate;
64. (2S)-(3-hydroxypropane-1-sulfonyl)amino-3-[4-{(3S)-(pyrimidin-2-ylamino)piperidin-1-yl}benzoylamino]-propionic acid;
65. (2S)-(3-hydroxypropane-1-sulfonyl)amino-3-[4-{(3S)-(1,4,5,6-tetrahydropyrimidin-2-ylamino)piperidin-1 -yl}benzoylamino]propionic acid;
66. t-butyl (2S)-(morpholine-4-sulfonyl)amino-3-[4 -{(3S)-(pyrimidin-2-ylamino)piperidin-1-yl}benzoylamino] propionate;
69. benzhydryl (2S)-hydroxy-3-[4-{(3S)-(pyrimidin-2-ylamino)piperidin-1-yl}benzoylamino]propionate;
70. (2S)-hydroxy-3-[4-{(3S)-(pyrimidin-2-ylamino)-piperidin-1-yl}benzoylamino]propionic acid;
71. (2S)-hydroxy-3-[4-{(3S)-(1,4,5,6-tetrahydropyrimidin-2-ylamino)piperidin-1-yl}benzoylamino]-propionic acid;
72. t-butyl (2S)-benzenesulfonylamino-3-[4-{(3S)-(pyrimidin-2-ylamino)azepan-1-yl}benzoylamino]-propionate;
73. (2S)-benzenesulfonylamino-3-[4-{(3S)-(pyrimidin-2-ylamino)azepan-1-yl}benzoylamino]-propionic acid;

74. (2S)-benzenesulfonylamino-3-(4-{(3S)-(1,4,5,6-tetrahydropyrimidin-2-ylamino)azepan-1-yl}benzoylamino]-propionic acid;
75. t-butyl (2S)-benzenesulfonylamino-3-[4-{(3S)-guanidinopiperidin-1-yl}benzoylamino]propionate;
76. (2S)-benzenesulfonylamino-3-[4-{(3S)-guanidinopiperidin-1-yl}benzoylamino]propionic acid;
77. t-butyl (2S)-benzenesulfonylamino-3-[4-{(3S)-(6-hydroxypyridin-2-ylamino)piperidin-1-yl}benzoylamino] propionate;
78. (2S)-benzenesulfonylamino-3-[4-{(3S)-(6-hydroxypyridin-2-ylamino)piperidin-1-yl}benzoylamino]-propionic acid;
89. t-butyl (2S)-benzenesulfonylamino-3-[4-[2-{(pyrimidin-2-ylamino)methyl}pyrrolidin-1-yl]benzoylamino] propionate;
90. (2S)-benzenesulfonylamino-3-[4-[2-{(pyrimidin-2-ylamino)methyl}pyrrolidin-1-yl]benzoylamino]propionic acid; and
91. (2S)-benzenesulfonylamino-3-[4-[2-{(1,4,5,6-tetrahydropyrimidin-2-ylamino)methyl}pyrrolidin-1-yl]-benzoylamino]propionic acid.

**12.** A pharmaceutical composition comprising as active ingredient the compound according to any one of claims 1 to 11 or a pharmaceutically acceptable salt or solvate thereof.

**13.** The pharmaceutical composition according to claim 12, for use in the treatment of integrin $\alpha_v\beta_3$-mediated diseases.

**14.** The pharmaceutical composition according to claim 12, for use in the treatment of diseases where the inhibition of cell adhesion is therapeutically effective.

**15.** The pharmaceutical composition according to claim 12, for use in the treatment of diseases where GP IIb/IIIa antagonistic activity and/or platelet aggregation inhibitory activity are therapeutically effective.

**16.** The pharmaceutical composition according to claim 12, for use in the treatment of a disease selected from the group consisting of cardiovascular diseases, angiogenesis-related diseases, cerebrovascular diseases, cancers and metastasis thereof, immunological diseases, and osteopathy.

**17.** The pharmaceutical composition according to claim 16, wherein the cardiovascular disease is selected from acute myocardial infarction, neointima formation hypertrophy, restenosis after PTCA/stent operation, unstable angina, acute coronary syndrome, angina pectoris after PTCA/stent operation, and arterial sclerosis, particularly atherosclerosis, the angiogenesis-related disease is selected from diabetic retinopathy, diabetic vascular complication, and vascular grafting, the cerebrovascular disease is cerebral infarction, the cancer and metastasis thereof are solid tumors and metastasis thereof, the immunological disease is arthritis, particularly rheumatic arthritis, and the osteopathy is selected from osteoporosis, hypercalcemia, periodontitis, hyperparathyroidism, periarticular sore, and Paget's diseases.

**18.** The pharmaceutical composition according to claim 12, for use in the treatment of platelet thrombosis or thromboembolism, the improvement of peripheral circulating blood stream, the inhibition of blood clotting during extracorporeal circulation, or the treatment of thrombotic thrombocytopenic purpura or hemolytic uremic syndrome.

**19.** The pharmaceutical composition according to claim 12, for use as an agent for inhibiting platelet aggregation.

**20.** Use of the compound according to any one of claims 1 to 11 or a pharmaceutically acceptable salt or solvate thereof, for the manufacture of a medicament for use in the treatment of a disease selected from the group consisting of integrin $\alpha_v\beta_3$-mediated diseases, diseases where the inhibition of cell adhesion is therapeutically effective, and diseases where GP IIb/IIIa antagonistic activity and/or platelet aggregation inhibitory activity are therapeutically effective.

**21.** A method for treating a disease selected from the group consisting of integrin $\alpha_v\beta_3$-mediated diseases, diseases where the inhibition of cell adhesion is therapeutically effective, and diseases where GP IIb/IIIa antagonistic activity and/or platelet aggregation inhibitory activity are therapeutically effective, which comprises the step of administering an effective amount of the compound according to any one of claims 1 to 11 or a pharmaceutically acceptable salt or solvate thereof, together with a pharmaceutically acceptable carrier, to a mammal including a human.

**22.** A process for producing a compound represented by formula (IX)

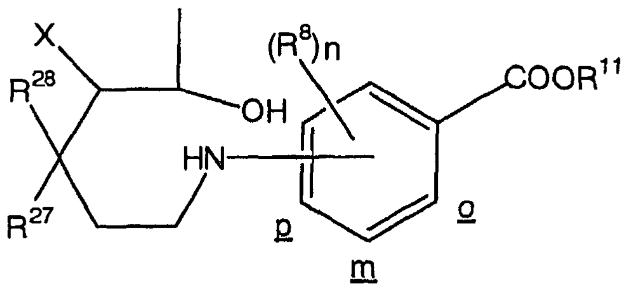

(IX)

wherein $R^{27}$ and $R^{28}$, which may be the same or different, represent a hydrogen atom, hydroxyl, $C_{1-4}$ alkoxy, or $C_{1-4}$ alkyl; X represents hydroxyl, azide, or optionally protected amino; $R^8$, $R^{11}$, and n are as defined in claim 1; and the nitrogen atom is attached to the ortho-, meta-, or para-position of the phenyl group, which comprises the step of:

reacting a compound represented by formula (X)

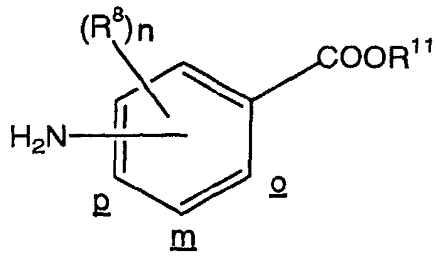

(X)

wherein $R^8$, $R^{11}$, and n are as defined in claim 1; and the nitrogen atom is attached to the ortho-, meta-, or para-position of the phenyl group,
with a compound represented by formula (XI)

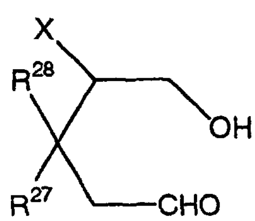

(XI)

wherein X, $R^{27}$, and $R^{28}$ are as defined above.

**23.** A process for producing a compound represented by formula (III')

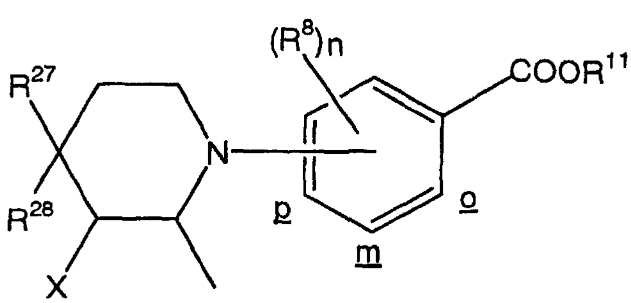

(III')

wherein $R^8$, $R^{11}$, and n are as defined in claim 1; $R^{27}$, $R^{28}$, and X are as defined in claim 22; and the nitrogen atom is attached to the ortho-, meta-, or para-position of the phenyl group, which comprises the step of:

cyclizing a compound represented by formula (XII)

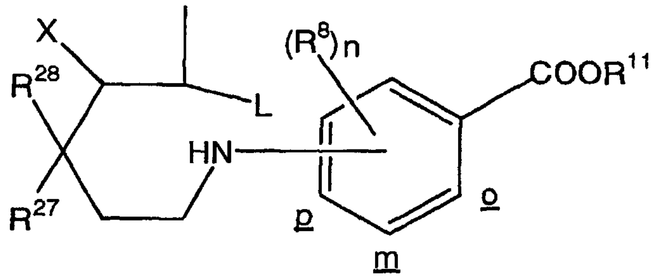

(XII)

wherein $R^8$, $R^{11}$, and n are as defined in claim 1; $R^{27}$, $R^{28}$, and X are as defined in claim 22; L represents a leaving group; and the nitrogen atom is attached to the ortho-, meta-, or para-position of the phenyl group, by an intramolecular ring-closing reaction.

**24.** The process according to claim 23, which further comprises, before the intramolecular ring-closing reaction, the step of converting a secondary hydroxyl group in the compound represented by formula (XI) according to claim 22 to leaving group L to produce the compound represented by formula (XII).

**25.** The process according to any one of claims 22 to 24, wherein $R^{27}$ and $R^{28}$ represent respective groups selected from the group consisting of the following:

- $R^{27}$ represents a hydrogen atom and $R^{28}$ represents methoxy;
- $R^{27}$ represents methyl and $R^{28}$ represents hydroxyl; and
- $R^{27}$ represents methyl and $R^{28}$ represents methoxy.

**26.** A compound represented by formula (XIII)

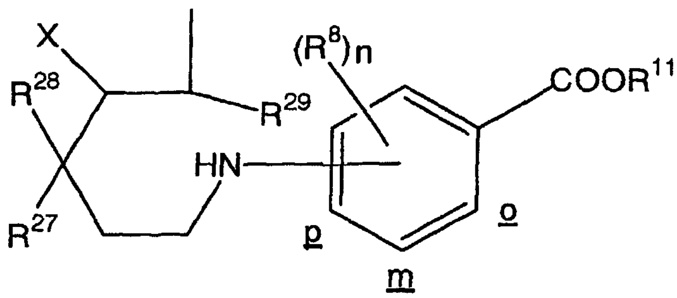

(XIII)

wherein $R^8$, $R^{11}$, and n are as defined in claim 1; $R^{27}$, $R^{28}$, and X are as defined in claim 22; $R^{29}$ represents hydroxyl or a leaving group; and the nitrogen atom is attached to the ortho-, meta-, or para-position of the phenyl group.

**27.** The compound according to claim 26, wherein $R^{27}$ and $R^{28}$ represent respective groups selected from the group consisting of the following:

- $R^{27}$ represents a hydrogen atom and $R^{28}$ represents methoxy;
- $R^{27}$ represents methyl and $R^{28}$ represents hydroxyl; and
- $R^{27}$ represents methyl and $R^{28}$ represents methoxy.

**28.** A compound represented by formula (III')

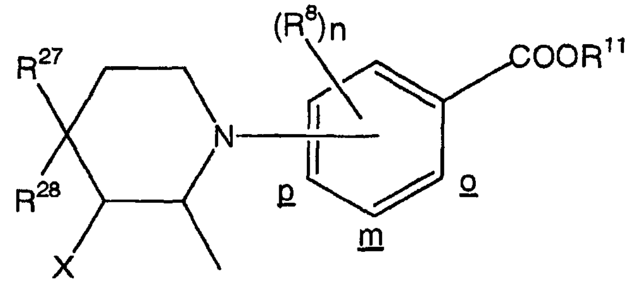

(III')

wherein $R^8$, $R^{11}$, and n are as defined in claim 1; $R^{27}$, $R^{28}$, and X are as defined in claim 22; and the nitrogen atom is attached to the ortho-, meta-, or para-position of the phenyl group.

**29.** The compound according to claim 28, wherein $R^{27}$ and $R^{28}$ represent respective groups selected from the group consisting of the following:

- $R^{27}$ represents a hydrogen atom and $R^{28}$ represents methoxy;
- $R^{27}$ represents methyl and $R^{28}$ represents hydroxyl; and
- $R^{27}$ represents methyl and $R^{28}$ represents methoxy.

# INTERNATIONAL SEARCH REPORT

International application No.
PCT/JP00/07033

A. CLASSIFICATION OF SUBJECT MATTER
Int.Cl[7] C07D211/40, 56, C07D401/12, 14, C07D403/12, C07D409/14, A61K31/444, 31/445, 31/4545, 31/506, 31/5377, 31/55, A61P43/00, 7/02, 9/00, 9/08, 9/10, 35/00, 35/04, 19/02, 19/10, 3/14, C07C227/18, 229/54

According to International Patent Classification (IPC) or to both national classification and IPC

B. FIELDS SEARCHED

Minimum documentation searched (classification system followed by classification symbols)
Int.Cl[7] C07D211/40, 56, C07D401/12, 14, C07D403/12, C07D409/14, A61K31/444, 31/445, 31/4545, 31/506, 31/5377, 31/55, C07C227/18, 229/54

Documentation searched other than minimum documentation to the extent that such documents are included in the fields searched

Electronic data base consulted during the international search (name of data base and, where practicable, search terms used)
CA(STN), CAPLUS(STN), CAOLD(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| PA | AU, 9931678, A1 (MEIJI SEIKA KAISHA,LTD.), 01 November, 1999 (01.11.99) & WO, 99/52872, A1, (21.10.99) | 1-20,22-29 |
| A | WO, 97/36861, A1 (G.D.SEARLE & CO.), 09 October, 1997 (09.10.97) & AU, 9724208, A & US, 5843906, A & EP, 889876, A1 & JP, 2000-507952, A | 1-20 |
| A | WO, 97/23451, A1 (MERCK PATENT GESELLSCHAFT MIT BESCHRANKTER HAFTUNG), 03 July, 1997 (03.07.97) & DE, 19548709, A1 & ZA, 9610725, A & AU, 9713016, A & NO, 9802907, A & CZ, 9801951, A3 & EP, 879227, A1 & SK, 9800783, A3 & CN, 1205687, A & BR, 9612201, A & MX, 9804971, A1 & JP, 2000-502664, A& HU, 9903716, A2 & KR, 99076676, A | 1-20 |
| A | WO, 97/08145, A1 (G.D.SEARLE & CO.), 06 March, 1997 (06.03.97) | 1-20 |

☒ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>11 December, 2000 (11.12.00) | Date of mailing of the international search report<br>19 December, 2000 (19.12.00) |
| Name and mailing address of the ISA/<br>Japanese Patent Office<br>Facsimile No. | Authorized officer<br>Telephone No. |

Form PCT/ISA/210 (second sheet) (July 1992)

**INTERNATIONAL SEARCH REPORT**

International application No.
PCT/JP00/07033

C (Continuation). DOCUMENTS CONSIDERED TO BE RELEVANT

| Category* | Citation of document, with indication, where appropriate, of the relevant passages | Relevant to claim No. |
|---|---|---|
| | & AU, 9671039, A & NO, 9800817, A<br>& ZA, 9607379, A & EP, 850221, A1<br>& CZ, 9800341, A3 & CN, 1201454, A<br>& NZ, 318926, A & BR, 9610422, A<br>& JP, 11-510814, A & MX, 9801716, A1<br>& US, 6013651, A & US, 6028223, A<br>& US, 6100423, A & KR, 99063584, A | |
| A | WOLFGANG GRELL, et al., "Repaglinide and Related Hypoglycemic Benzoic Acid Derivatives", J. Med. Chem., Vol.41, No.26 (1998) pp.5219-5246 | 22-29 |
| A | JOHN P. WOLFE, et al., "Improved Functional Group Compatibility in the Palladium-Catalyzed Amination of Aryl Bromides", Tetrahedron Letters, Vol.38, No.36 (1997) pp.6359-6362 | 22-29 |

Form PCT/ISA/210 (continuation of second sheet) (July 1992)

## INTERNATIONAL SEARCH REPORT

International application No.
PCT/JP00/07033

**Box I Observations where certain claims were found unsearchable (Continuation of item 1 of first sheet)**

This international search report has not been established in respect of certain claims under Article 17(2)(a) for the following reasons:

1. ☒ Claims Nos.: 21
because they relate to subject matter not required to be searched by this Authority, namely:
The invention as set forth in claim 21 pertains to methods for treatment of the human body by therapy.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box II Observations where unity of invention is lacking (Continuation of item 2 of first sheet)**

This International Searching Authority found multiple inventions in this international application, as follows:

I. The inventions as set forth in claims 1 to 11 relate to the compounds of the formula (I), the inventions as set forth in claims 12 to 19 relate to medicinal compositions containing the compounds of the formula (I) as the active ingredient, the invention as set forth in claim 20 relates to use of the compounds of the formula (I) for producing drugs for treating specific diseases, and the invention as set forth in claim 21 relates to methods for treating specific diseases by using the compounds of the formula (I).

1. ☒ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest** ☐ The additional search fees were accompanied by the applicant's protest.
☒ No protest accompanied the payment of additional search fees.

Form PCT/ISA/210 (continuation of first sheet (1)) (July 1992)

**INTERNATIONAL SEARCH REPORT**

International application No.
PCT/JP00/07033

Continuation of Box No.II of continuation of first sheet(1)

II. The inventions as set forth in claims 22 and 25 relate to processes for producing the compounds of the formula (IX), the inventions as set forth in claims 23 to 25 relate to processes for producing the compounds of the formula (III'), the inventions as set forth in claims 26 and 27 relate to processes for producing the compounds of the formula (XIII), and the inventions as set forth in claims 28 and 29 relates to the compounds of the formula (III').

The group I of inventions relates to the novel compounds represented by the formula (I) and use thereof. The group II of inventions relates to the compounds of the formula (XIII) and processes for producing the same and the compounds of the formula (III') and processes for producing the same. The compounds of the formula (XIII) and the compounds of the formula (III') correspond respectively to intermediates and final products.

Although the compounds of the formula (I) and the compounds of the formulae (XIII) and (III') correspond respectively to final products and intermediates and these compounds have each a benzene ring in common as a fundamental chemical structure, it cannot be recognized that a benzene ring is a technically remarkable structure from the viewpoint of the prior art.

Such being the case, these two groups I and II of inventions are not considered as a group of inventions so linked as to form a single general inventive concept.

Form PCT/ISA/210 (extra sheet) (July 1992)